(19) Europäisches Patentamt
European Patent Office
Office européen des brevets

(11) EP 3 705 481 A1

(12) **EUROPEAN PATENT APPLICATION**
published in accordance with Art. 153(4) EPC

(43) Date of publication:
**09.09.2020 Bulletin 2020/37**

(21) Application number: **18872080.9**

(22) Date of filing: **02.11.2018**

(51) Int Cl.:
*C07D 513/04* (2006.01) *A61K 31/425* (2006.01)
*A61P 35/00* (2006.01)

(86) International application number:
**PCT/CN2018/113549**

(87) International publication number:
**WO 2019/085978 (09.05.2019 Gazette 2019/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.11.2017 CN 201711069948**

(71) Applicants:
- **Rui Jin Hospital Affiliated To Shanghai Jiao Tong University School of Medicine**
  **Shanghai 200025 (CN)**
- **Xiamen University**
  **Xiamen, Fujian 361005 (CN)**

(72) Inventors:
- **REN, Ruibao**
  **Shanghai 200025 (CN)**
- **DENG, Xianming**
  **Xiamen**
  **Fujian 361005 (CN)**
- **WU, Min**
  **Shanghai 200025 (CN)**
- **ZHANG, Ting**
  **Xiamen**
  **Fujian 361005 (CN)**
- **JIAO, Bo**
  **Shanghai 200025 (CN)**
- **KANG, Qiaofeng**
  **Xiamen**
  **Fujian 361005 (CN)**
- **HUANG, Wei**
  **Xiamen**
  **Fujian 361005 (CN)**

(74) Representative: **Held, Stephan**
**Meissner Bolte Patentanwälte**
**Rechtsanwälte Partnerschaft mbB**
**Widenmayerstraße 47**
**80538 München (DE)**

(54) **HETEROARYL AMIDE COMPOUNDS, PREPARATION METHOD THEREFOR, PHARMACEUTICAL COMPOSITIONS THEREOF, AND APPLICATIONS THEREOF**

(57) The present invention relates to heteroaryl amide compounds, a preparation method therefor, pharmaceutical compositions thereof and applications thereof, and specifically, relates to a type of compounds having broad-spectrum tumor resistant activity, a preparation method therefor, pharmaceutical compositions containing the compounds, and uses of the compounds in the preparation of drugs for treating tumors.

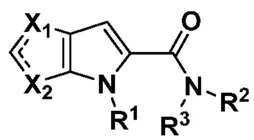

EP 3 705 481 A1

## Description

Technical field

The invention relates to the field of medicinal chemistry, and in particular to a type of compounds having broad-spectrum antitumor activity, a method for the preparation thereof, a pharmaceutical composition comprising the same, and use of these compounds in the manufacture of a medicament for treating tumors.

Background Art

Cancer is a malignant disease that seriously threatens human life and health, and its overall incidence is still on the rise in the world. The large-scale sequencing results of tumor genomes in recent years have revealed the high complexity of genetic variations in tumors. The research of tumor therapy faces challenges including most tumor cells having multiple tumor-driven mutation genes, tumor heterogeneity and tumor evolution, and it is difficult for most tumor-driven mutation genes to be as a treatment target and the like. The present invention aims to obtain compounds having broad-spectrum antitumor activity.

Summary of the Invention

In order to find tumor inhibitors, the inventors of the present invention have designed and synthesized after in-depth research a series of heteroarylamide derivatives having novel structures, high safety, and relatively high activity, and have studied inhibitory activity against tumors of this novel type of derivatives.

The present invention provides a compound having the following general formula:

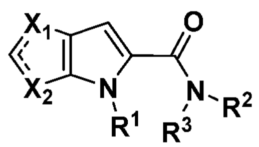

or a stereoisomer thereof, a prodrug thereof, a pharmaceutically acceptable salt thereof or a pharmaceutically acceptable solvate thereof.

More specifically, the present invention provides a compound having the following general formulas (I, II):

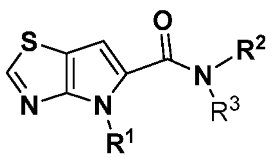

I

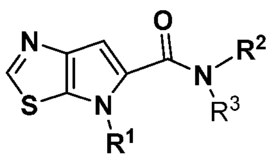

II

The definitions of substituents and symbols are described in detail below.

One object of the present invention is to provide a compound having broad-spectrum antitumor activity, and a stereoisomer thereof, a prodrug thereof, a pharmaceutically acceptable salt thereof or a pharmaceutically acceptable solvate thereof.

Another object of the present invention is to provide a method for the preparation of the above compound.

Another object of the present invention is to provide a pharmaceutical composition comprising the above compound.

Another object of the present invention is to provide use of the above compound and a pharmaceutical composition comprising the above compound in the manufacture of a medicament for treating tumors.

Specific modes for carrying out the invention

Various specific embodiments, modes and examples are described herein, including exemplary embodiments and definitions employed to understand the claimed invention. While the following detailed description sets forth specific preferred embodiments, those skilled in the art will appreciate that these embodiments are illustrative only, and that the present invention can be practiced in other ways. For the purpose of determining infringement, the scope of the present

invention will cover any one or more of the appended claims, including equivalents thereof, and elements or limitations equivalent to those recited.

The present invention is achieved by the following technical solutions.

In the first aspect, the present invention provides a compound having the general formula:

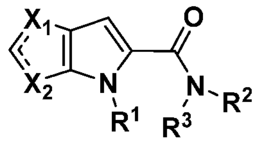

wherein, $X_1$ is selected from the group consisting of N, S; $X_2$ is selected from the group consisting of N, S; and, $X_1$ and $X_2$ are not the same;

$R^1$ is selected from the group consisting of H, C1-C6 alkyl, C3-C6 cycloalkyl;

$R^2$ is selected from the group consisting of H, C1-C6 alkyl, C3-C6 cycloalkyl;

$R^3$ is selected from the group consisting of:

1)

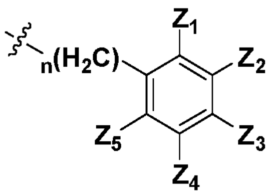

,

wherein n=0, 1 or 2, $Z_1$, $Z_2$, $Z_3$, $Z_4$, $Z_5$ each are independently selected from the group consisting of:

(1) H, F, Cl, Br, I, nitro, cyano, amino; amino optionally substituted by -C1-C6 alkyl, C1-C3 alkyl, -C1-C6 alkylsulfonyl or -C1-C6 alkylcarbonyl; hydroxy, hydroxyformyl, methoxyformyl, ethoxyformyl, n-propoxyformyl, isopropoxyformyl, aminoformyl, N-methylaminoformyl, *N*-ethylaminoformyl, *N*-n-propylaminoformyl, *N*-isopropylaminoformyl, *N*-cyclopropylaminoformyl, *N*-n-butylaminoformyl, *N*-isobutylaminoformyl, *N*-t-butylaminoformyl, *N*-cyclobutylaminoformyl, *N*-n-pentylaminoformyl, *N*-isopentylaminoformyl, *N*-cyclopentylaminoformyl, *N*-n-hexylaminoformyl, *N*-isohexylaminoformyl, *N*-cyclohexylaminoformyl, *N,N*-dimethylaminoformyl, *N*,*N*-diethylaminoformyl, *N*,N-di-n-propylaminoformyl, *N,N*-diisopropylaminoformyl, cyclopropylaminoformyl, cyclobutylaminoformyl, cyclopentylaminoformyl, cyclohexylaminoformyl, 4-hydroxypiperidinylformyl, piperazinylformyl, 4-methylpiperazinylformyl, 4-ethylpiperazinylformyl, 4-n-propylpiperazinylformyl, 4-isopropylpiperazinylformyl, methanesulfonyl, ethylsulfonyl, n-propylsulfonyl, isopropylsulfonyl, n-butylsulfonyl, isobutylsulfonyl, hydroxysulfonyl, aminosulfonyl, *N*-methylaminosulfonyl, *N*-ethylaminosulfonyl, *N*-n-propylaminosulfonyl, *N*-isopropylaminosulfonyl, *N*-cyclopropylaminosulfonyl, *N*-n-butylaminosulfonyl, *N*-isobutylaminosulfonyl, *N*-t-butylaminosulfonyl, *N*-cyclobutylaminosulfonyl, *N*-n-pentylaminosulfonyl, *N*-isopentylaminosulfonyl, *N*-cyclopentylaminosulfonyl, *N*-n-hexylaminosulfonyl, *N*-isohexylaminosulfonyl, *N*-cyclohexylaminosulfonyl, *N,N*-dimethylaminosulfonyl, *N*,*N*-diethylaminosulfonyl, *N*,*N*-di-n-propylaminosulfonyl, *N,N*-diisopropylaminosulfonyl, cyclopropylaminosulfonyl, cyclobutylaminosulfonyl, cyclopentylaminosulfonyl, cyclohexylaminosulfonyl, 4-hydroxypiperidinylsulfonyl, piperazinylsulfonyl, 4-methylpiperazinylsulfonyl, 4-ethylpiperazinylsulfonyl, 4-n-propylpiperazinylsulfonyl, 4-isopropylpiperazinylsulfonyl, formamido, acetamido, propionamido, n-butyramido, isobutyramido, cyclopropylformamido, cyclobutylformamido, cyclopentylformamido, cyclohexylformamido, methanesulfonamido, ethanesulfonamido, n-propanesulfonamido, isopropanesulfonamido, n-butanesulfonamido, isobutanesulfonamido, substituted phenyl-C1-C6 alkyl-aminocarbonyl-C1-C6 alkyl; phenyl-O-C1-C6 alkyl, the phenyl is substituted by C1-C6 alkyl-O-, halogen, C1-C6 alkyl-S- or C1-C6 alkylsulfonyl; 6-membered heterocyclyl-C1-C6 alkyl, the 6-membered heterocyclyl is substituted by - C1-C6 alkyl;

(2) -O-C1-C6 alkyl, -O-C1-C6 fluorine-containing alkyl, -C1-C6 fluorine-containing alkyl, -C1-C6 alkoxycarbonyl, C1-C3 alkyl, C1-C3 alkoxy, C1-C3 oxygen-containing alkyl, C1-C3 fluorine-containing alkyl, C1-C3 fluorine-containing alkoxy;

(3) 5- or 6-membered heterocyclyl containing one or more heteroatoms selected from N, O and S, the 5- or 6-membered heterocyclyl is optionally substituted by C1-C6 alkyl, C1-C6 alkoxy, hydroxy, amino, C1-C6 alkoxycarbonyl, C1-C6 acyl, cyano or optionally substituted heterocyclyl,

[1] including but not limited to: piperidinyl, 4-*N*,*N*-dimethylaminopiperidinyl, 4-*N*,*N*-diethylaminopiperidinyl, 4-*N*,*N*-diisopropylaminopiperidinyl, 4-hydroxypiperidinyl, 4-(*4*-methylpiperazinyl)piperidinyl, 4-(*4*-ethylpiperazinyl)piperidinyl, 4-(*4*-isopropylpiperazinyl)piperidinyl, 4-(*4*-acetylpiperazinyl)piperidinyl, 4-(*4*-t-butoxyformylpiperazinyl)piperidinyl, 4-(*4*-methanesulfonylpiperazinyl)piperidinyl, 4-(*4*-(2-hydroxyethyl)piperazinyl)piperidinyl, 4-(*4*-(2-cyanoethyl)piperazinyl)piperidinyl, 4-(*4*-(3-hydroxypropyl)piperazinyl)piperidinyl, 4-(*4*-(2-*N*,*N*-dimethylaminoethyl)piperazinyl)piperidinyl, 4-(*4*-(2-*N*,*N*-diethylaminoethyl)piperazinyl)piperidinyl, 4-(4-(3-*N*,*N*-dimethylaminopropyl)piperazinyl)piperidinyl, 4-(4-(3-*N*,*N*-diethylaminopropyl)piperazinyl)piperidinyl, 4-(tetrahydropyrrolyl)piperidinyl, 4-(3-*N*,*N*-dimethylaminotetrahydropyrrolyl)piperidinyl; *4*-methylpiperazinyl, *4*-ethylpiperazinyl, *4*-isopropylpiperazinyl, *4*-acetylpiperazinyl, *4*-t-butoxyformylpiperazinyl, *4*-methanesulfonylpiperazinyl, *4*-(2-hydroxyethyl)piperazinyl, *4*-(2-cyanoethyl)piperazinyl, *4*-(3-hydroxypropyl)piperazinyl, *4*-(2-*N,N*-dimethylaminoethyl)piperazinyl, 4-(2-*N*,*N*-diethylaminoethyl)piperazinyl, *4*-(3-*N,N*-dimethylaminopropyl)piperazinyl, 4-(3-*N*,*N*-diethylaminopropyl)piperazinyl, 2-oxo-piperazin-4-yl, 4-(*N*-methyl-4-piperidinyl)piperazinyl, 4-(*N*-ethyl-4-piperidinyl)piperazinyl, 4-(*N*-acetyl-4-piperidinyl)piperazinyl; morpholinyl, 3,5-dimethylmorpholinyl, thiomorpholinyl, tetrahydropyrrolyl, 3-*N*,*N*-dimethyltetrahydropyrrolyl, 3-*N*,*N*-diethyltetrahydropyrrolyl;

(4) heteroaryl, for example, but not limited to pyridyl, furyl, thienyl, benzofuryl;

(5) $Z_2$ and $Z_3$ may form oxygen-containing substituted or unsubstituted 5-membered ring or 6-membered ring; the substituent may be selected from the substituents identical with those of $Z_1$;

(6) $Z_4$ and $Z_5$ may form nitrogen-containing substituted or unsubstituted 5-membered ring or 6-membered ring; the substituent may be selected from the substituents identical with those of $Z_1$;

2) H, C1-C6 alkyl, cyclopropyl, cyclobutyl, cyclopentyl, cyclohexyl, N,N-dimethylamino, N,N-diethylamino, N,N-diisopropylamino, 2-N,N-dimethylaminoethyl, 2-hydroxyethyl, 2-morpholinylethyl, 2-thiomorpholinylethyl, 2-(4-methylpiperazinyl)ethyl, 3-N,N-dimethylaminopropyl, 3-N,N-diethylaminopropyl, 3-N,N-diisopropylaminopropyl, 3-hydroxypropyl, 3-morpholinylpropyl, 3-thiomorpholinylpropyl, 3-(4-methylpiperazinyl)propyl, N-methyl-4-piperidinyl, N-ethyl-4-piperidinyl, N-isopropyl-4-piperidinyl, N-acetyl-4-piperidinyl;

or a stereoisomer thereof, a prodrug thereof, a pharmaceutically acceptable salt thereof or a pharmaceutically acceptable solvate thereof.

In some embodiments, $R^1$ is selected from the group consisting of H, C1-C3 alkyl.

In some embodiments, $R^1$ is selected from the group consisting of H, methyl, ethyl.

In some embodiments, $R^2$ is selected from the group consisting of H, C1-C3 alkyl.

In some embodiments, $R^2$ is selected from the group consisting of H, methyl, ethyl.

In some embodiments, the pharmaceutically acceptable salt is an inorganic acid salt or an organic acid salt, wherein the inorganic acid salt is hydrochloride, hydrobromide, hydroiodide, nitrate, bicarbonate and carbonate, sulfate or phosphate, the organic acid salt is formate, acetate, propionate, benzoate, maleate, fumarate, succinate, tartrate, citrate, ascorbate, α-ketoglutarate, α-glycerophosphate, alkyl sulfonate or aryl sulfonate; preferably, said alkyl sulfonate is methyl sulfonate or ethyl sulfonate; said aryl sulfonate is benzenesulfonate or p-toluenesulfonate.

In the second aspect, the present invention provides a compound having the following general formula I, a stereoisomer thereof, a prodrug thereof, a pharmaceutically acceptable salt thereof or a pharmaceutically acceptable solvate thereof,

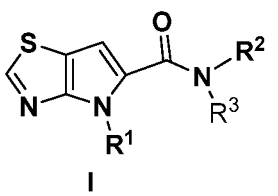

I

wherein:

$R^1$ is selected from the group consisting of H, C1-C6 alkyl, C3-C6 cycloalkyl;
$R^2$ is selected from the group consisting of H, C1-C6 alkyl, C3-C6 cycloalkyl;
$R^3$ is selected from the group consisting of:

1)

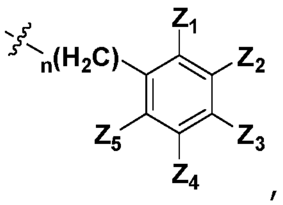

wherein n=0, 1 or 2, $Z_1$, $Z_2$, $Z_3$, $Z_4$, $Z_5$ each are independently selected from the group consisting of:

(1) H, F, Cl, Br, I, nitro, cyano, amino; amino optionally substituted by -C1-C6 alkyl, C1-C3 alkyl, -C1-C6 alkylsulfonyl or -C1-C6 alkylcarbonyl; hydroxy, hydroxyformyl, methoxyformyl, ethoxyformyl, n-propoxyformyl, isopropoxyformyl, aminoformyl, *N*-methylaminoformyl, *N*-ethylaminoformyl, *N*-n-propylaminoformyl, *N*-isopropylaminoformyl, *N*-cyclopropylaminoformyl, *N*-n-butylaminoformyl, *N*-isobutylaminoformyl, *N*-t-butylaminoformyl, *N*-cyclobutylaminoformyl, *N*-n-pentylaminoformyl, *N*-isopentylaminoformyl, *N*-cyclopentylaminoformyl, *N*-n-hexylaminoformyl, *N*-isohexylaminoformyl, *N*-cyclohexylaminoformyl, *N,N*-dimethylaminoformyl, *N,N*-diethylaminoformyl, *N,N*-di-n-propylaminoformyl, *N,N*-diisopropylaminoformyl, cyclopropylaminoformyl, cyclobutylaminoformyl, cyclopentylaminoformyl, cyclohexylaminoformyl, 4-hydroxypiperidinylformyl, piperazinylformyl, 4-methylpiperazinylformyl, 4-ethylpiperazinylformyl, 4-n-propylpiperazinylformyl, 4-isopropylpiperazinylformyl, methanesulfonyl, ethylsulfonyl, n-propylsulfonyl, isopropylsulfonyl, n-butylsulfonyl, isobutylsulfonyl, hydroxysulfonyl, aminosulfonyl, *N*-methylaminosulfonyl, *N*-ethylaminosulfonyl, *N*-n-propylaminosulfonyl, *N*-isopropylaminosulfonyl, *N*-cyclopropylaminosulfonyl, *N*-n-butylaminosulfonyl, *N*-isobutylaminosulfonyl, *N*-t-butylaminosulfonyl, *N*-cyclobutylaminosulfonyl, *N*-n-pentylaminosulfonyl, *N*-isopentylaminosulfonyl, *N*-cyclopentylaminosulfonyl, *N*-n-hexylaminosulfonyl, *N*-isohexylaminosulfonyl, *N*-cyclohexylaminosulfonyl, *N,N*-dimethylaminosulfonyl, *N,N*-diethylaminosulfonyl, *N,N*-di-n-propylaminosulfonyl, *N,N*-diisopropylaminosulfonyl, cyclopropylaminosulfonyl, cyclobutylaminosulfonyl, cyclopentylaminosulfonyl, cyclohexylaminosulfonyl, 4-hydroxypiperidinylsulfonyl, piperazinylsulfonyl, 4-methylpiperazinylsulfonyl, 4-ethylpiperazinylsulfonyl, 4-n-propylpiperazinylsulfonyl, 4-isopropylpiperazinylsulfonyl, formamido, acetamido, propionamido, n-butyramido, isobutyramido, cyclopropylformamido, cyclobutylformamido, cyclopentylformamido, cyclohexylformamido, methanesulfonamido, ethanesulfonamido, n-propanesulfonamido, isopropanesulfonamido, n-butanesulfonamido, isobutanesulfonamido, substituted phenyl-C1-C6 alkyl-aminocarbonyl-C1-C6 alkyl; phenyl-O-C1-C6 alkyl, the phenyl is substituted by C1-C6 alkyl-O-, halogen, C1-C6 alkyl-S- or C1-C6 alkylsulfonyl; 6-membered heterocyclyl-C1-C6 alkyl, the 6-membered heterocyclyl is substituted by - C1-C6 alkyl;
(2) -O-C1-C6 alkyl, -O-C1-C6 fluorine-containing alkyl, -C1-C6 fluorine-containing alkyl, -C1-C6 alkoxycarbonyl, C1-C3 alkyl, C1-C3 alkoxy, C1-C3 oxygen-containing alkyl, C1-C3 fluorine-containing alkyl, C1-C3 fluorine-containing alkoxy;
(3) 5- or 6-membered heterocyclyl containing one or more heteroatoms selected from N, O and S, the 5- or 6-membered heterocyclyl is optionally substituted by C1-C6 alkyl, C1-C6 alkoxy, hydroxy, amino, C1-C6 alkoxycarbonyl, C1-C6 acyl, cyano or optionally substituted heterocyclyl,
[2] including but not limited to: piperidinyl, 4-*N*,*N*-dimethylaminopiperidinyl, 4-*N*,*N*-diethylaminopiperidinyl, 4-*N*,*N*-diisopropylaminopiperidinyl, 4-hydroxypiperidinyl, 4-(*4*-methylpiperazinyl)piperidinyl, 4-(*4*-ethylpiperazinyl)piperidinyl, 4-(*4*-isopropylpiperazinyl)piperidinyl, 4-(*4*-acetylpiperazinyl)piperidinyl, 4-(*4*-t-butoxyformylpiperazinyl)piperidinyl, 4-(*4*-methanesulfonylpiperazinyl)piperidinyl, 4-(*4*-(2-hydroxyethyl)piperazinyl)piperidinyl, 4-(*4*-(2-cyanoethyl)piperazinyl)piperidinyl, 4-(*4*-(3-hydroxypropyl)piperazinyl)piperidinyl, 4-(*4*-(2-*N*,*N*-dimethylaminoethyl)piperazinyl)piperidinyl, 4-(*4*-(2-*N*,*N*-diethylaminoethyl)piperazinyl)piperidinyl, 4-(*4*-(3-*N*,*N*-dimethylaminopropyl)piperazinyl)piperidinyl, 4-(*4*-(3-*N*,*N*-diethylaminopropyl)piperazinyl)piperidinyl, 4-(tetrahydropyrrolyl)piperidinyl, 4-(3-*N*,*N*-dimethylaminotetrahydropyrrolyl)piperidinyl;
*4*-methylpiperazinyl, *4*-ethylpiperazinyl, *4*-isopropylpiperazinyl, *4*-acetylpiperazinyl, *4*-t-butoxyformylpiperazinyl, *4*-methanesulfonylpiperazinyl, *4*-(2-hydroxyethyl)piperazinyl, *4*-(2-cyanoethyl)piperazinyl, *4*-(3-hydroxypropyl)piperazinyl, *4*-(2-*N*,*N*-dimethylaminoethyl)piperazinyl, *4*-(2-*N*,*N*-diethylaminoethyl)piperazinyl, *4*-(3-*N*,*N*-dimethylaminopropyl)piperazinyl, *4*-(3-*N*,*N*-diethylaminopropyl)piperazinyl, 2-oxo-piperazin-4-yl, 4-(*N*-methyl-4-piperidinyl)piperazinyl, *4*-(*N*-ethyl-4-piperidinyl)piperazinyl, 4-(*N*-acetyl-4-piperidinyl)piperazinyl; morpholinyl, 3,5-dimethylmorpholinyl, thiomorpholinyl, tetrahydropyrrolyl, 3-*N*,*N*-dimethylaminotetrahydropyrrolyl, 3-*N*,*N*-diethylaminotetrahydropyrrolyl;
(4) heteroaryl, for example, but not limited to pyridyl, furyl, thienyl, benzofuryl;
(5) $Z_2$ and $Z_3$ may form oxygen-containing substituted or unsubstituted 5-membered ring or 6-membered ring; the substituent may be selected from the substituents identical with those of $Z_1$;

(6) $Z_4$ and $Z_5$ may form nitrogen-containing substituted or unsubstituted 5-membered ring or 6-membered ring; the substituent may be selected from the substituents identical with those of $Z_1$;

2) H, C1-C6 alkyl, cyclopropyl, cyclobutyl, cyclopentyl, cyclohexyl, N,N-dimethylamino, N,N-diethylamino, N,N-diisopropylamino, 2-N,N-dimethylaminoethyl, 2-hydroxyethyl, 2-morpholinylethyl, 2-thiomorpholinylethyl, 2-(4-methylpiperazinyl)ethyl, 3-N,N-dimethylaminopropyl, 3-N,N-diethylaminopropyl, 3-N,N-diisopropylaminopropyl, 3-hydroxypropyl, 3-morpholinylpropyl, 3-thiomorpholinylpropyl, 3-(4-methylpiperazinyl)propyl, N-methyl-4-piperidinyl, N-ethyl-4-piperidinyl, N-isopropyl-4-piperidinyl, N-acetyl-4-piperidinyl.

In some embodiments, $R^1$ is selected from the group consisting of H, C1-C3 alkyl.

In some embodiments, $R^1$ is selected from the group consisting of H, methyl, ethyl.

In some embodiments, $R^2$ is selected from the group consisting of H, C1-C3 alkyl.

In some embodiments, $R^2$ is selected from the group consisting of H, methyl, ethyl.

In some embodiments, $R^3$ is selected from the group consisting of:

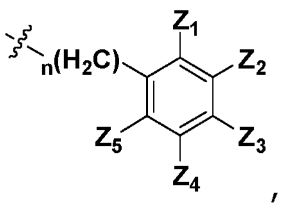

wherein n=0, 1 or 2,
when n=0, one of $Z_1$, $Z_2$, $Z_3$, $Z_4$, $Z_5$ is selected from the following groups, the rest being -H: hydroxy, -O-C1-C6 alkyl, -O-C1-C6 fluorine-containing alkyl, -C1-C6 fluorine-containing alkyl, -C1-C6 alkoxycarbonyl, amino; amino optionally substituted by -C1-C6 alkyl, -C1-C6 alkylsulfonyl or -C1-C6 alkylcarbonyl; aminosulfonyl, nitro, substituted phenyl-C1-C6 alkyl-aminocarbonyl-C1-C6 alkyl (more preferably phenyl-C1-C6 alkylaminocarbonyl-C1-C6 alkyl, the phenyl is substituted by halogen); phenyl-O-C1-C6 alkyl, the phenyl is substituted by C1-C6 alkyl-O-, halogen, C1-C6 alkyl-S- or C1-C6 alkylsulfonyl;
or, two of $Z_1$, $Z_2$, $Z_3$, $Z_4$, $Z_5$ each are independently selected from the following groups, the rest being -H (more preferably $Z_2$, $Z_3$ each or $Z_1$, $Z_4$ each or $Z_2$, $Z_4$ each are independently selected from the following groups, the rest being -H): -C1-C6 fluorine-containing alkyl; 6-membered heterocyclyl-C1-C6 alkyl, the 6-membered heterocyclyl is substituted by -C1-C6 alkyl (more preferably piperazinyl-C1-C6 alkyl, the piperazinyl is substituted by -C1-C6 alkyl); -C1-C6 alkyl, substituted phenylcarbonyl-amino, -C1-C6 alkyl-O-carbonyl, 5-membered heteroaryl substituted by -C1-C6 alkyl (more preferably imidazolyl substituted by -C1-C6 alkyl);
when n=1, one of $Z_1$, $Z_2$, $Z_3$, $Z_4$, $Z_5$ is selected from the following groups, the rest being -H: pyridyl, furyl, thienyl, benzofuryl;
when n=2, one of $Z_1$, $Z_2$, $Z_3$, $Z_4$, $Z_5$ is selected from the following groups, the rest being -H: aminosulfonyl.

In some embodiments, $R^3$ is selected from the group consisting of:

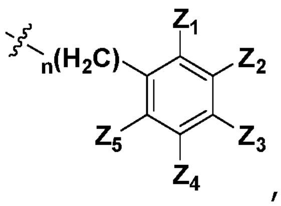

wherein n=0 or 1,
when n=0, $Z_1$, $Z_2$, $Z_4$, $Z_5$ each are -H, $Z_3$ is selected from the following groups: hydroxy, -O-C1-C6 alkyl, -O-C1-C6 fluorine-containing alkyl, -C1-C6 fluorine-containing alkyl, -C1-C6 alkoxycarbonyl, amino; amino optionally substituted by -C1-C6 alkyl, -C1-C6 alkylsulfonyl or -C1-C6 alkylcarbonyl; aminosulfonyl, nitro;
or, $Z_2$ or $Z_4$ is selected from the following groups, the rest being -H: -C1-C6 alkoxycarbonyl, substituted phenyl-C1-C6 alkyl-aminocarbonyl-C1-C6 alkyl (more preferably phenyl-C1-C6 alkyl-aminocarbonyl-C1-C6 alkyl, the phenyl is substituted by halogen); phenyl-O-C1-C6 alkyl, the phenyl is substituted by C1-C6 alkyl-O-, halogen, C1-C6 alkyl-S- or C1-C6 alkylsulfonyl,
or, $Z_2$, $Z_3$ each are independently selected from the following groups, the rest being - H: -C1-C6 fluorine-containing alkyl; 6-membered heterocyclyl-C1-C6 alkyl, the 6-membered heterocyclyl is substituted by -C1-C6 alkyl (more preferably

piperazinyl-C1-C6 alkyl, the piperazinyl is substituted by -C1-C6 alkyl);
or, $Z_1$, $Z_4$ each are independently selected from the following groups, the rest being - H: -C1-C6 alkyl, substituted phenylcarbonyl-amino, -C1-C6 alkyl-O-carbonyl;
or, $Z_2$, $Z_4$ each are independently selected from the following groups, the rest being - H: -C1-C6 fluorine-containing alkyl, 5-membered heteroaryl substituted by -C1-C6 alkyl (more preferably imidazolyl substituted by -C1-C6 alkyl);
when n=1, $Z_1$ or $Z_5$ is selected from the following groups, the rest being -H: pyrid-4-yl, pyrid-3-yl, fur-2-yl, fur-3-yl, thien-2-yl, thien-3-yl, benzofuryl;
when n=2, $Z_1$, $Z_2$, $Z_4$, $Z_5$ each are -H, $Z_3$ is aminosulfonyl.

In some embodiments, $R^3$ is selected from the group consisting of:

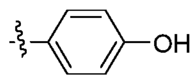, 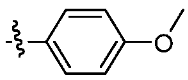, 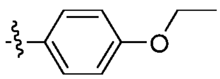, 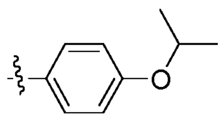, 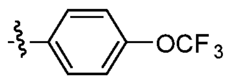,

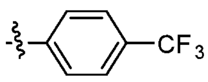, 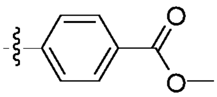,

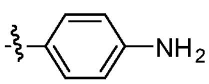, 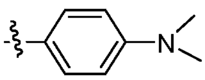, 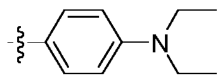, 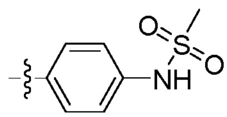, 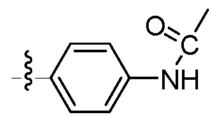,

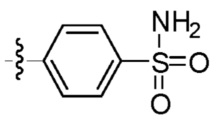, 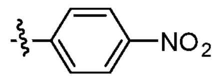,

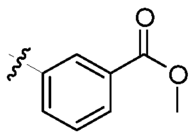, 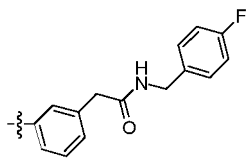, 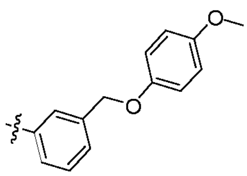, 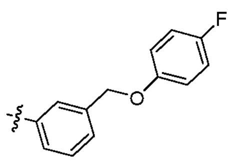, 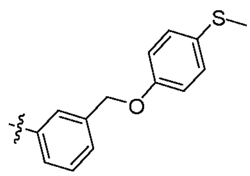

, 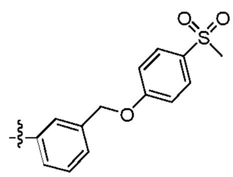,

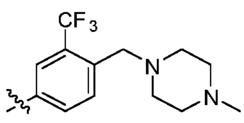, 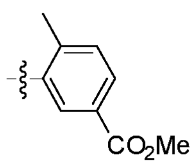, 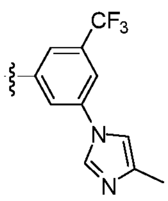, 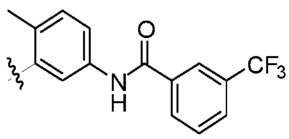,

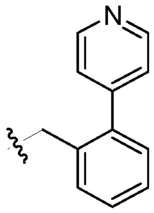, 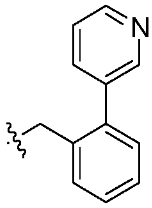, 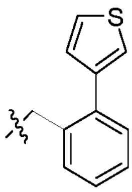, 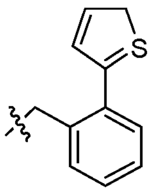, 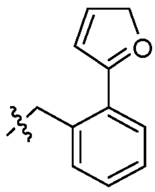, 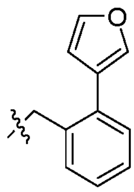, 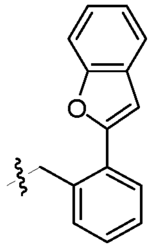,

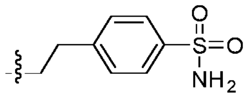.

In some embodiments, the pharmaceutically acceptable salt is an inorganic acid salt or an organic acid salt, wherein the inorganic acid salt is hydrochloride, hydrobromide, hydroiodide, nitrate, bicarbonate and carbonate, sulfate or phosphate, the organic acid salt is formate, acetate, propionate, benzoate, maleate, fumarate, succinate, tartrate, citrate, ascorbate, α-ketoglutarate, α-glycerophosphate, alkyl sulfonate or aryl sulfonate; preferably, said alkyl sulfonate is methyl sulfonate or ethyl sulfonate; said aryl sulfonate is benzenesulfonate or p-toluenesulfonate.

In the third aspect, the present invention provides a compound having the following general formula II, a stereoisomer thereof, a prodrug thereof, a pharmaceutically acceptable salt thereof or a pharmaceutically acceptable solvate thereof:

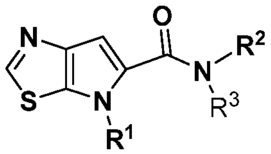

**II**

wherein:

$R^1$ is selected from the group consisting of H, C1-C6 alkyl, C3-C6 cycloalkyl;
$R^2$ is selected from the group consisting of H, C1-C6 alkyl, C3-C6 cycloalkyl;
$R^3$ is selected from the group consisting of:

1)

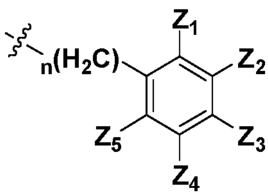,

wherein n=0, 1 or 2, $Z_1$, $Z_2$, $Z_3$, $Z_4$, $Z_5$ each are independently selected from the group consisting of:

(1) H, F, Cl, Br, I, nitro, cyano, amino, hydroxy, hydroxyformyl, methoxyformyl, ethoxyformyl, n-propoxyformyl, isopropoxyformyl, aminoformyl, *N*-methylaminoformyl, *N*-ethylaminoformyl, *N*-n-propylaminoformyl, *N*-isopropylaminoformyl, *N*-cyclopropylaminoformyl, *N*-n-butylaminoformyl, *N*-isobutylaminoformyl, *N*-t-butylaminoformyl, *N*-cyclobutylaminoformyl, *N*-n-pentylaminoformyl, *N*-isopentylaminoformyl, *N*-cyclopentylaminoformyl, *N*-n-hexylaminoformyl, *N*-isohexylaminoformyl, *N*-cyclohexylaminoformyl, *N*,*N*-dimethylaminoformyl, *N,N*-diethylaminoformyl, *N*,*N*-di-n-propylaminoformyl, *N*,*N*-diisopropylaminoformyl, cyclopropylaminoformyl, cyclobutylaminoformyl, cyclopentylaminoformyl, cyclohexylaminoformyl, 4-hydroxypiperidinylformyl, piperazinylformyl, 4-methylpiperazinylformyl, 4-ethylpiperazinylformyl, 4-n-propylpiperazinylformyl, 4-isopropylpiperazinylformyl, methanesulfonyl, ethylsulfonyl, n-propylsulfonyl, isopropylsulfonyl, n-butylsulfonyl, isobutylsulfonyl, hydroxysulfonyl, aminosulfonyl, N-methylaminosulfonyl, *N*-ethylaminosulfonyl, *N*-n-propylaminosulfonyl, *N*-isopropylaminosulfonyl, *N*-cyclopropylaminosulfonyl, *N*-n-butylaminosulfonyl, *N*-isobutylaminosulfonyl, *N*-t-butylaminosulfonyl, *N*-cyclobutylaminosulfonyl, *N*-n-pentylaminosulfonyl, *N*-isopentylaminosulfonyl, *N*-cyclopentylaminosulfonyl, *N*-n-hexylaminosulfonyl, *N*-isohexylaminosulfonyl, *N*-cyclohexylaminosulfonyl, *N,N*-dimethylaminosulfonyl, *N*,*N*-diethylaminosulfonyl, *N*,*N*-di-n-propylaminosulfonyl, *N,N*-diisopropylaminosulfonyl, cyclopropylaminosulfonyl, cyclobutylaminosulfonyl, cyclopentylaminosulfonyl, cyclohexylaminosulfonyl, 4-hydroxypiperidinylsulfonyl, piperazinylsulfonyl, 4-methylpiperazinylsulfonyl, 4-ethylpiperazinylsulfonyl, 4-n-propylpiperazinylsulfonyl, 4-isopropylpiperazinylsulfonyl, formamido, acetamido, propionamido, n-butyramido, isobutyramido, cyclopropylformamido, cyclobutylformamido, cyclopentylformamido, cyclohexylformamido, methanesulfonamido, ethanesulfonamido, n-propanesulfonamido, isopropanesulfonamido, n-butanesulfonamido, isobutanesulfonamido;

(2) -O-C1-C6 alkyl, -O-C1-C6 fluorine-containing alkyl, -C1-C6 fluorine-containing alkyl, C1-C3 alkyl, C1-C3 alkoxy, C1-C3 oxygen-containing alkyl, C1-C3 fluorine-containing alkyl, C1-C3 fluorine-containing alkoxy; 6-membered heterocyclyl-C1-C6 alkyl, the 6-membered heterocyclyl is substituted by -C1-C6 alkyl;

(3) 5- or 6-membered heterocyclyl containing one or more heteroatoms selected from N, O and S, the 5- or 6-membered heterocyclyl is optionally substituted by C1-C6 alkyl, C1-C6 alkoxy, hydroxy, amino, C1-C6 alkoxycarbonyl, C1-C6 acyl, cyano or optionally substituted heterocyclyl,

[3] including but not limited to: piperidinyl, 4-*N*,*N*-dimethylaminopiperidinyl, 4-*N*,*N*-diethylaminopiperidinyl, 4-*N*,*N*-diisopropylaminopiperidinyl, 4-hydroxypiperidinyl, 4-(*4*-methylpiperazinyl)piperidinyl, 4-(*4*-ethylpiperazinyl)piperidinyl, 4-(*4*-isopropylpiperazinyl)piperidinyl, 4-(*4*-acetylpiperazinyl)piperidinyl, 4-(*4*-t-butoxyformylpiperazinyl)piperidinyl, 4-(*4*-methanesulfonylpiperazinyl)piperidinyl, 4-(*4*-(2-hydroxyethyl)piperazinyl)piperidinyl, 4-(*4*-(2-cyanoethyl)piperazinyl)piperidinyl, 4-(*4*-(3-hydroxypropyl)piperazinyl)piperidinyl, 4-(*4*-(2-*N*,*N*-dimethylaminoethyl)piperazinyl)piperidinyl, 4-(*4*-(2-*N*,*N*-diethylaminoethyl)piperazinyl)piperidinyl, 4-(*4*-(3-*N*,*N*-dimethylaminopropyl)piperazinyl)piperidinyl, 4-(*4*-(3-*N*,*N*-diethylaminopropyl)piperazinyl)piperidinyl, 4-(tetrahydropyrrolyl)piperidinyl, 4-(3-*N*,*N*-dimethylaminotetrahydropyrrolyl)piperidinyl; *4*-methylpiperazinyl, *4*-ethylpiperazinyl, *4*-isopropylpiperazinyl, *4*-acetylpiperazinyl, *4*-t-butoxyformylpiperazinyl, *4*-methanesulfonylpiperazinyl, *4*-(2-hydroxyethyl)piperazinyl, *4*-(2-cyanoethyl)piperazinyl, *4*-(3-hydroxypropyl)piperazinyl, *4*-(2-*N,N*-dimethylaminoethyl)piperazinyl, *4*-(2-*N*,*N*-diethylaminoethyl)piperazinyl, *4*-(3-*N,N*-dimethylaminopropyl)piperazinyl, *4*-(3-*N*,*N*-diethylaminopropyl)piperazinyl, 2-oxo-piperazin-4-yl, *4*-(*N*-methyl-4-piperidinyl)piperazinyl, *4*-(*N*-ethyl-4-piperidinyl)piperazinyl, 4-(*N*-acetyl-4-piperidinyl)piperazinyl; morpholinyl, 3,5-dimethylmorpholinyl, thiomorpholinyl, tetrahydropyrrolyl, 3-*N*,*N*-dimethylaminotetrahydropyrrolyl, 3-*N*,*N*-diethylaminotetrahydropyrrolyl;

(4) heteroaryl, for example, but not limited to pyridyl, furyl, thienyl, benzofuryl;

(5) $Z_2$ and $Z_3$ may form oxygen-containing substituted or unsubstituted 5-membered ring or 6-membered ring; the substituent may be selected from the substituents identical with those of $Z_1$;

(6) $Z_4$ and $Z_5$ may form nitrogen-containing substituted or unsubstituted 5-membered ring or 6-membered ring; the substituent may be selected from the substituents identical with those of $Z_1$;

2) H, C1-C6 alkyl, cyclopropyl, cyclobutyl, cyclopentyl, cyclohexyl, N,N-dimethylamino, N,N-diethylamino, N,N-diisopropylamino, 2-N,N-dimethylaminoethyl, 2-hydroxyethyl, 2-morpholinylethyl, 2-thiomorpholinylethyl, 2-(4-methylpiperazinyl)ethyl, 3-N,N-dimethylaminopropyl, 3-N,N-diethylaminopropyl, 3-N,N-diisopropylaminopropyl, 3-hydroxypropyl, 3-morpholinylpropyl, 3-thiomorpholinylpropyl, 3-(4-methylpiperazinyl)propyl, N-methyl-4-piperidinyl, N-ethyl-4-piperidinyl, N-isopropyl-4-piperidinyl, N-acetyl-4-piperidinyl.

In some embodiments, $R^1$ is selected from the group consisting of H, C1-C3 alkyl.

In some embodiments, $R^1$ is selected from the group consisting of H, methyl, ethyl.

In some embodiments, $R^2$ is selected from the group consisting of H, C1-C3 alkyl.

In some embodiments, $R^2$ is selected from the group consisting of H, methyl, ethyl.

In some embodiments, $R^3$ is selected from the group consisting of:

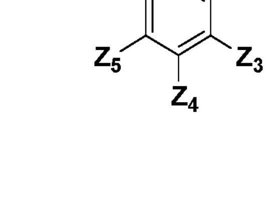

,

wherein n=0 or 1,

when n=0, one of $Z_1$, $Z_2$, $Z_3$, $Z_4$, $Z_5$ is selected from the following groups, the rest being -H: hydroxy, -O-C1-C6 alkyl, -O-C1-C6 fluorine-containing alkyl, -C1-C6 fluorine-containing alkyl;

or, two of $Z_1$, $Z_2$, $Z_3$, $Z_4$, $Z_5$ each are independently selected from the following groups, the rest being -H (more preferably $Z_2$, $Z_4$ each or $Z_2$, $Z_3$ each are independently selected from the following groups, the rest being -H): -C1-C6 fluorine-containing alkyl, 5-membered heteroaryl substituted by -C1-C6 alkyl (more preferably imidazolyl substituted by -C1-C6 alkyl); 6-membered heterocyclyl-C1-C6 alkyl, the 6-membered heterocyclyl is substituted by -C1-C6 alkyl (more preferably piperazinyl-C1-C6 alkyl, the piperazinyl is substituted by -C1-C6 alkyl);

when n=1, one of $Z_1$, $Z_2$, $Z_3$, $Z_4$, $Z_5$ is benzofuryl, the rest being -H.

In some embodiments, $R^3$ is selected from the group consisting of:

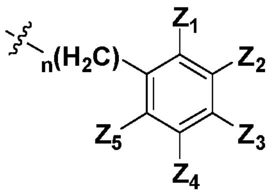

,

wherein n=0 or 1,

when n=0, $Z_1$, $Z_2$, $Z_4$, $Z_5$ each are -H, $Z_3$ is selected from the following groups: hydroxy, -O-C1-C6 alkyl, -O-C1-C6 fluorine-containing alkyl, -C1-C6 fluorine-containing alkyl;

or, $Z_2$, $Z_4$ each are independently selected from the following groups, the rest being - H: -C1-C6 fluorine-containing alkyl, 5-membered heteroaryl substituted by -C1-C6 alkyl (more preferably imidazolyl substituted by -C1-C6 alkyl);

or, $Z_2$, $Z_3$ each, or $Z_3$, $Z_4$ each are independently selected from the following groups, the rest being -H: -C1-C6 fluorine-containing alkyl; 6-membered heterocyclyl-C1-C6 alkyl, the 6-membered heterocyclyl is substituted by -C1-C6 alkyl (more preferably piperazinyl-C1-C6 alkyl, the piperazinyl is substituted by -C1-C6 alkyl);

when n=1, $Z_1$, $Z_3$, $Z_4$, $Z_5$ each are -H, $Z_2$ is benzofuryl.

In some embodiments, $R^3$ is selected from the group consisting of:

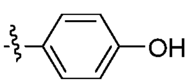 , 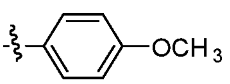 , 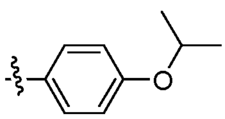 , 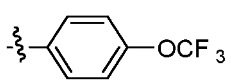 , 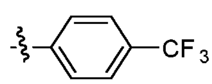 ,

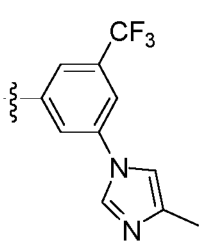 , 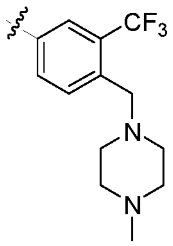 , 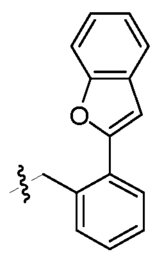 .

In some embodiments, the pharmaceutically acceptable salt is an inorganic acid salt or an organic acid salt, wherein the inorganic acid salt is hydrochloride, hydrobromide, hydroiodide, nitrate, bicarbonate and carbonate, sulfate or phosphate, the organic acid salt is formate, acetate, propionate, benzoate, maleate, fumarate, succinate, tartrate, citrate, ascorbate, $\alpha$-ketoglutarate, $\alpha$-glycerophosphate, alkyl sulfonate or aryl sulfonate; preferably, said alkyl sulfonate is methyl sulfonate or ethyl sulfonate; said aryl sulfonate is benzenesulfonate or p-toluenesulfonate.

Unless otherwise indicated, the above groups and substituents have the ordinary meanings in the field of medicinal chemistry.

The term "C1-C6 alkyl" refers to any straight-chain or branched-chain group having 1 to 6 carbon atoms, such as methyl, ethyl, n-propyl, isopropyl, n-butyl, isobutyl, t-butyl, sec-butyl, n-pentyl, tert-pentyl, n-hexyl and the like.

The term "C1-C3 alkyl" refers to any straight-chain or branched-chain group having 1 to 3 carbon atoms, such as methyl, ethyl, n-propyl, isopropyl and the like.

It should be noted that "oxygen-containing alkyl" refers to a group in which the H in alkyl skeleton is substituted

by one or more alkoxy groups, for example, methoxyethyl, methoxyethoxymethyl and the like.

For example, C1-C6 oxygen-containing alkyl refers to a group in which the H in C1-C6 alkyl skeleton is substituted by one or more C1-C6 alkoxy groups, for example, methoxyethyl, methoxyethoxymethyl and the like. Similarly, C1-C3 oxygen-containing alkyl refers to a group in which the H in C1-C3 alkyl skeleton is substituted by one or more C1-C6 alkoxy groups.

The term "fluorine-containing alkyl" refers to a group in which the H in alkyl skeleton is substituted by one or more fluoro groups, for example, monofluoromethyl, difluoroethyl, trifluoromethyl, and the like.

The term "C3-C6 cycloalkyl" refers to a hydrocarbon of a 3-6 membered monocyclic system having a saturated ring. The C3-C6 cycloalkyl may be cyclopropyl, cyclobutyl, cyclopentyl, cyclohexyl and the like.

The term "cyano" refers to -CN residue.

The term "nitro" refers to $-NO_2$ group.

The terms "alkoxy", "cycloalkoxy" and derivatives thereof refer to any of the above-mentioned alkyl (for example, $C_1$-$C_6$ alkyl, $C_1$-$C_3$ alkyl and the like), cycloalkyl (for example, $C_3$-$C_6$ cycloalkyl), which is attached to the remainder of molecules through oxygen atom (-O-).

The term "heteroaryl" refers to an aromatic heterocyclic ring, which is usually a 5-, 6-, 7-, 8-membered heterocyclic ring having from 1 to 3 heteroatoms selected from N, O and S; a heteroaryl ring may be optionally further fused or attached to aromatic or non-aromatic carbocyclic rings or heterocyclic rings. Non-limiting examples of the heteroaryl group are, for example, pyridyl, pyrazinyl, pyrimidinyl, pyridazinyl, indolyl, imidazolyl, thiazolyl, isothiazolyl, thioxazolyl, pyrrolyl, phenyl-pyrrolyl, furyl, phenyl-furyl, oxazolyl, isoxazolyl, pyrazolyl, thienyl, benzofuryl, benzothienyl, benzo 1,3-dioxolanyl (benzodioxolanyl), isoindolinyl, benzoimidazolyl, indazolyl, quinolyl, isoquinolyl, 1,2,3-triazolyl, 1-phenyl-1,2,3-triazolyl, 2,3-indolinyl, 2,3-dihydrobenzofuryl, 2,3-dihydrobenzothienyl, benzopyranyl, 2,3-dihydrobenzoxazinyl, 2,3-dihydroquinoxalinyl and the like.

The term "heterocyclyl" (also referred to as "heterocycloalkyl") refers to 3-, 4-, 5-, 6- and 7-membered saturated or partially unsaturated carbocyclic rings, wherein one or more carbon atoms are replaced by heteroatoms such as nitrogen, oxygen and sulfur. Non-limiting examples of the heterocyclic group are, for example, pyranyl, pyrrolidinyl, pyrrolinyl, imidazolinyl, imidazolidinyl, pyrazolidinyl, pyrazolinyl, thiazolinyl, thiazolidinyl, dihydrofuryl, tetrahydrofuryl, 1,3-dioxolanyl, piperidinyl, piperazinyl, morpholino, morpholinyl, tetrahydropyrrolyl, thiomorpholinyl and the like.

For example, "6-membered heterocyclyl" refers to 6-membered saturated or partially unsaturated carbocyclic rings, wherein one or more carbon atoms are replaced by heteroatoms such as nitrogen, oxygen and sulfur. Non-limiting examples of the 6-membered heterocyclyl are, for example, pyranyl, piperidinyl, piperazinyl, morpholino, morpholinyl, thiomorpholinyl and the like.

"5-membered heterocyclyl" refers to 5-membered saturated or partially unsaturated carbocyclic rings, wherein one or more carbon atoms are replaced by heteroatoms such as nitrogen, oxygen and sulfur. Non-limiting examples of the 5-membered heterocyclyl are, for example, pyrrolidinyl, pyrrolinyl, imidazolinyl, imidazolidinyl, pyrazolidinyl, pyrazolinyl, thiazolinyl, thiazolidinyl, 1,3-dioxolanyl and the like.

The term "optionally substituted heterocyclyl" refers to the group formed in the situation the above-mentioned "heterocyclyl" is substituted by one or more "C1-C6 alkyl", "C1-C3 alkyl", "C3-C6 cycloalkyl" and the like.

The term "C1-C6 fluorine-containing alkyl" refers to a group in which the H in C1-C6 alkyl skeleton is substituted by one or more fluoro groups, for example, tetrafluoromethane, monofluoromethyl, difluoroethyl, trifluoromethyl and the like. Similarly, the term "C1-C3 fluorine-containing alkyl" refers to a group in which the H in C1-C3 alkyl skeleton is substituted by one or more fluoro groups, for example, monofluoromethyl, difluoroethyl, trifluoromethyl, and the like.

The term "C1-C6 acyl" refers to -C(=O)-H or -C(=O)-C1-C5 alkyl, for example, formyl, acetyl, propionyl, butyryl, and the like.

The term "sulfonyl" refers to $-S(=O)_2-$.

The term "C1-C6 alkylsulfonyl" refers to $-S(=O)_2$-C1-C6 alkyl, for example, methanesulfonyl, ethylsulfonyl, propylsulfonyl, butylsulfonyl and the like.

The terms "alkoxy", "cycloalkoxy" and derivatives thereof refer to any of the above-mentioned alkyl (for example, $C_1$-$C_6$ alkyl, $C_1$-$C_3$ alkyl and the like), cycloalkyl (for example, $C_3$-$C_6$ cycloalkyl), which is attached to the remainder of molecules through oxygen atom (-O-).

From all of the above description, it will be apparent to those skilled in the art that any group whose name is a compounded name, for example, "fluorine-containing oxygen-containing alkyl" shall mean to conventionally construct from the moiety that is derived, such as the oxygen-containing alkyl substituted by the fluoro, wherein the alkyl is as defined above. Similarly, this also applies to "fluorine-containing alkoxy". For another example, "arylamino" shall mean to conventionally construct from the moiety that is derived, such as the amino substituted by the aryl, wherein the aryl is as defined above. Similarly, the meaning of "heteroarylamino" can be understood. Similarly, the meanings of "hydroxysulfonyl", "aminosulfonyl" and the like can be understood.

Similarly, any term such as alkylamino, dialkylamino, alkoxycarbonyl, alkoxycarbonylamino, heterocyclylcarbonyl, heterocyclyl carbonylamino, cycloalkyloxycarbonyl, alkoxyformyl and the like includes groups, wherein alkyl, alkoxy,

aryl, C3-C7 cycloalkyl and heterocyclyl moieties are as defined above.

According to the present invention and unless otherwise provided, any of the above groups may optionally be substituted at any of its free positions by one or more groups, for example by 1 to 6 groups, the groups being independently selected from: halogen atom, nitro, oxo (=O), cyano, C1-C6 alkyl, polyfluorinated alkyl, polyfluorinated alkoxy, alkenyl, alkynyl, hydroxyalkyl, hydroxyalkylamino, hydroxyheterocyclyl, aryl, aryl-alkyl, heteroaryl, heteroaryl-alkyl, heterocyclyl, heterocyclyl-alkyl, C3-C7 cycloalkyl, cycloalkyl-alkyl, alkyl-aryl, alkyl-heteroaryl, alkyl-heterocyclyl, alkyl-cycloalkyl, alkyl-aryl-alkyl, alkyl-heteroaryl-alkyl, alkyl-heterocyclyl-alkyl, alkyl-cycloalkyl-alkyl, alkyl-heterocyclyl-heterocyclyl, heterocyclyl-heterocyclyl, heterocyclyl-alkyl-heterocyclyl, heterocyclyl-alkylamino, alkyl-heterocyclyl-alkyl-amino, hydroxy, alkoxy, aryloxy, heterocyclyloxy, alkyl-heterocyclyloxy, methylenedioxy, alkylcarbonyloxy, arylcarbonyloxy, cycloalkenyloxy, heterocyclylcarbonyloxy, alkyleneaminooxy, carboxy, alkoxycarbonyl, aryloxycarbonyl, cycloalkyloxycarbonyl, heterocyclyloxycarbonyl, amino, ureido, alkylamino, amino-alkylamino, dialkylamino, dialkylamino-heterocyclyl, dialkylamino-alkylamino, arylamino, arylalkylamino, diarylamino, heterocyclylamino, alkyl-heterocyclylamino, alkyl-heterocyclylcarbonyl, formamido, alkylcarbonylamino, arylcarbonylamino, heterocyclylcarbonylamino, alkyl-heterocyclylcarbonylamino, aminocarbonyl, alkylaminocarbonyl, dialkylaminocarbonyl, arylaminocarbonyl, heterocyclylaminocarbonyl, alkoxycarbonylamino, alkoxycarbonylamino-alkylamino, alkoxycarbonylheterocyclyl-alkylamino, alkoxy-aryl-alkyl, hydroxyamino-carbonyl, alkoxyimino, alkylsulfonylamino, arylsulfonylamino, heterocyclylsulfonylamino, formyl, alkylcarbonyl, arylcarbonyl, cycloalkylcarbonyl, heterocyclylcarbonyl, alkylsulfonyl, arylsulfonyl, aminosulfonyl, alkylaminosulfonyl, dialkylaminosulfonyl, arylaminosulfonyl, heterocyclylaminosulfonyl, arylthio, alkylthio, phosphonate and alkylphosphonate.

Further, if appropriate, each of the above substituents may be further substituted by one or more of the above-exemplified groups.

From all of the above description, it will be apparent to those skilled in the art that any group whose name is a compounded name, for example, "fluorine-containing oxygen-containing alkyl" shall mean to conventionally construct from the moiety that is derived, such as the oxygen-containing alkyl substituted by the fluoro, wherein the alkyl is as defined above.

The term "oxygen-containing substituted or unsubstituted 5-membered ring or 6-membered ring" or "nitrogen-containing substituted or unsubstituted 5-membered ring or 6-membered ring" refers to 5- or 6-membered saturated or partially unsaturated carbocyclic ring in which one or more carbon atoms are replaced by oxygen or nitrogen. Non-limiting examples include, for example, pyranyl, pyrrolidinyl, pyrrolinyl, imidazolinyl, imidazolidinyl, pyrazolidinyl, pyrazolinyl, dihydrofuryl, tetrahydrofuryl, 1,3-dioxolanyl, piperidinyl, piperazinyl, morpholinyl, tetrahydropyrrolyl and the like.

As used herein, unless otherwise indicated, the term "prodrug" refers to a derivative that can be hydrolyzed, oxidized or otherwise reacted under biological conditions (in *vitro* or *in vivo*) to provide a compound of the invention. Prodrugs can become active compounds only by carrying out the reaction under biological conditions, or they are inactive in their non-reacted form. Prodrugs can be generally prepared using known methods, for example, those methods described in Burger's Medicinal Chemistry and Drug Discovery (1995) 172-178, 949-982 (Manfred E. Wolff, ed. 5th edition).

As used herein, examples of the term "pharmaceutically acceptable salts of the compounds of formula (I)" are organic acid addition salts formed from organic acids that form pharmaceutically acceptable anions, including but not limited to formate, acetate, propionate, benzoate, maleate, fumarate, succinate, tartrate, citrate, ascorbate, $\alpha$-ketoglutarate, $\alpha$-glycerophosphate, alkyl sulfonate or aryl sulfonate; preferably, said alkyl sulfonate is methyl sulfonate or ethyl sulfonate; said aryl sulfonate is benzenesulfonate or p-toluenesulfonate. Suitable inorganic acid salts may also be formed, including but not limited to hydrochloride, hydrobromide, hydroiodide, nitrate, bicarbonate and carbonate, sulfate or phosphate and the like. Pharmaceutically acceptable salts can be obtained using standard procedures well known in the art, for example, by reacting a sufficient amount of an alkaline compound with a suitable acid that provides a pharmaceutically acceptable anion.

The term "treatment" as used herein generally refers to obtaining the desired pharmacological and/or physiological effect. The effect may be preventive according to complete or partial prevention of disease or its symptoms; and/or may be therapeutic according to partial or complete stabilization or cure of disease and/or side effects due to the disease. The term "treatment" as used herein encompasses any treatment on a patient's disease, including: (a) preventing the disease or symptom that occurs in a patient who is susceptible to the disease or symptom but not yet diagnosed to suffer from the disease; (b) suppressing symptoms of the disease, i.e., stopping its development; or (c) relieving symptoms of the disease, i.e., causing degeneration of the disease or symptom.

According to a specific embodiment of the present invention relating to the compound, a stereoisomer thereof, a prodrug thereof, or a pharmaceutically acceptable salt thereof or a pharmaceutically acceptable solvate thereof, the compound is one of the compounds described in the examples below.

In another aspect, the present invention provides a pharmaceutical composition comprising the compound, a stereoisomer thereof, a prodrug thereof, or a pharmaceutically acceptable salt thereof or pharmaceutically acceptable solvate thereof according to any one of the above embodiments, and a pharmaceutically acceptable carrier, diluent or

excipient.

Methods for preparing a pharmaceutical composition comprising a certain amount of an active ingredient are known or are obvious for a person skilled in the art according to the contents as disclosed in the invention. For example, as described in REMINGTON'S PHARMACEUTICAL SCIENCES, Martin, E.W., ed., Mack Publishing Company, 19th ed. (1995), methods for preparing a pharmaceutical composition comprise incorporating a suitable pharmaceutically acceptable excipient, carrier, diluent amd the like.

The known methods for preparing a pharmaceutical preparation according to the invention include the conventional mixing, dissolving or freeze-drying methods. The compound according to the invention can be used to prepare into a pharmaceutical composition, which is administered to a patient by various routes suitable for the selected administration mode, for example, oral, or parenteral route (intravenous, intramuscular, topical, or subcutaneous route).

Therefore, the compound of the invention in combination with a pharmaceutically acceptable carrier (such as an inert diluent or an assimilable edible carrier) can be administered systemically, e.g., orally. They can be encapsulated into a hard or soft shell gelatin capsule, or pressed into a tablet. For the treatment by oral administration, an active compound may be combined with one or more excipients, and be used in a form of a deglutible tablet, a buccal tablet, a troche, a capsule, an elixir, a suspension, a syrup, a wafer and the like The composition and preparation shall comprise at least 0.1% of an active compound. The ratio of the composition to the preparation can be varied certainly, and the composition may account for about 1 wt% to about 99 wt% of a given unit dosage form. In such a therapeutically active composition, the active compound is in an amount sufficient to obtain an effective dosage level.

A tablet, a troche, a pill, a capsule, and the like may include: a binder, such as tragacanth gum, arabic gum, maize starch or gelatin; an excipient, such as dicalcium phosphate; a disintegrant, such as maize starch, potato starch, and alginic acid etc; a lubricant, such as magnesium stearate; and a sweeting agent, such as sucrose, fructose, lactose or aspartame; or a flavoring agent, such as peppermint, winter green oil or cherry flavor. When the unit dosage form is a capsule, in addition to the above types of materials, it may comprise a liquid carrier, such as vegetable oil or polyethylene glycol. Various other materials may be present as a coating or change the physical form of a solid unit dosage form in other manners. For example, a tablet, a pill or a capsule may be coated with gelatin, wax, shellac or sugar etc. A syrup or elixir may comprise an active compound, sucrose or fructose as a sweeting agent, methyl p-hydroxybenzoate or propyl p-hydroxybenzoate as preservative, a dye and a flavoring agent (such as a cherry flavor or an orange flavor). Certainly, any material for preparing any unit dosage form should be pharmaceutically acceptable and be substantively not toxic in its applied amount. In addition, an active compound may be incorporated into a sustained release preparation and a sustained release device.

An active compound may also be administered intravenously or intraperitoneally by infusion or injection. An aqueous solution of an active compound or a salt thereof may be prepared, optionally, by mixing it with a non-toxic surfactant. A dispersible formulation in glycerol, liquid polyethylene glycol, glycerin triacetate and a mixture thereof and in oil may also be prepared. Under the common conditions of storage and use, the preparations may comprise a preservative in order to suppress the growth of microbes.

A pharmaceutical dosage form suitable for injection or infusion may include a sterile aqueous solution or a dispersible formulation or a sterile powder comprising an active ingredient (optionally encapsulated into a liposome) of an immediate preparation such as a solution or a dispersible formulation suitable for sterile injection or infusion. Under all the conditions, the final dosage form shall be sterile, liquid and stable under the production and storage conditions. A liquid carrier may be a solution or a liquid disperse medium, including, for example, water, ethanol, polyols (such as glycerol, propylene glycol, and liquid macrogol, etc), vegetable oil, a non-toxic glyceride and a suitable mixture thereof. A suitable fluidity may be retained, for example, by the formation of liposome, by retaining the desired particle size in the presence of a dispersing agent, or by using a surfactant. The effect of suppressing microbes can be obtained by various antibacterial agents and antifungal agents (such as paraben, chlorbutol, phenol, sorbic acid, and thiomersal, etc). In many conditions, an isotonizing agent, such as sugar, buffer agent or NaCl, is preferably comprised. By the use of a composition of delayed absorbents (e.g., aluminium monostearate and gelatin), an extended absorption of an injectable composition can be obtained.

A sterile injectable solution can be prepared by mixing a desired amount of an active compound in a suitable solvent with the desired various other ingredients as listed above, and then performing filtration and sterilization. In the case of a sterile powder for the preparation of a sterile injectable solution, the preferred preparation method is vacuum drying and freeze drying techniques, which will result in the production of the powder of the active ingredient and any other desired ingredient present in the previous sterile filtration solution.

A useful solid carrier includes crushed solid (such as talc, clay, microcrystalline cellulose, silicon dioxide, and aluminum oxide etc). A useful liquid carrier includes water, ethanol or ethylene glycol or water-ethanol/ ethylene glycol mixture, in which the compound of the invention may be dissolved or dispersed in an effective amount, optionally, with the aid of a non-toxic surfactant. An adjuvant (such as a flavor) and an additional antimicrobial agent may be added to optimize the property for a given use.

A thickener (such as synthetic polymer, fatty acid, fatty acid salt and ester, fatty alcohol, modified cellulose or

modified inorganic material) may also be used with a liquid carrier to form a coatable paste, gel, ointment, soap and the like, and be directly applied to the skin of a user.

A therapeutically effective amount of a compound or an active salt or derivative thereof not only depends on the specific salt selected, but also depends on the administration mode, the nature of the disease to be treated and the age and state of a patient, and finally depends on the decision made by an attending physician or a clinical physician.

Above preparation may be present in a unit dosage form, which is a physical dispersion unit comprising a unit dose, suitable for administration to a human body and other mammalian body. A unit dosage form may be capsule(s) or tablet(s). Depending on the particular treatment involved, the amount of an active ingredient in a unit dose may be varied or adjusted between about 0.1 and about 1000 mg or more.

In addition, the present invention further includes use of various new drug dosage forms such as milk liposomes, microspheres and nanospheres, for example, medicaments prepared with the use of a particulate dispersion system including polymeric micelles, nanoemulsions, submicroemulsions, microcapsules, microspheres, liposomes and niosomes (also known as nonionic surfactant vesicles) and the like.

In another aspect, the present invention further provides a preparation method of the compounds according to any of the above embodiments, comprising the following steps:

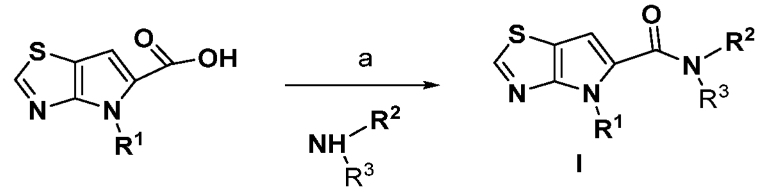


reaction conditions: (a) amide condensation reaction under alkaline condition (triethylamine, diisopropylethylamine and the like);

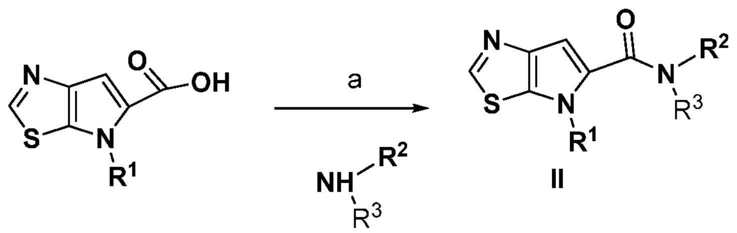


reaction conditions: (a) amide condensation reaction under alkaline condition (triethylamine, diisopropylethylamine and the like).

In another aspect, the present invention further provides use of the compound according to any one of the above embodiments, a stereoisomer thereof, a prodrug thereof, or a pharmaceutically acceptable salt thereof or a pharmaceutically acceptable solvate thereof, and a pharmaceutical composition comprising the compound in the manufacture of a medicament for treating tumors, wherein the tumor includes but not limited to: gastric cancer, liver cancer, blood tumor, osteosarcoma, prostate cancer, breast cancer, lung cancer.

## Experimental Section

Regarding the examples described below, the compounds of the present invention are synthesized using the methods described herein or other methods well known in the art.

## General methods of purification and analysis

Thin layer chromatography was carried out on a silica gel GF254 precoated plate (Qingdao Marine Chemical Plant). Column chromatography was carried out by silica gel (300-400 mesh, Yantai Zhifu Huangwu Silica Gel Development Test Factory) under medium pressure or by a pre-packed silica gel cartridge (ISCO or Welch) with the use of an ISCO Combiflash Rf200 rapid purification system. The ingredient was visualized by UV light ($\lambda$: 254 nm) or iodine vapor. When necessary, the compound was prepared by preparative HPLC and purified by a Waters Symmetry C18 (19 x 50 mm, 5 $\mu$m) column or a Waters X Terra RP 18 (30 x 150 mm, 5 $\mu$m) column, wherein a Waters preparative HPLC 600 equipped with a 996 Waters PDA detector and Micromass mod. ZMD single quadrupole mass spectrometry

(electrospray ionization, cationic mode) were used. Method 1: Phase A: 0.1% TFA/MeOH 95/5; Phase B: MeOH/$H_2O$ 95/5. Gradient: proceeding from 10% to 90% B for 8 min, keeping at 90% B for 2 min; flow rate 20 mL/min. Method 2: Phase A: 0.05% $NH_4OH$/MeOH 95/5; Phase B: MeOH/$H_2O$ 95/5. Gradient: proceeding from 10% to 100% B for 8 min, keeping at 100% B for 2 min. Flow rate 20 mL/min.

$^1H$-NMR spectra were recorded via a Bruker Avance 600 spectrometer (for $^1H$) operated at 600 MHz. The tetramethylsilane signal was used as a reference ($\delta$= 0 ppm). Chemical shift ($\delta$) was reported in parts per million (ppm) and coupling constant (J) in Hz. The following abbreviations were used for peak splitting: s = singlet; br. s. = broad signal; d = doublet; t = triplet; m = multiplet; dd = doublet of doublets. Electrospray (ESI) mass spectra were obtained via Finnigan LCQ ion trap.

Unless otherwise indicated, all final compounds were homogeneous (with purity not less than 95%), as determined by high performance liquid chromatography (HPLC). HPLC-UV-MS analysis for evaluation of compound purity was performed by combining an ion trap MS device and an HPLC system SSP4000 (Thermo Separation Products) equipped with an autosampler LC Pal (CTC Analytics) and a UV6000LP diode array detector (UV detection 215-400 nm). Device control, data acquisition and processing were performed with Xcalibur 1.2 software (Finnigan). HPLC chromatography was carried out at room temperature and at a flow rate of 1 mL/min using a Waters X Terra RP 18 column (4.6 x 50 mm; 3.5 $\mu$m). Mobile phase A was ammonium acetate 5 mM buffer (pH 5.5 with acetic acid): acetonitrile 90:10, mobile phase B was ammonium acetate 5 mM buffer (pH 5.5 with acetic acid): acetonitrile 10:90; proceeding at a gradient of 0 to 100% B for 7 min and then keeping at 100% B for 2 min before rebalancing.

Reagent purification was carried out in accordance with the book Purification of Laboratory Chemicals (Perrin, D. D., Armarego, W. L. F. and Perrins Eds, D. R.; Pergamon Press: Oxford, 1980). Petroleum ether was 60-90°C fraction, ethyl acetate, methanol, dichloromethane were all analytically pure.

The abbreviations hereinafter have the following meanings:

HPLC: high performance liquid chromatography

A. TFA: trifluoroacetic acid
B. HATU: O-(7-azabenzotriazolyl)-N,N,N',N'-tetramethyluronium hexafluorophosphate
C. DIEA: N,N-diisopropylethylamine
D. EDCI·HCl: 1-ethyl-(3-dimethylaminopropyl) carbodiimide hydrochloride

HOBt: 1-hydroxybenzotriazole
DCM: dichloromethane
MsCl: methanesulfonyl chloride
rt: room temperature
DMF: N,N-dimethylformamide
Zn: zinc
UV: ultraviolet
DMSO: dimethyl sulfoxide
Methanol
DMAP: 4-dimethylamino pyridine

Mode of carrying out the invention

The embodiments of the present invention are described in detail below by way of specific examples, but in any case they cannot be construed as limiting the present invention.

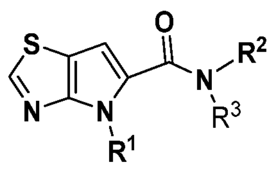
I

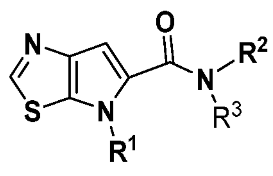
II

wherein, synthetic scheme of compound I is:

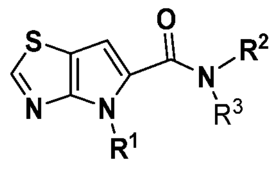

I

Method I:

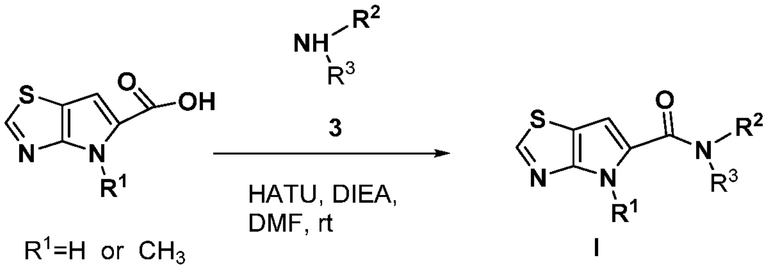


I

$R^1$=H or $CH_3$

1

Preparation of compound I:

Method 1:

Compound 1 (0.2mmol) was dissolved in N,N-dimethylformamide, to which were added HATU (0.3 mmol), DIEA (0.8 mmol), and then added compound 3 (0.2 mmol) with stirring at room temperature. The reaction was carried out at room temperature overnight. The reaction system was extracted with water/ethyl acetate (3 x 15 mL), then the organic phase was washed with saturated sodium chloride solution, dried with anhydrous sodium sulfate, concentrated, and separated by silica gel column chromatography (dichloromethane/methanol) to obtain compound I.

Method 2:

Compound 1 (0.2mmol) was dissolved in N,N-dimethylformamide, to which were added HATU(0.3 mmol), DIEA (0.8 mmol), and then added compound 3 (0.2 mmol) with stirring at room temperature. The reaction was carried out at room temperature overnight. The reaction system was extracted with water/ethyl acetate (3 x 15 mL), then the organic phase was washed with saturated sodium chloride solution, dried with anhydrous sodium sulfate, concentrated, purified by reverse-phase preparative HPLC (using 0.35% trifluoroacetic acid-containing aqueous solution and methanol as mobile phase), and concentrated in vacuum to obtain compound I.

Method II:

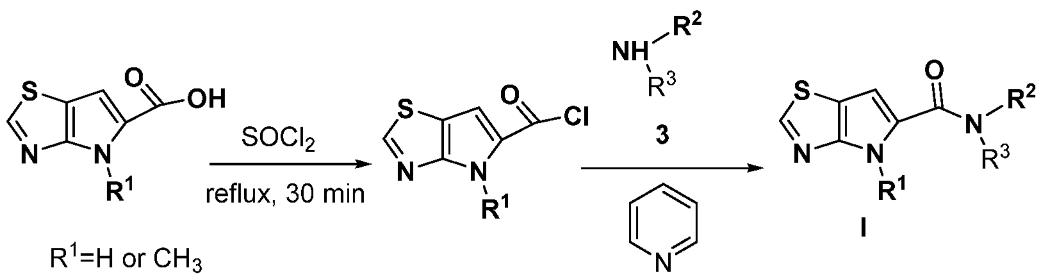


I

$R^1$=H or $CH_3$

**1**

Preparation of compound 2:

Compound 1 (0.5mmol) was dissolved in thionyl chloride (5 mmol), and refluxed for 30 min. After complete reaction, the reaction system was cooled to room temperature, concentrated and dried on vacuum pump to obtain compound 2.

Preparation of compound I:

Method 1:

Compound 2 (0.3mmol) was dissolved in 0.5mL of pyridine, and then added slowly to pyridine solution of compound 3 (0.2mmol) in an ice bath, followed by returning to room temperature and reacting for 4 h. The reaction system was concentrated, and extracted with 1N HCl/ethyl acetate (3 x 15 mL), then the organic phase was washed with saturated sodium chloride solution, dried with anhydrous sodium sulfate, concentrated, and separated by silica gel column chromatography (dichloromethane/methanol) to obtain compound I.

Method 2:

Compound 2 (0.3mmol) was dissolved in 0.5mL of pyridine, and then added slowly to pyridine solution of compound 3 (0.2mmol) in an ice bath, followed by returning to room temperature and reacting for 4 h. The reaction system was concentrated, and extracted with 1N HCl/ethyl acetate (3 x 15 mL), then the organic phase was washed with saturated sodium chloride solution, dried with anhydrous sodium sulfate, concentrated, purified by reverse-phase preparative HPLC (using 0.35% trifluoroacetic acid-containing aqueous solution and methanol as mobile phase), and concentrated in vacuum to obtain compound I.

The synthesis of the compounds of examples is described in detail below.

1. Compound I-1:

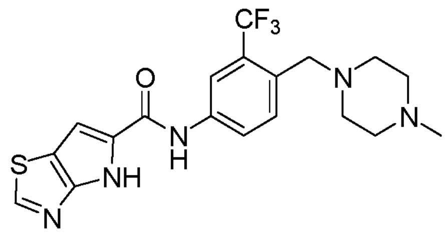

Compound

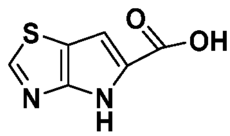

(0.2mmol, 33.6 mg) (CAS: 1007386-72-2, Sandia, Shanghai) was dissolved in N,N-dimethylformamide, to which were added HATU (0.3 mmol, 114.1 mg), DIEA (0.8 mmol, 0.132 mL), and then added compound

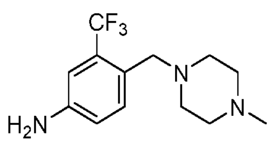

(0.2 mmol, 54.7 mg) (CAS: 694499-26-8, Sandia, Shanghai) with stirring at room temperature. The reaction was carried out at room temperature overnight. The reaction system was extracted with water/ethyl acetate (3 x 15 mL), then the organic phase was washed with saturated sodium chloride solution, dried with anhydrous sodium sulfate, concentrated, purified by reverse-phase preparative HPLC (using 0.35% trifluoroacetic acid-containing aqueous

solution and methanol as mobile phase), and concentrated in vacuum to obtain compound 1-1 (5.1 mg, 4.7%).

2. Compound 1-2:

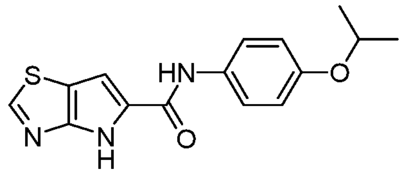

Using compounds

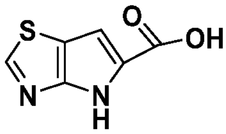

and

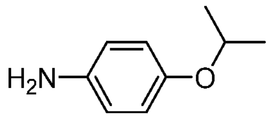

(CAS: 7664-66-6, Energy, Shanghai) as raw materials, compound 1-2 was synthesized by a method similar to that for the synthesis of compound I-1.

3. Compound 1-3:

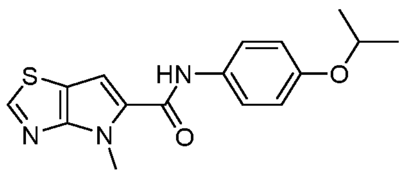

The synthesis of compound

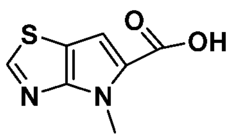

:

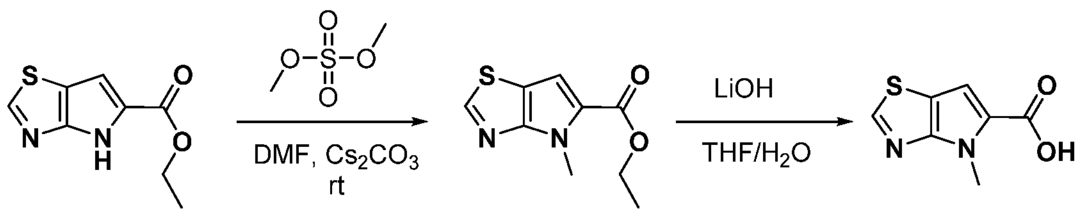


E. Ethyl 4H-pyrrolo[2,3-d]thiazole-5-carboxylate (4 mmol, 784.92 mg) (CAS:238749-53-6, Sandia, Shanghai), cesium carbonate ($Cs_2CO_3$) (4.8 mmol, 1.563 g) were dissolved in 15 mL of N,N-dimethylformamide (DMF). Then, dimethyl sulfate (4.8 mmol, 605.43 mg) was added dropwise slowly at 0°C, followed by returning to room temperature and reacting overnight. The reaction system was extracted with water/ethyl acetate, then the organic phase was washed with saturated sodium chloride solution, dried with anhydrous sodium sulfate, and concentrated to obtain 704 mg of ethyl 4-methyl-4H-pyrrolo[2,3-d]thiazole-5-carboxylate.

Ethyl 4-methyl-4H-pyrrolo[2,3-d]thiazole-5-carboxylate (3 mmol, 630.75 mg) was dissolved in 12 mL of tetrahydrofuran, to which was added 4 mL of 1 N lithium hydroxide solution, followed by reacting at 52°C for 7 h. After removing most of the solvent by concentration under reduced pressure, ice water was added, and the pH was adjusted to weak acidity with 1 N dilute hydrochloric acid, to precipitate a solid. After centrifugation, the solid was washed with water, and the precipitate was collected to obtain 480 mg of 4-methyl-4H-pyrrolo[2,3-d]thiazole-5-carboxylic acid.

Using compounds

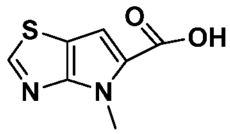

and

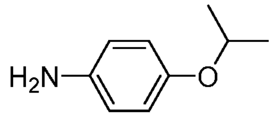

as raw materials, compound 1-3 was synthesized by a method similar to that for the synthesis of compound I-1.

4. Compound 1-4:

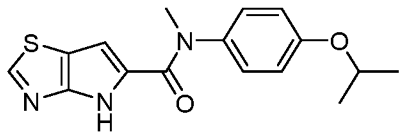

The synthesis of intermediate:

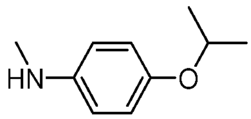

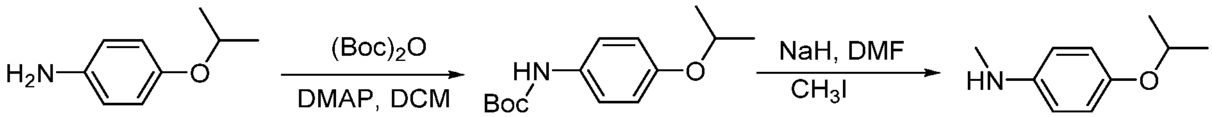


p-isopropoxyaniline (3 mmol, 453.6 mg), DMAP (0.3 mmol, 45 mg) were dissolved in 5 mL of DCM. $(Boc)_2O$ (3 mmol, 654 mg) was added dropwise slowly at 0°C, followed by returning to room temperature and reacting for 20 h. The reaction system was extracted with water/dichloromethane, then the organic phase was washed with saturated sodium chloride solution, dried with anhydrous sodium sulfate, and concentrated to obtain 720 mg of tert-butyl (4-isopropoxyphenyl)carbamate. Tert-butyl (4-isopropoxyphenyl)carbamate (1.26 mmol, 316.25 mg), NaH (2.52 mmol, 60.48 mg) were dissolved in 3 mL of N,N-dimethylformamide. Methyl iodide ($CH_3I$) (1.26 mmol, 178.84 mg) was added dropwise slowly at 0°C, followed by returning to room temperature and reacting for 20 h. The reaction system was extracted with water/ethyl acetate, then the organic phase was washed with saturated sodium chloride solution, dried with anhydrous sodium sulfate, and concentrated to obtain 250 mg of 4-isopropoxy-N-methylaniline.
Using compounds

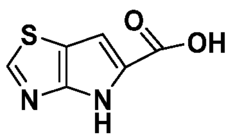

and

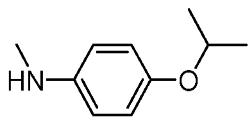

as raw materials, compound 1-4 was synthesized by a method similar to that for the synthesis of compound I-1.

5. Compound 1-5:

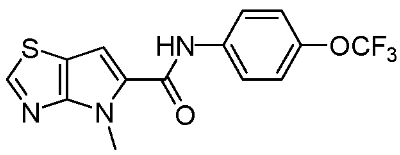

Using compounds

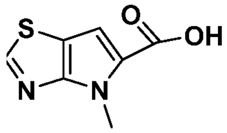
and
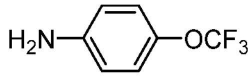

(CAS: 461-82-5, Energy, Shanghai) as raw materials, compound 1-5 was synthesized by a method similar to that for the synthesis of compound I-1.

6. Compound 1-6:

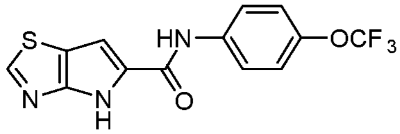

Using compounds

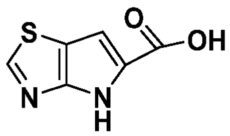
and
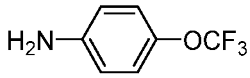

as raw materials, compound 1-6 was synthesized by a method similar to that for the synthesis of compound I-1.

7. Compound 1-7:

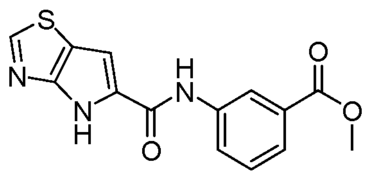

Using compounds

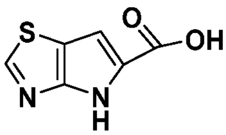
and
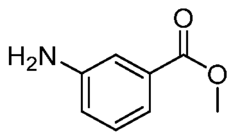

(CAS: 4518-10-9, Energy, Shanghai) as raw materials, compound 1-7 was synthesized by a method similar to that for the synthesis of compound I-1.

8. Compound 1-8:

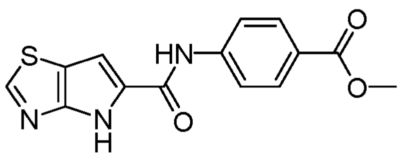

Using compounds

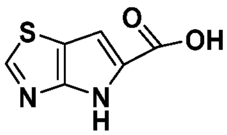

and

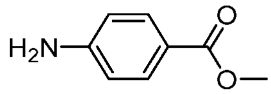

(CAS: 619-45-4, Energy, Shanghai) as raw materials, compound 1-8 was synthesized by a method similar to that for the synthesis of compound I-1.

9. Compound 1-9:

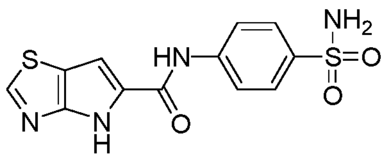

Using compounds

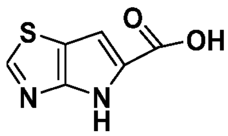

and

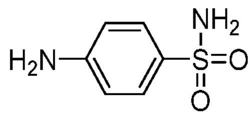

(CAS: 63-74-1, Energy, Shanghai) as raw materials, compound 1-9 was synthesized by a method similar to that for the synthesis of compound I-1.

10. Compound I-10:

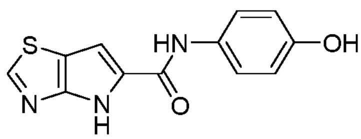

Using compounds

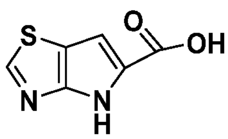

and

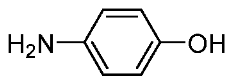

(CAS: 123-30-8, Energy, Shanghai) as raw materials, compound I-10 was synthesized by a method similar to that for the synthesis of compound I-1.

11. Compound I-11:

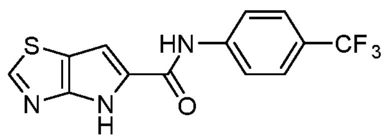

Using compounds

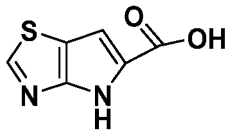 and 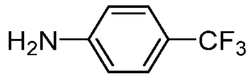

(CAS: 455-14-1, Energy, Shanghai) as raw materials, compound I-11 was synthesized by a method similar to that for the synthesis of compound I-1.

12. Compound 1-12:

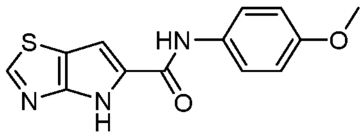

Using compounds

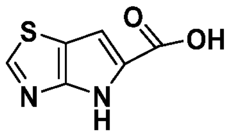 and 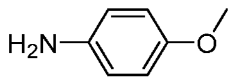

(CAS: 104-94-9, Energy, Shanghai) as raw materials, compound 1-12 was synthesized by a method similar to that for the synthesis of compound I-1.

13. Compound 1-13:

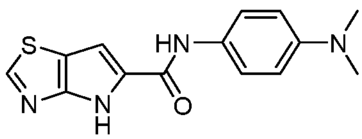

Using compounds

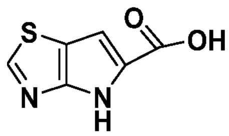 and 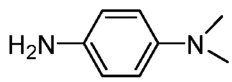

(CAS: 99-98-9, Energy, Shanghai) as raw materials, compound 1-13 was synthesized by a method similar to that for the synthesis of compound I-1.

14. Compound 1-14:

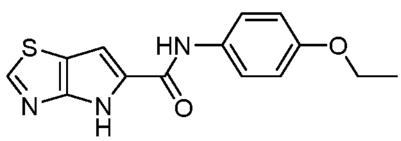

Using compounds

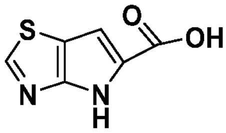
and
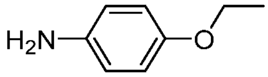

(CAS: 156-43-4, Energy, Shanghai) as raw materials, compound 1-14 was synthesized by a method similar to that for the synthesis of compound I-1.

15. Compound 1-15:

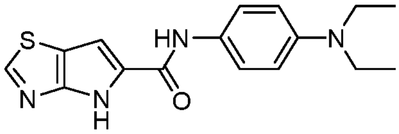

Using compounds

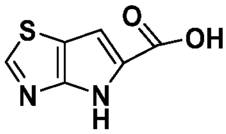
and
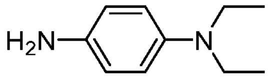

(CAS: 93-05-0, Energy, Shanghai) as raw materials, compound 1-15 was synthesized by a method similar to that for the synthesis of compound I-1.

16. Compound 1-16:

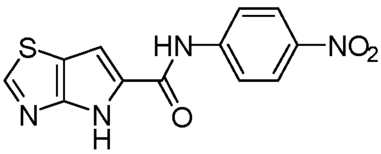

Using compounds

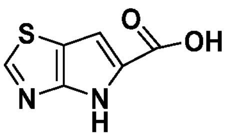
and
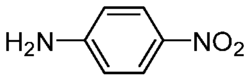

(CAS: 100-01-6, Energy, Shanghai) as raw materials, compound 1-16 was synthesized by a method similar to that for the synthesis of compound I-1.

17. Compound 1-17:

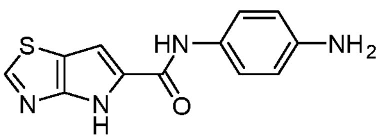

Compound 16 (0.1 mmol, 28.8 mg) and Zn (1 mmol, 65 mg) were dissolved in 1 mL of EtOH, to which was then added 0.2 mL of $NH_4Cl$ (0.4 mmol, 21.3 mg) aqueous solution dropwise. The reaction system was heated to 50°C, and reacted overnight. The reaction system was filtered through silica gel, extracted with water/ethyl acetate, then

the organic phase was washed with saturated sodium chloride solution, dried with anhydrous sodium sulfate, concentrated, and separated by silica gel column chromatography (dichloromethane/methanol) to obtain compound 1-17.

18. Compound 1-18:

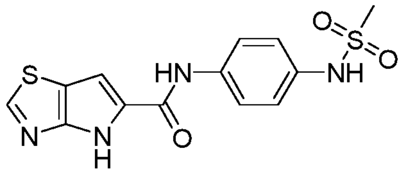

Using compounds

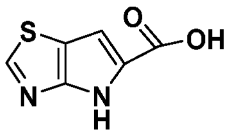

and

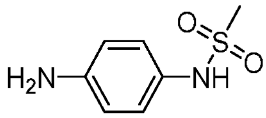

(CAS: 53250-82-1, Energy, Shanghai) as raw materials, compound 1-18 was synthesized by a method similar to that for the synthesis of compound I-1.

19. Compound 1-19:

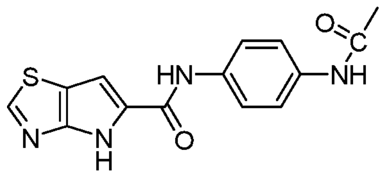

Using compounds

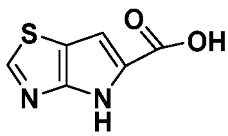

and

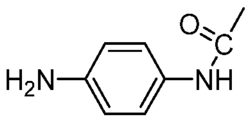

(CAS: 122-80-5, Energy, Shanghai) as raw materials, compound 1-19 was synthesized by a method similar to that for the synthesis of compound I-1.

20. Compound 1-20:

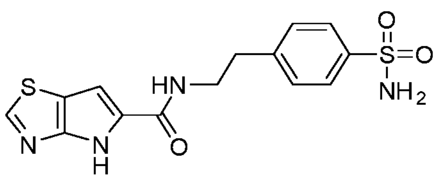

Using compounds

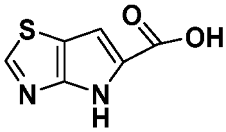 and 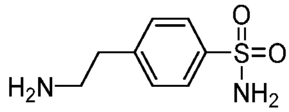

(CAS: 35303-76-5, Energy, Shanghai) as raw materials, compound 1-20 was synthesized by a method similar to that for the synthesis of compound I-1.

21. Compound 1-21:

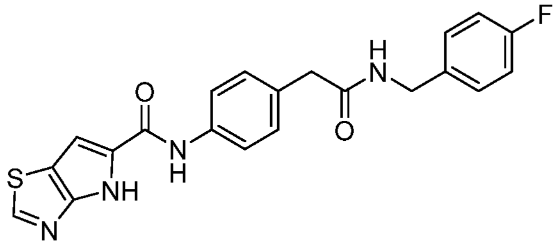

The synthesis of intermediate:

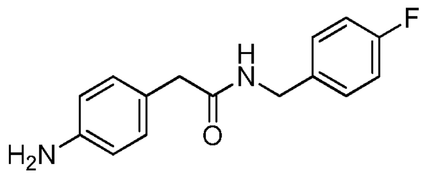

4-aminophenylacetic acid (6.7 mmol, 1.01 g) was dissolved in 15 mL of N,N-dimethylformamide, to which were added EDCI·HCl (10.05 mmol, 1.926 g), HOBt (7.37 mmol, 995.8 mg), DIEA (26.8mmol, 4.67 mL). After stirring at room temperature for 30 min, p-fluorobenzylamine (6.7 mmol, 838.4 mg) was added, the reaction was carried out at room temperature overnight. The reaction system was extracted with water/ethyl acetate, then the organic phase was washed with saturated sodium chloride solution, dried with anhydrous sodium sulfate, concentrated, and separated by silica gel column chromatography (dichloromethane/methanol) to obtain 520 mg of the above intermediate. Compound

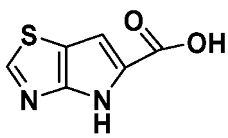

(0.2mmol, 33.6 mg) was dissolved in N,N-dimethylformamide, to which were added HATU(0.3 mmol, 114.1 mg), DIEA (0.8 mmol, 0.132 mL), and then added the above-synthesized intermediate (0.2 mmol, 51.6mg) with stirring at room temperature. The reaction was carried out at room temperature overnight. The reaction system was extracted with water/ethyl acetate (3 x 15 mL), then the organic phase was washed with saturated sodium chloride solution, dried with anhydrous sodium sulfate, concentrated, purified by reverse-phase preparative HPLC (using 0.35% trifluoroacetic acid-containing aqueous solution and methanol as mobile phase), and concentrated in vacuum to obtain compound 1-21 (26.1 mg, 32%).

22. Compound 1-22:

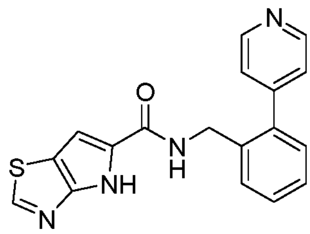

Intermediate

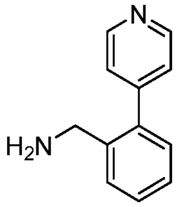

was synthesized by a method similar to that for the synthesis of intermediate

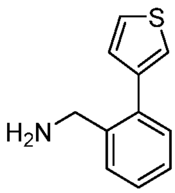

in 24 below. Using compound

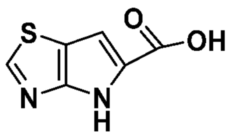

and the above intermediate as raw materials, compound 1-22 was synthesized by a method similar to that for the synthesis of compound I-1.

23. Compound 1-23:

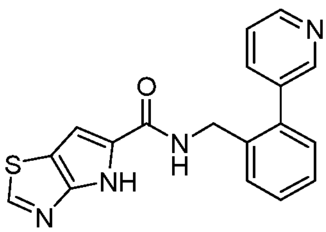

Intermediate

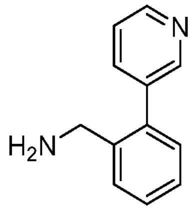

was synthesized by a method similar to that for the synthesis of intermediate

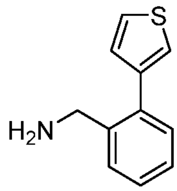

in 24 below.
Using compound

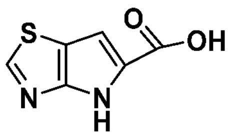

and the above intermediate as raw materials, compound 1-23 was synthesized by a method similar to that for the synthesis of compound I-1.

24. Compound 1-24:

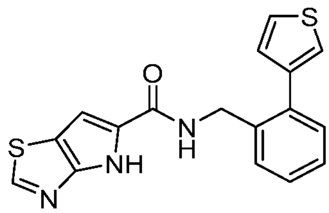

Intermediate

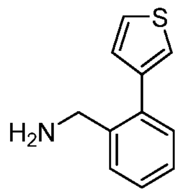

was synthesized with a reference to the document: European Journal of Medicinal Chemistry, 87, 529-539; 2014.
Compound

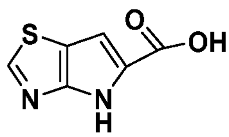

(0.2mmol, 33.6 mg) was dissolved in N,N-dimethylformamide, to which were added HATU(0.3 mmol, 114.1 mg), DIEA (0.8 mmol, 0.132 mL), and then added the above-synthesized intermediate (0.2 mmol, 37.8 mg) with stirring at room temperature. The reaction system was extracted with water/ethyl acetate (3 x 15 mL), then the organic phase was washed with saturated sodium chloride solution, dried with anhydrous sodium sulfate, concentrated, and separated by silica gel column chromatography (dichloromethane/methanol) to obtain compound 1-24 (23.5 mg, 34.6%).

25. Compound 1-25:

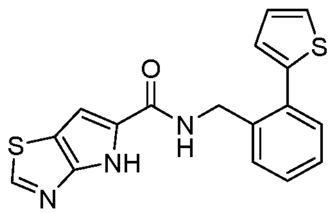

Intermediate

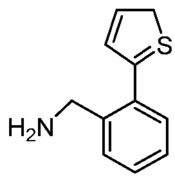

was synthesized by a method similar to that for the synthesis of intermediate

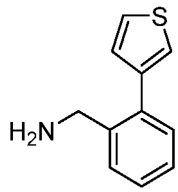

in 24 above.
Using compound

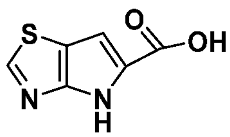

and the above intermediate as raw materials, compound 1-25 was synthesized by a method similar to that for the synthesis of compound I-1.

26. Compound 1-26:

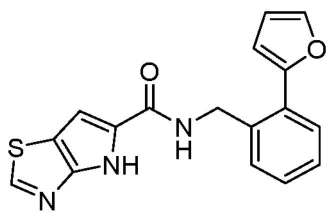

Intermediate

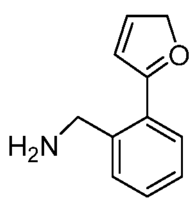

was synthesized by a method similar to that for the synthesis of intermediate

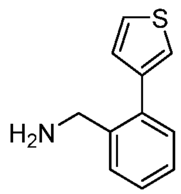

in 24 above.
Using compound

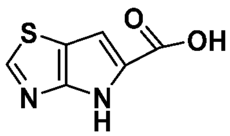

and the above intermediate as raw materials, compound 1-26 was synthesized by a method similar to that for the synthesis of compound I-1.

27. Compound 1-27:

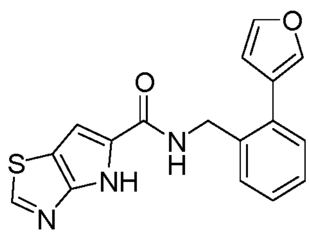

Intermediate

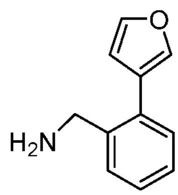

was synthesized by a method similar to that for the synthesis of intermediate

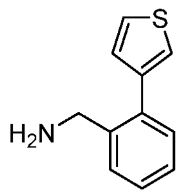

in 24 above.

Using compound

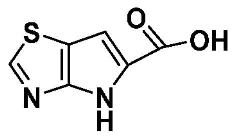

and the above intermediate as raw materials, compound 1-27 was synthesized by a method similar to that for the synthesis of compound I-1.

28. Compound 1-28:

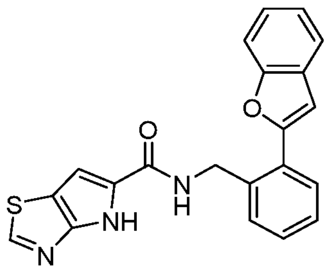

Intermediate

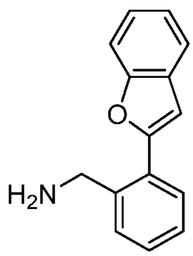

was synthesized by a method similar to that for the synthesis of intermediate

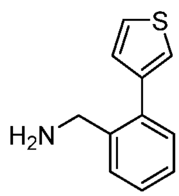

in 24 above.

Using compound

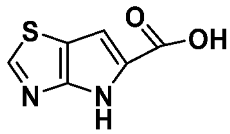

and the above intermediate as raw materials, compound 1-28 was synthesized by a method similar to that for the synthesis of compound I-1.

29. Compound 1-29:

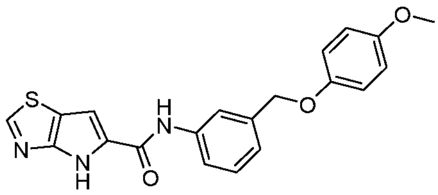

Intermediate

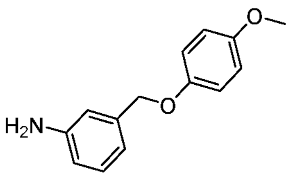

was synthesized by a method similar to that for the synthesis of intermediate in 31 below.
Using compound

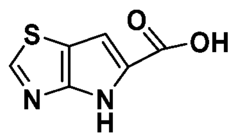

and the above intermediate as raw materials, compound 1-29 was synthesized by a method similar to that for the synthesis of compound I-1.

30. Compound 1-30:

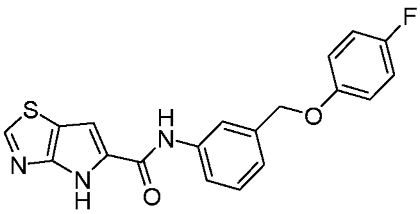

Intermediate

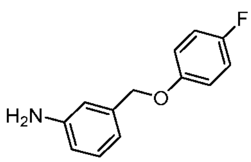

was synthesized by a method similar to that for the synthesis of intermediate in 31 below.
Using compound

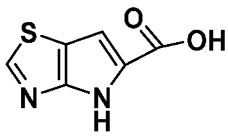

and the above intermediate as raw materials, compound 1-30 was synthesized by a method similar to that for the synthesis of compound I-1.

31. Compound 1-31:

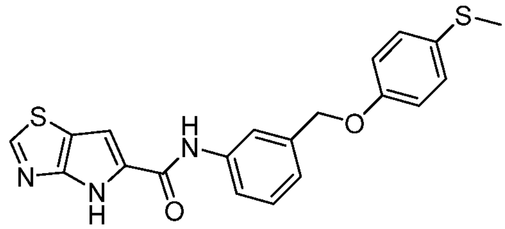

The synthesis of intermediate:

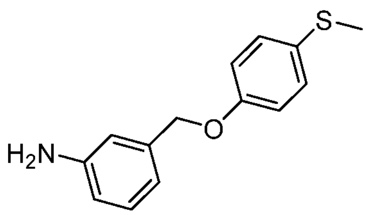

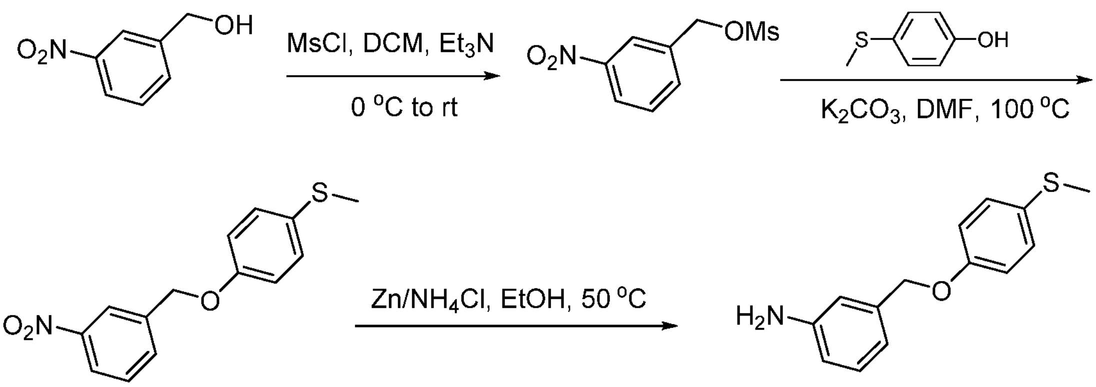


m-nitrobenzyl alcohol (20 mmol, 3.06 g) and triethylamine ($Et_3N$) (60 mmol, 8.3 mL) were dissolved in 50 mL of dichloromethane (DCM), followed by stirring at 0°C for 15 min. Methylsulfonyl chloride (MsCl) (30 mmol, 2.32 mL) was added dropwise slowly at 0°C, followed by gradually returning to room temperature and reacting for 5 h. The reaction system was extracted with water/dichloromethane, then the organic phase was washed with saturated sodium chloride solution, dried with anhydrous sodium sulfate, and concentrated to obtain 1.8 g of 3-nitrobenzyl mesylate.

3-nitrobenzyl mesylate (2.16 mmol, 500 mg), $K_2CO3$ (4.32 mmol, 596.2 mg) were dissolved in 8 mL of N,N-dimethylformamide (DMF), to which was added 4-(methylthio)phenol (3.24 mmol, 454.2 mg), followed by reacting at 100°C for 2 h. The reaction system was extracted with water/ethyl acetate, then the organic phase was washed with saturated sodium chloride solution, dried with anhydrous sodium sulfate, concentrated, and separated by silica gel column chromatography to obtain 470 mg of methyl (4-((3-nitrobenzyl)oxy)phenyl)sulfane.

Methyl (4-((3-nitrobenzyl)oxy)phenyl)sulfane (2 mmol, 614.6 mg) and Zn (10 mmol, 650 mg) were dissolved in 10 mL of EtOH, to which was then added 2 mL of $NH_4Cl$ (4 mmol, 213.9 mg) aqueous solution dropwise. The reaction system was heated to 50°C, and reacted overnight. The reaction system was filtered through silica gel, extracted with water/ethyl acetate, then the organic phase was washed with saturated sodium chloride solution, dried with anhydrous sodium sulfate, and concentrated to obtain 502 mg of the intermediate 3-((4-(methylthio)phenoxy)methyl)aniline.
Compound

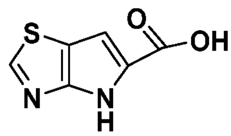

(0.2mmol, 33.6 mg) was dissolved in N,N-dimethylformamide, to which were added HATU(0.3 mmol, 114.1 mg), DIEA (0.8 mmol, 0.132 mL), and then added the above-synthesized intermediate 3-((4-methylthio)phenoxy)methyl)aniline (0.2 mmol, 49.1 mg) with stirring at room temperature. The reaction system was extracted with water/ethyl acetate (3 x 15 mL), then the organic phase was washed with saturated sodium chloride solution, dried with anhydrous sodium sulfate, concentrated, and separated by silica gel column chromatography (dichloromethane/methanol) to obtain compound 1-31 (12.8 mg, 16.2%).

32. Compound 1-32:

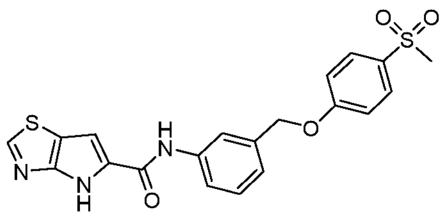

The synthesis of intermediate:

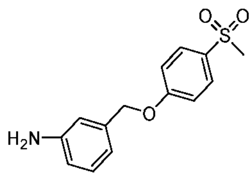

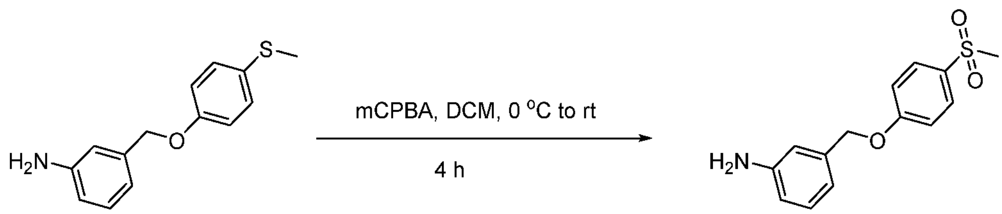


3-((4-methylthio)phenoxy)methyl)aniline (4 mmol, 1.1 g) was dissolved in 15 mL of dichloromethane (DCM), to which was added m-chloroperoxybenzoic acid (mCPBA) (12 mmol, 2.07 g) at 0°C, followed by reacting at room temperature for 4 h. The reaction system was extracted with water/ethyl acetate, then the organic phase was washed with saturated sodium chloride solution, dried with anhydrous sodium sulfate, concentrated, and separated by silica gel column chromatography to obtain 1.2 g of the above intermediate.
Using compound

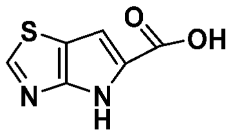

and the above intermediate as raw materials, compound 1-32 was synthesized by a method similar to that for the synthesis of compound I-1.

33. Compound 1-33:

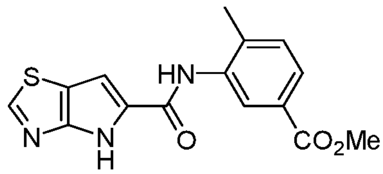

Using compounds

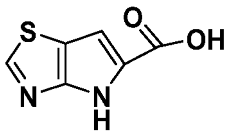

and

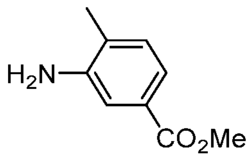

(CAS: 18595-18-1, Energy, Shanghai) as raw materials, compound 1-33 was synthesized by a method similar to that for the synthesis of compound I-1.

34. Compound 1-34:

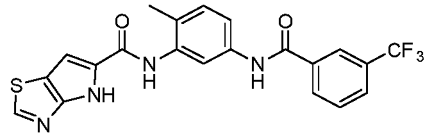

Using compounds

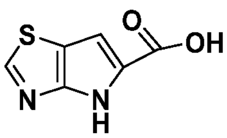

and

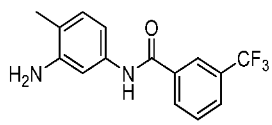

(CAS: 30069-31-9, Sandia, Shanghai) as raw materials, compound 1-34 was synthesized by a method similar to that for the synthesis of compound I-1.

35. Compound 1-35:

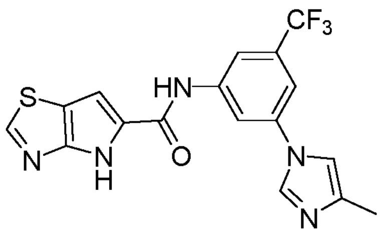

Using compounds

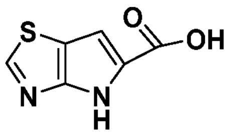

and

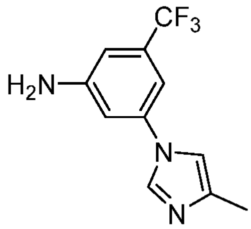

(CAS: 641571-11-1, Energy, Shanghai) as raw materials, compound 1-35 was synthesized by a method similar to that for the synthesis of compound I-1.

36. Compound II-1:

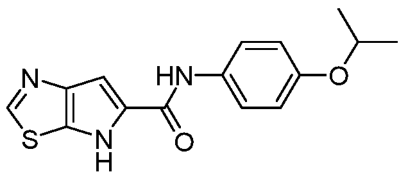

Using compounds

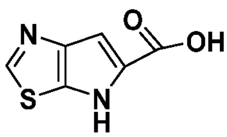

(CAS: 1007386-66-4, Sandia, Shanghai) and

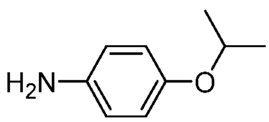

as raw materials, compound II-1 was synthesized by a method similar to that for the synthesis of the specific compound of the above general formula I.

37. Compound II-2:

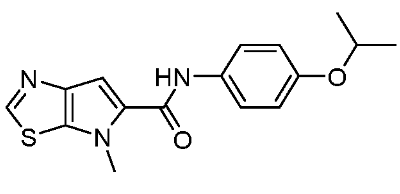

The synthesis of compound

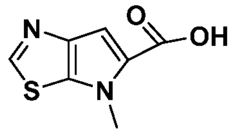
:

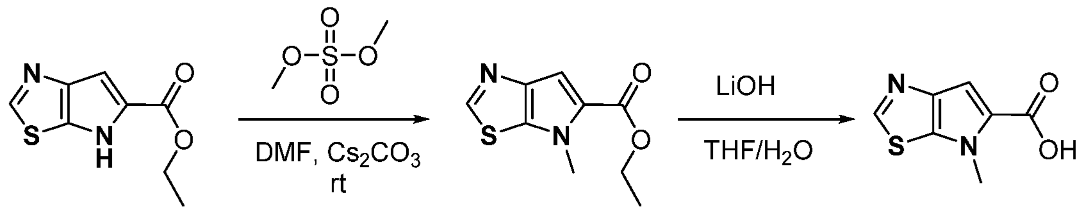


F. Ethyl 4H-pyrrolo[3,2-d]thiazole-5-carboxylate (4 mmol, 784.92 mg) (CAS:75103-40-1, Sandia, Shanghai), cesium carbonate ($Cs_2CO_3$) (4.8 mmol, 1.563 g) were dissolved in 15 mL of N,N-dimethylformamide (DMF). Then, dimethyl sulfate (4.8 mmol, 605.43 mg) was added dropwise slowly at 0°C, followed by returning to room temperature and reacting overnight. The reaction system was extracted with water/ethyl acetate, then the organic phase was washed with saturated sodium chloride solution, dried with anhydrous sodium sulfate, and concentrated to obtain 680 mg of ethyl 4-methyl-4H-pyrrolo[3,2-d]thiazole-5-carboxylate.

Ethyl 4-methyl-4H-pyrrolo[3,2-d]thiazole-5-carboxylate (3 mmol, 630.75 mg) was dissolved in 12 mL of tetrahydrofuran(THF), to which was added 4 mL of 1 N lithium hydroxide solution(LiOH), followed by reacting at 52°C for 7 h. After removing most of the solvent by concentration under reduced pressure, ice water was added, and the pH was adjusted to weak acidity with 1 N dilute hydrochloric acid, to precipitate a solid. After centrifugation, the solid was washed with water, and the precipitate was collected to obtain 471 mg of 4-methyl-4H-pyrrolo[3,2-d]thiazole-5-carboxylic acid.

Using compounds

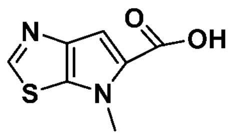
and
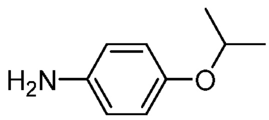

as raw materials, compound II-2 was synthesized by a method similar to that for the synthesis of the specific compound of the above general formula I.

38. Compound II-3:

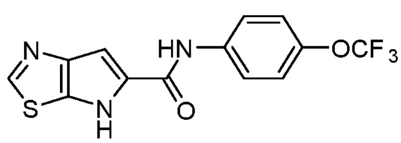

Using compounds

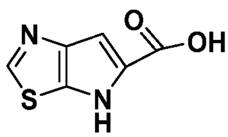
and
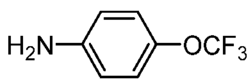

as raw materials, compound II-3 was synthesized by a method similar to that for the synthesis of the specific compound of the above general formula I.

39. Compound II-4:

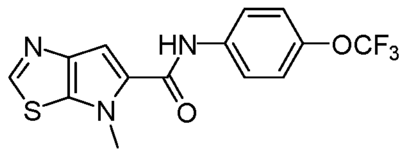

Using compounds

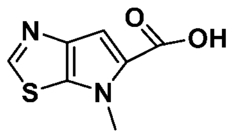

and

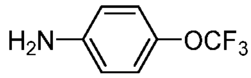

as raw materials, compound II-4 was synthesized by a method similar to that for the synthesis of the specific compound of the above general formula I.

40. Compound II-5:

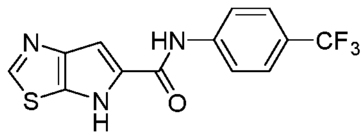

Using compounds

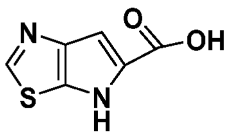

and

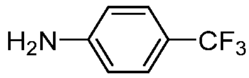

as raw materials, compound II-5 was synthesized by a method similar to that for the synthesis of the specific compound of the above general formula I.

41. Compound II-6:

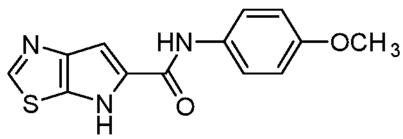

Using compounds

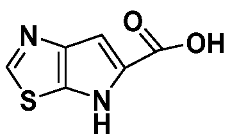

and

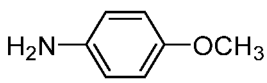

as raw materials, compound II-6 was synthesized by a method similar to that for the synthesis of the specific compound of the above general formula I.

42. Compound II-7:

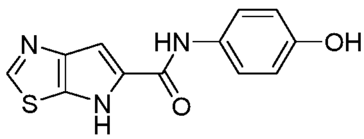

Using compounds

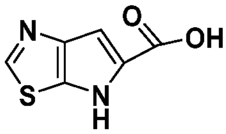

and

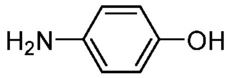

as raw materials, compound II-7 was synthesized by a method similar to that for the synthesis of the specific compound of the above general formula I.

43. Compound II-8:

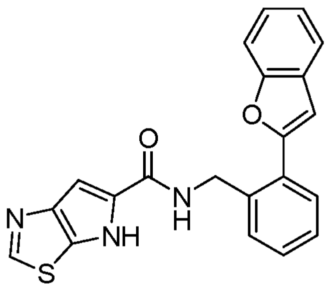

Using compounds

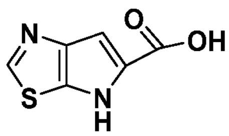

and

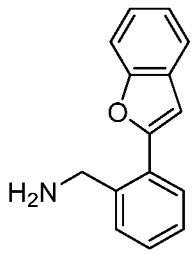

as raw materials, compound II-8 was synthesized by a method similar to that for the synthesis of the specific compound of the above general formula I.

44. Compound II-9:

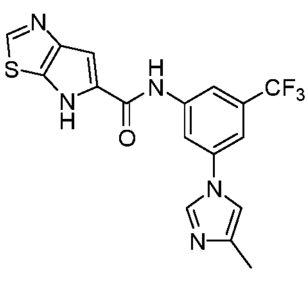

Using compounds

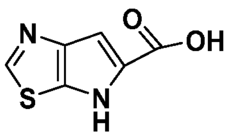
and
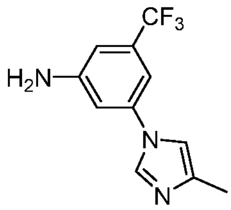

as raw materials, compound II-9 was synthesized by a method similar to that for the synthesis of the specific compound of the above general formula I.

45. Compound II-10:

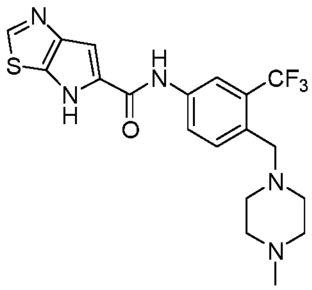

Using compounds

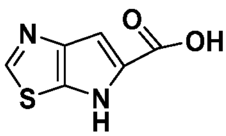
and
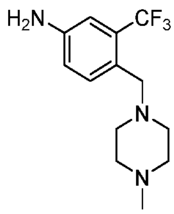

as raw materials, compound II-10 was synthesized by a method similar to that for the synthesis of the specific compound of the above general formula I.

The raw materials used that are not described in the above synthesis are all commercially avaiable.

The table below lists the specific compounds and structure identification data.

Table 1. Structure and characterization of compounds I

| Cmpd ID | Structure | 1H NMR and/or MS data |
|---|---|---|
| I-1 | 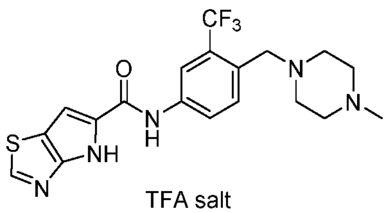 TFA salt | $^1$H NMR (600 MHz, DMSO-$d_6$) δ 12.67 (s, 1H), 10.37 (s, 1H), 9.03 (s, 1H), 8.21 (s, 1H), 8.07 (d, *J* = 8.5 Hz, 1H), 7.71 (d, *J* = 8.5 Hz, 1H), 7.45 (s, 1H), 3.71 (s, 2H), 3.42 (s, 2H), 3.06 (s, 2H), 2.94 (s, 2H), 2.81 (s, 3H), 2.41 (s, 2H). MS (ESI) *m/z*: 424 [M+H]$^+$. |
| I-2 | 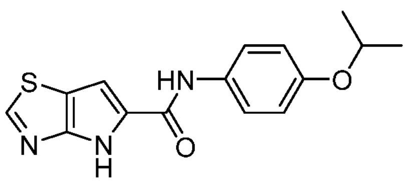 | $^1$H NMR (600 MHz, Chloroform-*d*) δ 10.01 (s, 1H), 8.72 (s, 1H), 7.61 (s, 1H), 7.52 - 7.46 (m, 2H), 6.95 - 6.86 (m, 3H), 4.87 - 3.98 (m, 1H), 1.34 (d, *J* = 6.0 Hz, 6H). MS (ESI) *m/z*: 302 [M+H]$^+$. |

(continued)

| Cmpd ID | Structure | 1H NMR and/or MS data |
|---|---|---|
| I-3 | 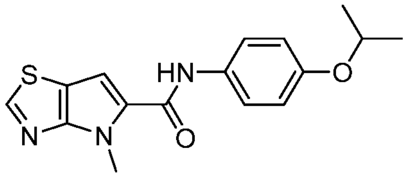 | $^1$H NMR (600 MHz, DMSO-$d_6$) δ 9.91 (d, *J* = 4.3 Hz, 1H), 8.95 (s, 1H), 7.59 - 7.44 (m, 2H), 7.24 (s, 1H), 6.92 - 6.69 (m, 2H), 4.47 (q, *J* = 6.0 Hz, 1H), 4.00 (s, 3H), 1.17 (d, *J* = 6.0 Hz, 6H). MS (ESI) *m/z*: 316 [M+H]$^+$. |
| I-4 | 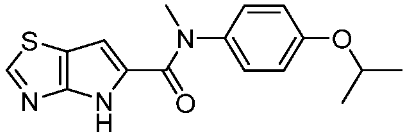 | $^1$H NMR (600 MHz, DMSO-$d_6$) δ 12.40 (s, 1H), 8.87 (s, 1H), 7.34 - 7.15 (m, 2H), 7.08 - 6.87 (m, 2H), 5.28 (s, 1H), 4.85 - 4.46 (m, 1H), 3.32 (s, 3H), 1.30 (d, *J* = 6.0 Hz, 6H). MS (ESI) *m/z*: 316 [M+H]$^+$. |
| I-5 | 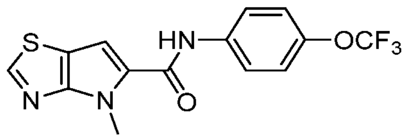 | $^1$H NMR (600 MHz, DMSO-$d_6$) δ 10.26 (s, 1H), 9.04 (s, 1H), 7.87 - 7.74 (m, 2H), 7.36 (s, 1H), 7.33 - 7.30 (m, 2H), 4.06 (s, 3H). MS (ESI) *m/z*: 342 [M+H]$^+$. |
| I-6 | 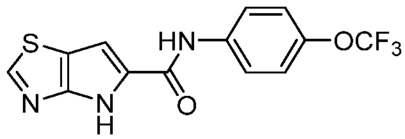 | $^1$H NMR (600 MHz, DMSO) δ 12.68 (s, 1H), 10.21 (s, 1H), 9.02 (s, 1H), 7.89 (d, *J* = 9.1 Hz, 2H), 7.43 (s, 1H), 7.38 (d, *J* = 8.6 Hz, 2H). MS (ESI) *m/z*: 328 [M+H]$^+$. |
| I-7 | 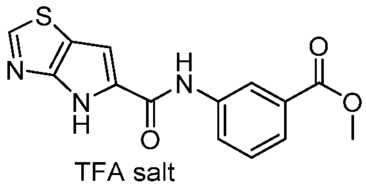 TFA salt | $^1$H NMR (600 MHz, DMSO-$d_6$) δ 12.64 (s, 1H), 10.22 (s, 1H), 9.00 (d, *J* = 0.9 Hz, 1H), 8.40 (t, *J* = 1.9 Hz, 1H), 8.08 (ddd, *J* = 8.2, 2.3, 1.1 Hz, 1H), 7.66 (dt, *J* = 7.8, 1.3 Hz, 1H), 7.49 (t, *J* = 7.9 Hz, 1H), 7.44 (d, *J* = 1.8 Hz, 1H), 3.86 (s, 3H). MS (ESI) *m/z*: 302[M+H]$^+$. |
| I-8 | 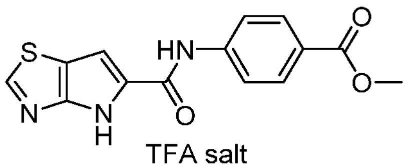 TFA salt | $^1$H NMR (600 MHz, DMSO) δ 12.75 (s, 1H), 10.42 (s, 1H), 9.30 - 8.80 (m, 1H), 7.97 (s, 4H), 7.72 - 7.27 (m, 1H), 3.85 (s, 3H). MS (ESI) *m/z*: 302[M+H]$^+$. |
| I-9 | 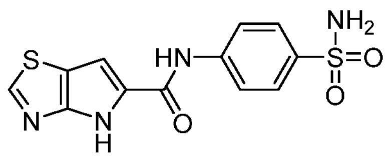 | $^1$H NMR (600 MHz, DMSO) δ 12.69 (s, 1H), 10.31 (s, 1H), 9.01 (s, 1H), 7.92 (d, *J* = 8.7 Hz, 2H), 7.79 (d, *J* = 8.7 Hz, 2H), 7.45 (s, 1H), 7.25 (s, 2H). MS (ESI) *m/z*: 323[M+H]$^+$. |
| I-10 | 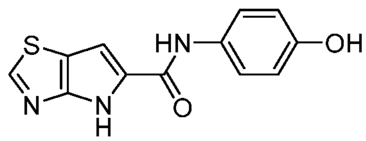 | $^1$H NMR (600 MHz, DMSO) δ 12.54 (s, 1H), 9.81 (s, 1H), 9.24 (s, 1H), 8.98 (s, 1H), 7.55 - 7.49 (m, 2H), 7.34 (s, 1H), 6.78 - 6.73 (m, 2H). MS (ESI) *m/z*: 260[M+H]$^+$. |
| I-11 | 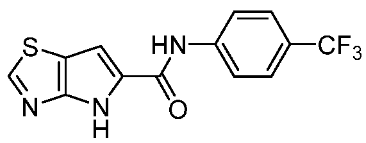 | $^1$H NMR (600 MHz, DMSO) δ 12.72 (s, 1H), 10.34 (s, 1H), 9.04 (s, 1H), 8.01 (d, *J* = 8.6 Hz, 2H), 7.74 (d, *J* = 8.7 Hz, 2H), 7.48 (d, *J* = 1.9 Hz, 1H). MS (ESI) m/z: 312[M+H]$^+$. |

(continued)

| Cmpd ID | Structure | 1H NMR and/or MS data |
|---|---|---|
| I-12 | 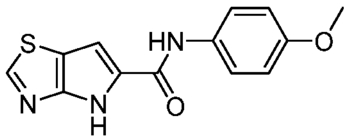 | $^1$H NMR (600 MHz, DMSO) δ 12.57 (s, 1H), 9.90 (s, 1H), 8.98 (s, 1H), 7.65 (d, *J* = 9.0 Hz,2H), 7.36 (s, 1H), 6.94 (d, *J* = 9.0 Hz, 2H), 3.75 (s, 3H). MS (ESI) *m/z*: 274[M+H]$^+$. |
| I-13 | 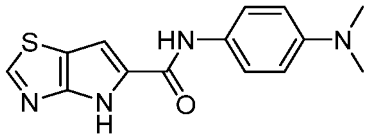 | $^1$H NMR (600 MHz, DMSO) δ 12.53 (s, 1H), 9.78 (s, 1H), 8.97 (s, 1H), 7.56 (d, *J* = 8.6 Hz, 2H), 7.34 (s, 1H), 6.75 (d, *J* = 8.7 Hz, 2H), 2.88 (s, 6H). MS (ESI) *m/z*: 287[M+H]$^+$. |
| I-14 | 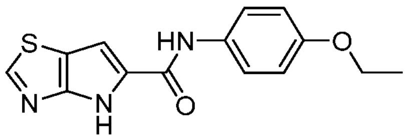 | $^1$H NMR (600 MHz, DMSO) δ 12.58 (s, 1H), 9.90 (s, 1H), 8.99 (s, 1H), 7.78 - 7.58 (m, 2H), 7.37 (d, *J* = 1.8 Hz, 1H), 7.04 - 6.81 (m, 2H), 4.02 (q, *J* = 7.0 Hz, 2H), 1.33 (s, 3H). MS (ESI) *m/z*: 288[M+H]$^+$. |
| I-15 | 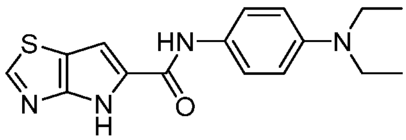 | $^1$H NMR (600 MHz, DMSO) δ 12.52 (s, 1H), 9.73 (s, 1H), 8.97 (s, 1H), 7.52 (d, *J* = 9.0 Hz, 2H), 7.34 (s, 1H), 6.67 (d, *J* = 9.0 Hz, 2H), 3.32 (d, *J* = 7.0 Hz, 4H), 1.09 (t, *J* = 7.0 Hz, 6H). MS (ESI) *m/z*: 315[M+H]$^+$. |
| I-16 | 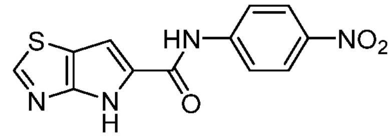 | $^1$H NMR (600 MHz, DMSO) δ 12.79 (s, 1H), 10.59 (s, 1H), 9.06 (s, 1H), 8.28 (d, *J* = 9.3 Hz, 2H), 8.07 (d, *J* = 9.3 Hz, 2H), 7.52 (s, 1H). MS (ESI) *m/z*: 289[M+H]$^+$. |
| I-17 | 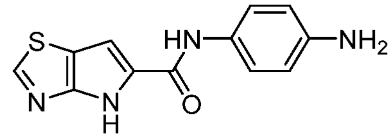 | $^1$H NMR (600 MHz, DMSO) δ 9.67 (s, 1H), 8.96 (s, 1H), 7.45 - 7.22 (m, 3H), 6.56 (d, *J* = 8.8 Hz, 2H), 5.76 (s, 1H), 4.92 (s, 2H). MS (ESI) *m/z*: 259[M+H]$^+$. |
| I-18 | 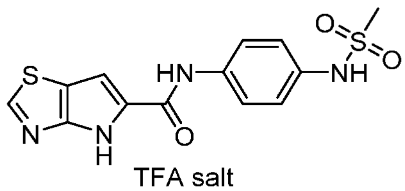 TFA salt | $^1$H NMR (600 MHz, DMSO) δ 12.60 (s, 1H), 10.04 (s, 1H), 9.57 (s, 1H), 8.98 (s, 1H), 7.71 (s, 2H), 7.39 (s, 1H), 7.19 (s, 2H), 2.95 (s,3H). MS (ESI) *m/z*: 337[M+H]$^+$. |
| I-19 | 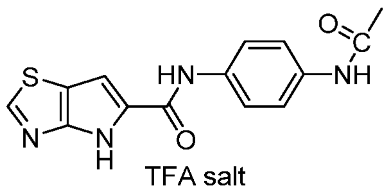 TFA salt | $^1$H NMR (600 MHz, DMSO) δ 12.59 (s, 1H), 10.09 - 9.74 (m, 2H), 9.00 (s, 1H), 7.67 (d, *J* = 8.8 Hz, 2H), 7.55 (d, *J* = 8.7 Hz, 2H), 7.39 (s, 1H), 2.04 (s, 3H). MS (ESI) *m/z*: 301[M+H]$^+$. |
| I-20 | 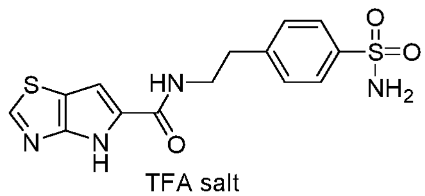 TFA salt | [1]b NMR (600 MHz, DMSO) δ 12.39 (s, 1H), 8.92 (s, 1H), 8.39 (t, *J* = 5.6 Hz, 1H), 7.74 (d, *J* = 8.3 Hz, 2H), 7.43 (d, *J* = 8.3 Hz, 2H), 7.28 (s, 2H), 7.08 (d, *J* = 1.6 Hz, 1H), 3.52 (dd, *J* = 13.1, 6.9 Hz, 2H), 2.93 (t, *J* = 7.1 Hz, 2H). MS (ESI) *m/z*: 351 [M+H]$^+$. |

(continued)

| Cmpd ID | Structure | 1H NMR and/or MS data |
|---|---|---|
| I-21 | 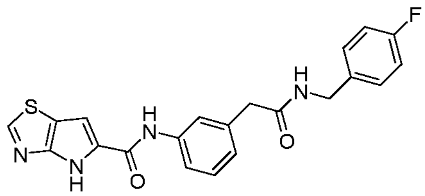 | $^1$H NMR (600 MHz, DMSO) δ 12.58 (s, 1H), 10.00 (s, 1H), 8.99 (s, 1H), 8.58 (s, 1H), 7.72 - 7.58 (m, 2H), 7.42 (s, 1H), 7.35 - 7.19 (m, 3H), 7.18 - 7.05 (m, 2H), 7.00 (s, 1H), 4.26 (d, *J* = 4.2 Hz, 2H), 3.38 (s, 2H). MS (ESI) *m*/*z*: 409[M+H]$^+$. |
| I-22 | 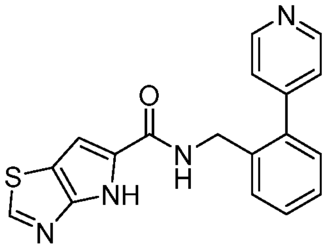 | $^1$H NMR (600 MHz, DMSO) δ 12.42 (s, 1H), 8.94 (s, 1H), 8.75 (d, *J* = 5.6 Hz, 1H), 8.66 (dd, *J* = 4.4, 1.6 Hz, 2H), 7.53 - 7.48 (m, 3H), 7.45 (td, *J* = 7.6, 1.3 Hz, 1H), 7.42 - 7.38 (m, 1H), 7.29 (dd, *J* = 7.6, 1.1 Hz, 1H), 7.16 (s, 1H), 4.44 (d, *J* = 5.7 Hz, 2H). MS (ESI) *m*/*z*: 335[M+H]$^+$. |
| I-23 | 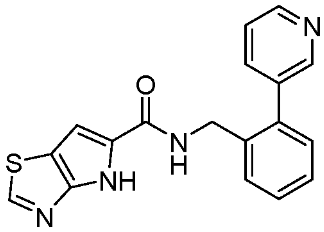 | $^1$H NMR (600 MHz, DMSO) δ 12.43 (s, 1H), 8.94 (s, 1H), 8.74 (t, *J* = 5.7 Hz, 1H), 8.66 (dd, *J* = 2.3, 0.7 Hz, 1H), 8.61 (dd, *J* = 4.8, 1.6 Hz, 1H), 7.94 - 7.87 (m, 1H), 7.53 - 7.48 (m, 2H), 7.44 (td, *J* = 7.6, 1.4 Hz, 1H), 7.39 (td, *J* = 7.5, 1.3 Hz, 1H), 7.29 (dd, *J* = 7.5, 1.3 Hz, 1H), 7.17 (d, *J* = 1.7 Hz, 1H), 4.42 (d, *J* = 5.7 Hz, 2H). MS (ESI) *m*/*z*: 335[M+H]$^+$. |
| I-24 | 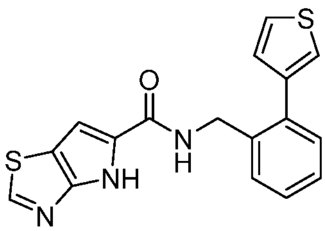 | $^1$H NMR (600 MHz, DMSO) δ 12.45 (s, 1H), 8.95 (s, 1H), 8.74 (s, 1H), 7.67 (dd, *J* = 4.9, 3.0 Hz, 1H), 7.64 - 7.60 (m, 1H), 7.44 (d, *J* = 7.6 Hz, 1H), 7.38 - 7.30 (m, 4H), 7.20 (s, 1H), 4.52 (d, *J* = 5.7 Hz, 2H). MS (ESI) m/z: 340[M+H]$^+$. |
| I-25 | 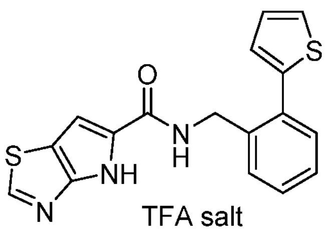 TFA salt | $^1$H NMR (600 MHz, DMSO) δ 12.46 (s, 1H), 8.94 (s, 1H), 8.75 (s, 1H), 7.65 (s, 1H), 7.56 - 7.31 (m, 4H), 7.31 - 7.15 (m, 3H), 4.58 (s, 2H). MS (ESI) *m*/*z*: 340[M+H]$^+$. |
| I-26 | 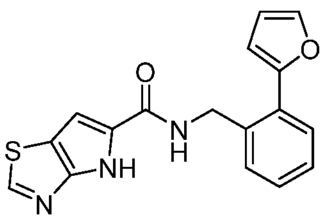 | $^1$H NMR (600 MHz, DMSO) δ 12.48 (s, 1H), 8.95 (s, 1H), 8.79 (t, *J* = 5.9 Hz, 1H), 7.83 (dd, *J* = 1.8, 0.7 Hz, 1H), 7.67 (dd, *J* = 7.3, 1.8 Hz, 1H), 7.41 (d, *J* = 2.2 Hz, 1H), 7.35 (ddd, *J* = 7.2, 4.6, 1.9 Hz, 2H), 7.22 (d, *J* = 1.9 Hz, 1H), 6.82 (dd, *J* = 3.4, 0.7 Hz, 1H), 6.65 (dd, *J* = 3.4, 1.8 Hz, 1H), 4.67 (d, *J* = 5.8 Hz, 2H). MS (ESI) *m*/*z*: 324[M+H]$^+$. |

(continued)

| Cmpd ID | Structure | 1H NMR and/or MS data |
|---|---|---|
| I-27 | 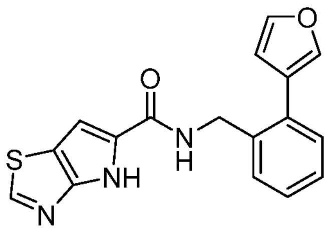 | 1H NMR (600 MHz, DMSO) δ 12.47 (s, 1H), 8.95 (s, 1H), 8.77 (t, *J* = 5.7 Hz, 1H), 7.94 (dd, *J* = 1.4, 0.9 Hz, 1H), 7.80 (t, *J* = 1.7 Hz, 1H), 7.45 - 7.38 (m, 2H), 7.35 - 7.29 (m, 2H), 7.22 (d, *J* = 1.8 Hz, 1H), 6.84 (dd, *J* = 1.8, 0.9 Hz, 1H), 4.57 (d, *J* = 5.8 Hz, 2H). MS (ESI) m/z: $324[M+H]^+$. |
| I-28 | 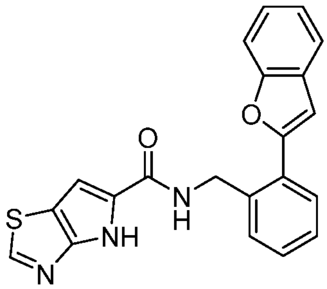 | 1H NMR (600 MHz, DMSO-$d_6$) δ 12.50 (s, 1H), 9.01 - 8.71 (m, 2H), 7.95 - 7.61 (m, 3H), 7.60 - 7.07 (m, 7H), 4.80 (s, 2H). MS (ESI) *m/z*: $374[M+H]^+$. |
| I-29 | 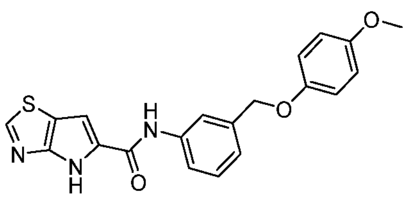 | 1H NMR (600 MHz, DMSO) δ 12.62 (s, 1H), 10.06 (s, 1H), 9.01 (s, 1H), 7.85 (s, 1H), 7.76 (d, *J* = 8.1 Hz, 1H), 7.43 (d, *J* = 1.9 Hz, 1H), 7.37 (t, *J* = 7.8 Hz, 1H), 7.15 (d, *J* = 7.8 Hz, 1H), 6.98 - 6.94 (m, 2H), 6.90 - 6.85 (m, 2H), 5.06 (s, 2H), 3.70 (s, 3H). MS (ESI) *m/z*: $380[M+H]^+$. |
| I-30 | 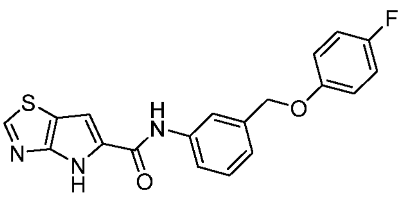 | 1H NMR (600 MHz, DMSO) δ 12.63 (s, 1H), 10.09 (s, 1H), 9.00 (s, 1H), 7.86 (s, 1H), 7.76 (d, *J* = 8.0 Hz, 1H), 7.43 (s, 1H), 7.37 (t, *J* = 7.9 Hz, 1H), 7.19 - 7.10 (m, 3H), 7.07 - 6.99 (m, 2H), 5.10 (s, 2H). MS (ESI) *m/z*: $368[M+H]^+$. |
| I-31 | 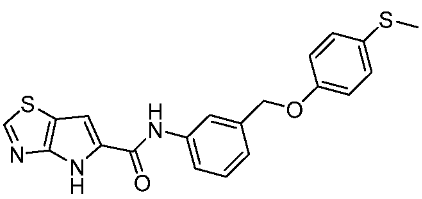 | 1H NMR (600 MHz, DMSO) δ 12.62 (s, 1H), 10.07 (s, 1H), 9.00 (s, 1H), 7.85 (s, 1H), 7.76 (d, *J* = 8.0 Hz, 1H), 7.43 (s, 1H), 7.37 (t, *J* = 7.8 Hz, 1H), 7.25 (d, *J* = 8.7 Hz, 2H), 7.16 (d, *J* = 7.5 Hz, 1H), 7.00 (d, *J* = 8.7 Hz, 2H), 5.11 (s, 2H), 2.42 (s, 3H). MS (ESI) *m/z*: $396[M+H]^+$. |
| I-32 | 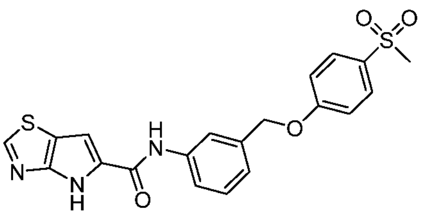 | 1H NMR (600 MHz, DMSO) δ 12.63 (s, 1H), 10.09 (s, 1H), 9.01 (s, 1H), 7.94 - 7.83 (m, 3H), 7.77 (d, *J* = 8.1 Hz, 1H), 7.49 - 7.36 (m, 2H), 7.31 - 7.23 (m, 2H), 7.19 (d, *J* = 7.7 Hz, 1H), 5.26 (s,2H) 3.16 (s, 3H). MS (ESI) *m/z*: $428[M+H]^+$. |
| I-33 | 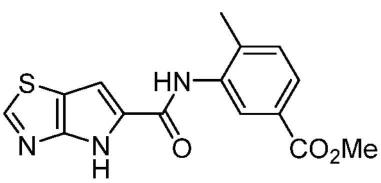 | 1H NMR (600 MHz, DMSO) δ 12.65 (s, 1H), 9.80 (s, 1H), 9.02 (s, 1H), 8.02 (s, 1H), 7.76 (d, *J* = 7.9 Hz, 1H), 7.45 (d, *J* = 7.9 Hz, 1H), 7.39 (d, *J* = 1.8 Hz, 1H), 3.87 (s, 3H), 2.35 (s, 3H). MS (ESI) m/z: $316[M+H]^+$. |

(continued)

| Cmpd ID | Structure | 1H NMR and/or MS data |
|---|---|---|
| I-34 | 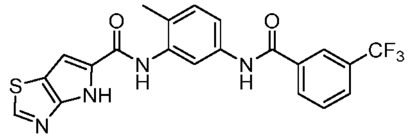 | $^1$H NMR (600 MHz, DMSO-$d_6$) δ 12.60 (d, $J$ = 1.9 Hz, 1H), 10.47 (s, 1H), 9.71 (s, 1H), 8.99 (s, 1H), 8.30 (s, 1H), 8.27 (d, $J$ = 7.9 Hz, 1H), 7.99 - 7.94 (m, 1H), 7.86 (d, $J$ = 2.2 Hz, 1H), 7.79 (t, $J$ = 7.8 Hz, 1H), 7.61 (dd, $J$ = 8.3, 2.2 Hz, 1H), 7.38 (d, $J$ = 1.9 Hz, 1H), 7.27 (d, $J$ = 8.3 Hz, 1H), 2.24 (s, 3H). MS (ESI) m/z: 445[M+H]$^+$ |
| I-35 | 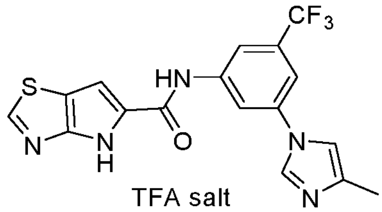 TFA salt | $^1$H NMR (600 MHz, Methanol-$d_4$) δ 9.43 (d, $J$ = 1.8 Hz, 1H), 8.90 (s, 1H), 8.56 (d, $J$ = 2.2 Hz, 1H), 8.16 (s, 1H), 7.86 (s, 1H), 7.77 (s, 1H), 7.39 (s, 1H), 2.46 (s, 3H). MS (ESI) *m/z*: 392 [M+H]$^+$ |

Synthetic scheme of compound II is:

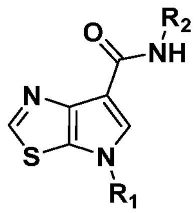

II

Compound II was synthesized by a method similar to that for the synthesis of compound I.

The table below lists the specific compounds and structure identification data.

Table 2. Structure and characterization of compounds II

| Cmpd ID | Structure | $^1$H NMR and/or MS data |
|---|---|---|
| II-1 | 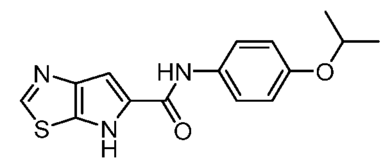 | $^1$H NMR (600 MHz, DMSO-$d_6$) δ 12.21 (s, 1H), 9.89 (s, 1H), 8.80 (d, $J$ = 0.9 Hz, 1H), 7.63 (d, $J$ = 8.7 Hz, 2H), 7.50 (t, $J$ = 1.3 Hz, 1H), 6.92 (d, $J$ = 8.9 Hz, 2H), 4.58 (p, $J$ = 6.0 Hz, 1H), 1.27 (dd, $J$ = 6.1, 0.9 Hz, 6H). MS (ESI) *m/z:* 302 [M+H]$^+$ |
| II-2 | 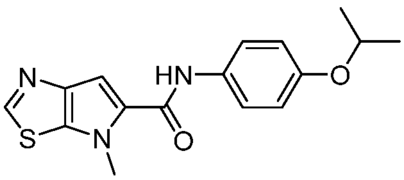 | $^1$H NMR (600 MHz, DMSO-$d_6$) δ 9.93 (s, 1H), 8.85 (s, 1H), 7.60 (d, $J$ = 9.0 Hz, 2H), 7.45 (s, 1H), 6.90 (d, $J$ = 9.0 Hz, 2H), 4.57 (p, $J$ = 6.0 Hz, 1H), 4.05 (s, 3H), 1.26 (d, $J$ = 6.0 Hz, 6H). MS (ESI) *m/z:* 316 [M+H]$^+$ |
| II-3 | 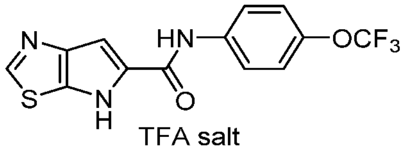 TFA salt | $^1$H NMR (600 MHz, DMSO-$d_6$) δ 12.29 (s, 1H), 10.18 (s, 1H), 8.81 (s, 1H), 7.87 (d, $J$ = 9.1 Hz, 2H), 7.54 (d, $J$ = 2.0 Hz, 1H), 7.37 (d, $J$ = 8.7 Hz, 2H). MS (ESI) *m/z*: 328 [M+H]$^+$ |

(continued)

| Cmpd ID | Structure | $^1$H NMR and/or MS data |
|---|---|---|
| II-4 | 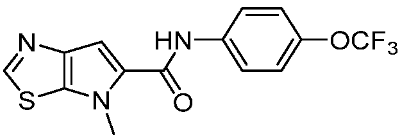 | $^1$H NMR (600 MHz, DMSO-$d_6$) δ 10.24 (s, 1H), 8.84 (s, 1H), 7.83 (d, *J* = 9.1 Hz, 2H), 7.49 (s, 1H), 7.35 (d, *J* = 8.6 Hz, 2H), 4.04 (s, 3H). MS (ESI) *m/z*: 342 [M+H]$^+$ |
| II-5 | 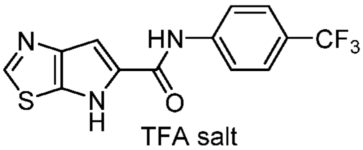 TFA salt | $^1$H NMR (600 MHz, DMSO-$d_6$) δ 12.36 (s, 1H), 10.36 (s, 1H), 8.83 (s, 1H), 8.02 (d, *J* = 8.5 Hz, 2H), 7.74 (d, *J* = 8.5 Hz, 2H), 7.62 (d, *J* = 1.9 Hz, 1H). MS (ESI) *m/z:* 312 [M+H]$^+$ |
| II-6 | 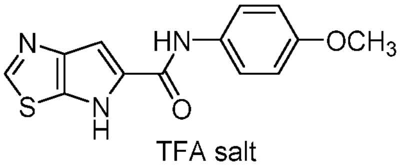 TFA salt | $^1$H NMR (600 MHz, DMSO-$d_6$) δ 12.19 (s, 1H), 9.92 (s, 1H), 8.79 (s, 1H), 7.69 - 7.59 (m, 2H), 7.49 (d, *J* = 2.0 Hz, 1H), 6.94 (d, *J* = 9.0 Hz, 2H), 3.75 (s, 3H). MS (ESI) *m/z*: 274 [M+H]$^+$ |
| II-7 | 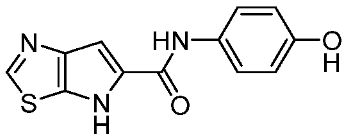 | MS (ESI) *m/z*: 260 [M+H]$^+$ |
| II-8 | 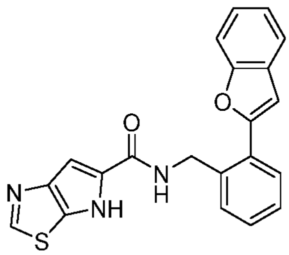 | $^1$H NMR (600 MHz, DMSO-$d_6$) δ 12.12 (s, 1H), 8.92 (t, *J* = 5.9 Hz, 1H), 8.77 (s, 1H), 7.88 - 7.84 (m, 1H), 7.72 (dt, *J* = 7.5, 1.1 Hz, 1H), 7.67 (dt, *J* = 8.2, 1.0 Hz, 1H), 7.50 (dd, *J* = 7.5, 1.8 Hz, 1H), 7.48 - 7.42 (m, 2H), 7.39 - 7.34 (m, 2H), 7.32 - 7.27 (m, 2H), 4.80 (d, *J* = 5.9 Hz, 2H). MS (ESI) *m/z*: 374 [M+H]$^+$ |
| II-9 | 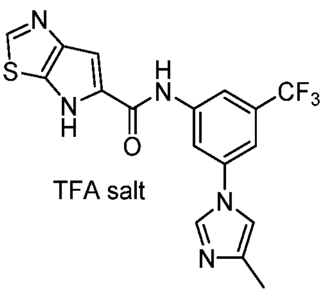 TFA salt | $^1$H NMR (600 MHz, Methanol-$d_4$) δ 9.45 (d, *J* = 1.5 Hz, 1H), 8.76 (s, 1H), 8.53 (t, *J* = 2.1 Hz, 1H), 8.23 (d, *J* = 1.7 Hz, 1H), 7.87 (d, *J* = 1.5 Hz, 1H), 7.78 (d, *J* = 1.8 Hz, 1H), 7.53 (s, 1H), 2.48 (s, 3H). MS (ESI) *m/z*: 392 [M+H]$^+$ |
| II-10 | 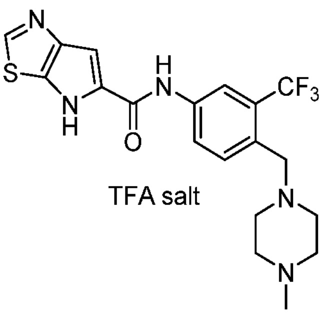 TFA salt | MS (ESI) *m/z*: 424 [M+H]$^+$ |

Test example

Biological activity assay:

The specific method was as follows:

1) Medium: DMEM (Dulbecco's modified eagle medium) or RPMI1640 (containing 10% fetal bovine serum, 100 μg/mL ampicillin, 100 μg/mL streptomycin).
2) Reagent: MTS reaction solution (containing 2 mg/mL of MTS (3-(4,5-dimethylthiazol-2-yl)-5-(3-carboxymethoxyphenyl)-2-(4-sulfophenyl)-2H- tetrazolium, inner salt); 100 μg/mL PES (phenazine methosulfate)).
3) Compound test:

(1) If the tumor cells tested are suspension cells: Human promyelocytic leukemia cells HL-60 ($2 \times 10^4$ cells/well) cultured *in vitro* were incubated into a 96-well culture plate, the volume of cell-culture medium was 90 μL, and then 10 μL of the compound at each gradient concentration was added (the highest concentration was 10 μM, which was diluted stepwise by 1/3, and 8 concentration points were set in total; the system contained 0.1% DMSO (dimethyl sulfoxide)). The cell plate with uniformly mixed compound was cultured in a cell culture incubator (37°C; 5% $CO_2$) for 48 h, then 20 μL of MTS reaction solution was added, uniformly mixed and incubated in the cell culture incubator (37°C; 5% $CO_2$) for 1-4 hr; OD values at 490 nm were measured by a microplate reader (VARIOSKAN FLASH, Thermo). Three parallels were set in each group of experiments, with 0.1% (a final concentration) DMSO as a negative control, and a medium without cells and compounds as a blank control. The cell growth inhibition rate was calculated by the following formula:

$$\text{Cell growth inhibition rate\%} = 1 - (OD_{\text{experimental group}} - OD_{\text{blank group}})/(OD_{\text{negative group}} - OD_{\text{blank group}}) \times 100\%$$

4) $IC_{50}$ calculation: The semi-inhibitory concentration of the compound acting on cell was calculated using GradPad Prism 5 software according to the measured cell growth inhibition rate.
(2) If the tumor cells tested are adherent cells:

Human lung cancer cells A549, human gastric cancer cells BGC-823, human liver cancer cells HepG-2 and human breast cancer cells MCF-7, human osteosarcoma cells U2OS, human prostate cancer cells LNCAP cultured *in vitro* were added in a 96-well culture plate at 1,000-10,000 cells/well, incubated until adherence, and then the compound was added. As to other procedures, please refer to the method (1) above.
The test results are shown in the following tables:

Table 3. Growth inhibition rates against different tumor cells of compounds at a single concentration (compound concentration C = 10 μM)

| Cmpd ID | Inhibition rate against A549 at 10 μM (%) | Inhibition rate against BGC-823 at 10 μM (%) | Inhibition rate against HepG-2 at 10 μM (%) | Inhibition rate against MCF-7 at 10 μM (%) | Inhibition rate against HL-60 at 10 μM (%) | Inhibition rate against U2OS at 10 μM (%) | Inhibition rate against LNCAP at 10 μM (%) |
|---|---|---|---|---|---|---|---|
| I-2 | 81.9% | 61.7% | 60.1% | 23.1% | N | 19.4% | 22.2% |
| I-6 | 6.3% | 64.5% | 66.5% | 3.4% | 69.1% | 56.2% | 55.8% |
| I-8 | 56.8% | 72.2% | 58.1% | 21.2% | 69.4% | 50.9% | / |
| I-11 | 7.6% | 58.8% | 62.9% | 7.4% | 71.2% | 56.2% | 56.1% |
| I-12 | 9.3% | 47.4% | 58.3% | 5.9% | 68.9% | 57.7% | 54.6% |
| I-13 | 3.4% | 54.3% | 54.7% | N | 69.7% | 53.2% | 48.6% |
| I-15 | 69.4% | 71.7% | 57.8% | 23.8% | 63.2% | 53.5% | / |
| I-16 | 32.4% | 63.3% | 38.6% | 17.6% | 44.6% | 40.2% | 58.0% |

(continued)

| Cmpd ID | Inhibition rate against A549 at 10 μM (%) | Inhibition rate against BGC-823 at 10 μM (%) | Inhibition rate against HepG-2 at 10 μM (%) | Inhibition rate against MCF-7 at 10 μM (%) | Inhibition rate against HL-60 at 10 μM (%) | Inhibition rate against U2OS at 10 μM (%) | Inhibition rate against LNCAP at 10 μM (%) |
|---|---|---|---|---|---|---|---|
| I-22 | 54.8% | 64.6% | 48.9% | 15.1% | 65.2% | 50% | 64.3% |
| I-23 | 53.4% | 71.1% | 60.4% | 27% | 70.5% | 56% | 64.4% |
| I-24 | 20.1% | 51.2% | 38.2% | 31.5% | 64.3% | 37.6% | 48.7% |
| I-26 | N | 23.4% | 29.7% | 3.1% | 64.8% | 44.5% | 44.9% |
| I-27 | 67.9% | 55.1% | 43.2% | 1.7% | 68.8% | 44.9% | / |
| I-28 | 50.3% | 60.3% | 44.4% | 21.0% | 74.8% | 48.7% | 71% |
| I-31 | N | 20.4% | 18.5% | 13.9% | 71.8% | 54.9% | 59.3% |
| I-32 | N | 9.2% | N | N | 70.3% | 45.2% | 41.5% |
| I-35 | 59.5% | 44.4% | 50.5% | N | 80.4% | 42.2% | 61.9% |

Table 4. Growth inhibitory activity of compounds on different tumor cells, $IC_{50}$ (unit: μM)

| Cmpd ID | BGC-823 ($IC_{50}$, μM) | HepG-2 ($IC_{50}$, μM) | HL-60 ($IC_{50}$, μM) | U20S ($IC_{50}$, μM) | LNCAP ($IC_{50}$, μM) |
|---|---|---|---|---|---|
| I-6 | 0.582 | 1.985 | 0.217 | 0.929 | 0.319 |
| I-8 | / | / | 1.777 | 6.352 | 6.043 |
| I-11 | 0.849 | 1.468 | 0.653 | 1.510 | 5.259 |
| I-12 | 0.952 | 0.223 | 0.590 | 1.516 | 6.31 |
| I-13 | 1.111 | 1.933 | 1.301 | 2.684 | N |
| I-15 | / | / | 2.712 | N | N |
| I-22 | / | / | 0.672 | 3.660 | 2.572 |
| I-23 | / | / | 0.475 | 2.925 | 1.578 |
| I-24 | / | / | 2.298 | 2.752 | 0.849 |
| I-26 | / | / | 3.212 | 6.172 | 0.163 |
| I-27 | / | / | 1.20E-05 | 0.006 | 0.032 |
| I-28 | / | / | 0.005 | 0.928 | 0.130 |
| I-31 | / | / | 0.491 | 8.918 | 2.597 |
| I-32 | / | / | 1.847 | 5.469 | 7.034 |
| I-35 | / | / | 7.034 | 7.850 | 4.738 |
| * N means non-active; "/" means not determined. | | | | | |

It could be seen from the above results that compounds I-27 and I-28 had relatively good growth inhibitory activity on human promyelocytic leukemia cells HL-60, human osteosarcoma cells U2OS, and human prostate cancer cells LNCAP.

Taking compound I-2 as an example, as shown in Table 5, 3 lung cancer cell lines (NCI-1975, HCC827, A549), 3 leukemia cell lines (SHI-1, THP-1, NB-4) and 2 human embryo lung cell lines (WI-38 and HFL-1) were tested. The compound showed selective inhibition on the growth of tumor cells ($IC_{50}$: 0.008-0.280 μM), while having less effect on the growth of normal human embryo lung cells ($IC_{50}$ > 5 μM), with a good treatment window.

Table 5. Growth inhibitory activity of compound I-2 on different tumor cells, $IC_{50}$ (unit: $\mu M$)

| Cmpd ID | Lung cancer cells $IC_{50}$ | | | Human embryo lung cells $IC_{50}$ | | Leukemia cells $IC_{50}$ | | |
|---|---|---|---|---|---|---|---|---|
| | NCI-1975 | HCC827 | A549 | WI-38 | HFL-1 | SHI-1 | THP-1 | NB4 |
| I-2 | 0.057 | 0.008 | 0.030 | 5.619 | 8.589 | 0.221 | 2.464 | 0.280 |

## Claims

**1.** A compound having the general formula:

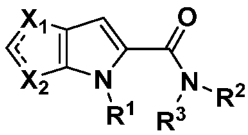

wherein, $X_1$ is selected from the group consisting of N, S; $X_2$ is selected from the group consisting of N, S; and, $X_1$ and $X_2$ are not the same;
$R^1$ is selected from the group consisting of H, C1-C6 alkyl, C3-C6 cycloalkyl; preferably $R^1$ is selected from the group consisting of H, C1-C3 alkyl; more preferably $R^1$ is selected from the group consisting of H, methyl, ethyl;
$R^2$ is selected from the group consisting of H, C1-C6 alkyl, C3-C6 cycloalkyl; preferably $R^2$ is selected from the group consisting of H, C1-C3 alkyl; more preferably $R^2$ is selected from the group consisting of H, methyl, ethyl;
$R^3$ is selected from the group consisting of:

1)

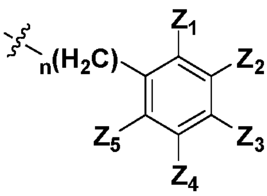

,

wherein n=0, 1 or 2, $Z_1$, $Z_2$, $Z_3$, $Z_4$, $Z_5$ each are independently selected from the group consisting of:

(1) H, F, Cl, Br, I, nitro, cyano, amino; amino optionally substituted by -C1-C6 alkyl, C1-C3 alkyl, -C1-C6 alkylsulfonyl or -C1-C6 alkylcarbonyl; hydroxy, hydroxyformyl, methoxyformyl, ethoxyformyl, n-propoxyformyl, isopropoxyformyl, aminoformyl, *N*-methylaminoformyl, *N*-ethylaminoformyl, *N*-n-propylaminoformyl, *N*-isopropylaminoformyl, *N*-cyclopropylaminoformyl, *N*-n-butylaminoformyl, *N*-isobutylaminoformyl, *N*-t-butylaminoformyl, *N*-cyclobutylaminoformyl, *N*-n-pentylaminoformyl, *N*-isopentylaminoformyl, *N*-cyclopentylaminoformyl, *N*-n-hexylaminoformyl, *N*-isohexylaminoformyl, *N*-cyclohexylaminoformyl, *N,N*-dimethylaminoformyl, *N,N*-diethylaminoformyl, *N,N*-di-n-propylaminoformyl, *N,N*-diisopropylaminoformyl, cyclopropylaminoformyl, cyclobutylaminoformyl, cyclopentylaminoformyl, cyclohexylaminoformyl, 4-hydroxypiperidinylformyl, piperazinylformyl, 4-methylpiperazinylformyl, 4-ethylpiperazinylformyl, 4-n-propylpiperazinylformyl, 4-isopropylpiperazinylformyl, methanesulfonyl, ethylsulfonyl, n-propylsulfonyl, isopropylsulfonyl, n-butylsulfonyl, isobutylsulfonyl, hydroxysulfonyl, aminosulfonyl, *N*-methylaminosulfonyl, *N*-ethylaminosulfonyl, *N*-n-propylaminosulfonyl, *N*-isopropylaminosulfonyl, *N*-cyclopropylaminosulfonyl, *N*-n-butylaminosulfonyl, *N*-isobutylaminosulfonyl, *N*-t-butylaminosulfonyl, *N*-cyclobutylaminosulfonyl, *N*-n-pentylaminosulfonyl, *N*-isopentylaminosulfonyl, *N*-cyclopentylaminosulfonyl, *N*-n-hexylaminosulfonyl, *N*-isohexylaminosulfonyl, *N*-cyclohexylaminosulfonyl, *N,N*-dimethylaminosulfonyl, *N,N*-diethylaminosulfonyl, *N,N*-di-n-propylaminosulfonyl, *N,N*-diisopropylaminosulfonyl, cyclopropylaminosulfonyl, cyclobutylaminosulfonyl, cyclopentylaminosulfonyl, cyclohexylaminosulfonyl, 4-hydroxypiperidinylsulfonyl, piperazinylsulfonyl, 4-methylpiperazinylsulfonyl, 4-ethylpiperazinylsulfonyl, 4-n-propylpiperazinylsulfonyl, 4-isopropylpiperazinylsulfonyl, formamido, acetamido, propionamido, n-butyramido, isobutyramido, cyclopropylformamido, cyclobutylformamido, cyclopentylformamido, cyclohexylformamido, methanesulfonamido, ethanesulfonamido, n-propanesulfonamido, isopropanesulfonamido, n-butanesulfonamido, isobutanesulfonamido, substituted phenyl-C1-C6 alkyl-aminocarbonyl-C1-C6 alkyl; phenyl-O-C1-C6 alkyl, the phenyl is substituted by C1-C6 alkyl-O-, halogen, C1-C6 alkyl-S- or C1-C6 alkylsulfonyl; 6-membered heterocyclyl-C1-C6 alkyl, the 6-membered heterocyclyl is substituted by - C1-C6 alkyl;

(2) -O-C1-C6 alkyl, -O-C1-C6 fluorine-containing alkyl, -C1-C6 fluorine-containing alkyl, -C1-C6 alkoxycarbonyl, C1-C3 alkyl, C1-C3 alkoxy, C1-C3 oxygen-containing alkyl, C1-C3 fluorine-containing alkyl, C1-C3 fluorine-containing alkoxy;

(3) 5- or 6-membered heterocyclyl containing one or more heteroatoms selected from N, O and S, the 5- or 6-membered heterocyclyl is optionally substituted by C1-C6 alkyl, C1-C6 alkoxy, hydroxy, amino, C1-C6 alkoxycarbonyl, C1-C6 acyl, cyano or optionally substituted heterocyclyl,

[4] including but not limited to: piperidinyl, 4-*N,N*-dimethylaminopiperidinyl, 4-*N,N*-diethylaminopiperidinyl, 4-*N,N*-diisopropylaminopiperidinyl, 4-hydroxypiperidinyl, 4-(*4*-methylpiperazinyl)piperidinyl, 4-(*4*-ethylpiperazinyl)piperidinyl, 4-(*4*-isopropylpiperazinyl)piperidinyl, 4-(*4*-acetylpiperazinyl)piperidinyl, 4-(*4*-t-butoxyformylpiperazinyl)piperidinyl, 4-(*4*-methanesulfonylpiperazinyl)piperidinyl, 4-(*4*-(2-hydroxyethyl)piperazinyl)piperidinyl, 4-(*4*-(2-cyanoethyl)piperazinyl)piperidinyl, 4-(*4*-(3-hydroxypropyl)piperazinyl)piperidinyl, 4-(4-(2-*N,N*-dimethylaminoethyl)piperazinyl)piperidinyl, 4-(*4*-(2-*N,N*-diethylaminoethyl)piperazinyl)piperidinyl, 4-(*4*-(3-*N,N*-dimethylaminopropyl)piperazinyl)piperidinyl, 4-(*4*-(3-*N,N*-diethylaminopropyl)piperazinyl)piperidinyl, 4-(tetrahydropyrrolyl)piperidinyl, 4-(3-*N,N*-dimethylaminotetrahydropyrrolyl)piperidinyl;

*4*-methylpiperazinyl, *4*-ethylpiperazinyl, *4*-isopropylpiperazinyl, *4*-acetylpiperazinyl, *4*-t-butoxyformylpiperazinyl, *4*-methanesulfonylpiperazinyl, *4*-(2-hydroxyethyl)piperazinyl, *4*-(2-cyanoethyl)piperazinyl, *4*-(3-hydroxypropyl)piperazinyl, *4*-(2-*N,N*-dimethylaminoethyl)piperazinyl, *4*-(2-*N,N*-diethylaminoethyl)piperazinyl, *4*-(3-*N,N*-dimethylaminopropyl)piperazinyl, *4*-(3-*N,N*-diethylaminopropyl)piperazinyl, 2-oxo-piperazin-4-yl, *4*-(*N*-methyl-4-piperidinyl)piperazinyl, *4*-(*N*-ethyl-4-piperidinyl)piperazinyl, *4*-(*N*-acetyl-4-piperidinyl)piperazinyl; morpholinyl, 3,5-dimethylmorpholinyl, thiomorpholinyl, tetrahydropyrrolyl, 3-*N,N*-dimethyltetrahydropyrrolyl, 3-*N,N*-diethyltetrahydropyrrolyl;

(4) heteroaryl, for example, but not limited to pyridyl, furyl, thienyl, benzofuryl;

(5) $Z_2$ and $Z_3$ may form oxygen-containing substituted or unsubstituted 5-membered ring or 6-membered ring; the substituent may be selected from the substituents identical with those of $Z_1$;

(6) $Z_4$ and $Z_5$ may form nitrogen-containing substituted or unsubstituted 5-membered ring or 6-membered ring; the substituent may be selected from the substituents identical with those of $Z_1$;

2) H, C1-C6 alkyl, cyclopropyl, cyclobutyl, cyclopentyl, cyclohexyl, N,N-dimethylamino, N,N-diethylamino, N,N-diisopropylamino, 2-N,N-dimethylaminoethyl, 2-hydroxyethyl, 2-morpholinylethyl, 2-thiomorpholinylethyl, 2-(4-methylpiperazinyl)ethyl, 3-N,N-dimethylaminopropyl, 3-N,N-diethylaminopropyl, 3-N,N-diisopropylaminopropyl, 3-hydroxypropyl, 3-morpholinylpropyl, 3-thiomorpholinylpropyl, 3-(4-methylpiperazinyl)propyl, N-methyl-4-piperidinyl, N-ethyl-4-piperidinyl, N-isopropyl-4-piperidinyl, N-acetyl-4-piperidinyl;

or a stereoisomer thereof, a prodrug thereof, a pharmaceutically acceptable salt thereof or a pharmaceutically acceptable solvate thereof.

**2.** The compound according to claim 1, which is:

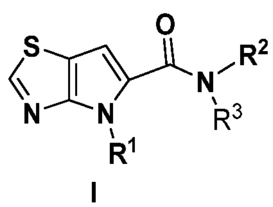

**I**

wherein:

$R^1$ is selected from the group consisting of H, C1-C6 alkyl, C3-C6 cycloalkyl; preferably $R^1$ is selected from the group consisting of H, C1-C3 alkyl; more preferably $R^1$ is selected from the group consisting of H, methyl, ethyl; $R^2$ is selected from the group consisting of H, C1-C6 alkyl, C3-C6 cycloalkyl; preferably $R^2$ is selected from the group consisting of H, C1-C3 alkyl; more preferably $R^2$ is selected from the group consisting of H, methyl, ethyl;

$R^3$ is selected from the group consisting of:

1)

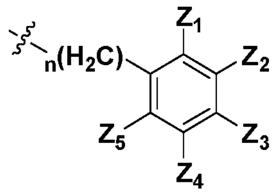

,

wherein n=0, 1 or 2, $Z_1$, $Z_2$, $Z_3$, $Z_4$, $Z_5$ each are independently selected from the group consisting of:

(1) H, F, Cl, Br, I, nitro, cyano, amino; amino optionally substituted by -C1-C6 alkyl, C1-C3 alkyl, -C1-C6 alkylsulfonyl or -C1-C6 alkylcarbonyl; hydroxy, hydroxyformyl, methoxyformyl, ethoxyformyl, n-propoxyformyl, isopropoxyformyl, aminoformyl, *N*-methylaminoformyl, *N*-ethylaminoformyl, *N*-n-propylaminoformyl, *N*-isopropylaminoformyl, *N*-cyclopropylaminoformyl, *N*-n-butylaminoformyl, *N*-isobutylaminoformyl, *N*-t-butylaminoformyl, *N*-cyclobutylaminoformyl, *N*-n-pentylaminoformyl, *N*-isopentylaminoformyl, *N*-cyclopentylaminoformyl, *N*-n-hexylaminoformyl, *N*-isohexylaminoformyl, *N*-cyclohexylaminoformyl, *N,N*-dimethylaminoformyl, *N,N*-diethylaminoformyl, *N,N*-di-n-propylaminoformyl, *N,N*-diisopropylaminoformyl, cyclopropylaminoformyl, cyclobutylaminoformyl, cyclopentylaminoformyl, cyclohexylaminoformyl, 4-hydroxypiperidinylformyl, piperazinylformyl, 4-methylpiperazinylformyl, 4-ethylpiperazinylformyl, 4-n-propylpiperazinylformyl, 4-isopropylpiperazinylformyl, methanesulfonyl, ethylsulfonyl, n-propylsulfonyl, isopropylsulfonyl, n-butylsulfonyl, isobutylsulfonyl, hydroxysulfonyl, aminosulfonyl, *N*-methylaminosulfonyl, *N*-ethylaminosulfonyl, *N*-n-propylaminosulfonyl, *N*-isopropylaminosulfonyl, *N*-cyclopropylaminosulfonyl, *N*-n-butylaminosulfonyl, *N*-isobutylaminosulfonyl, *N*-t-butylaminosulfonyl, *N*-cyclobutylaminosulfonyl, *N*-n-pentylaminosulfonyl, *N*-isopentylaminosulfonyl, *N*-cyclopentylaminosulfonyl, *N*-n-hexylaminosulfonyl, *N*-isohexylaminosulfonyl, *N*-cyclohexylaminosulfonyl, *N,N*-dimethylaminosulfonyl, *N,N*-diethylaminosulfonyl, *N,N*-di-n-propylaminosulfonyl, *N,N*-diisopropylaminosulfonyl, cyclopropylaminosulfonyl, cyclobutylaminosulfonyl, cyclopentylaminosulfonyl, cyclohexylaminosulfonyl, 4-hydroxypiperidinylsulfonyl, piperazinylsulfonyl, 4-methylpiperazinylsulfonyl, 4-ethylpiperazinylsulfonyl, 4-n-propylpiperazinylsulfonyl, 4-isopropylpiperazinylsulfonyl, formamido, acetamido, propionamido, n-butyramido, isobutyramido, cyclopropylformamido, cyclobutylformamido, cyclopentylformamido, cyclohexylformamido, methanesulfonamido, ethanesulfonamido, n-propanesulfonamido, isopropanesulfonamido, n-butanesulfonamido, isobutanesulfonamido, substituted phenyl-C1-C6 alkyl-aminocarbonyl-C1-C6 alkyl; phenyl-O-C1-C6 alkyl, the phenyl is substituted by C1-C6 alkyl-O-, halogen, C1-C6 alkyl-S- or C1-C6 alkylsulfonyl; 6-membered heterocyclyl-C1-C6 alkyl, the 6-membered heterocyclyl is substituted by - C1-C6 alkyl;
(2) -O-C1-C6 alkyl, -O-C1-C6 fluorine-containing alkyl, -C1-C6 fluorine-containing alkyl, -C1-C6 alkoxycarbonyl, C1-C3 alkyl, C1-C3 alkoxy, C1-C3 oxygen-containing alkyl, C1-C3 fluorine-containing alkyl, C1-C3 fluorine-containing alkoxy;
(3) 5- or 6-membered heterocyclyl containing one or more heteroatoms selected from N, O and S, the 5- or 6-membered heterocyclyl is optionally substituted by C1-C6 alkyl, C1-C6 alkoxy, hydroxy, amino, C1-C6 alkoxycarbonyl, C1-C6 acyl, cyano or optionally substituted heterocyclyl,
[5] including but not limited to: piperidinyl, 4-*N,N*-dimethylaminopiperidinyl, *4-N,N*-diethylaminopiperidinyl, 4-*N,N*-diisopropylaminopiperidinyl, 4-hydroxypiperidinyl, 4-(*4*-methylpiperazinyl)piperidinyl, 4-(*4*-ethylpiperazinyl)piperidinyl, 4-(*4*-isopropylpiperazinyl)piperidinyl, 4-(*4*-acetylpiperazinyl)piperidinyl, 4-(*4*-t-butoxyformylpiperazinyl)piperidinyl, 4-(*4*-methanesulfonylpiperazinyl)piperidinyl, 4-(*4*-(2-hydroxyethyl)piperazinyl)piperidinyl, 4-(*4*-(2-cyanoethyl)piperazinyl)piperidinyl, 4-(*4*-(3-hydroxypropyl)piperazinyl)piperidinyl, 4-(*4*-(2-*N,N*-dimethylaminoethyl)piperazinyl)piperidinyl, 4-(*4*-(2-*N,N*-diethylaminoethyl)piperazinyl)piperidinyl, 4-(*4*-(3-*N,N*-dimethylaminopropyl)piperazinyl)piperidinyl, 4-(*4*-(3-*N,N*-diethylaminopropyl)piperazinyl)piperidinyl, 4-(tetrahydropyrrolyl)piperidinyl, 4-(3-*N,N*-dimethylaminotetrahydropyrrolyl)piperidinyl;
*4*-methylpiperazinyl, *4*-ethylpiperazinyl, *4*-isopropylpiperazinyl, *4*-acetylpiperazinyl, *4*-t-butoxyformylpiperazinyl, *4*-methanesulfonylpiperazinyl, *4*-(2-hydroxyethyl)piperazinyl, *4*-(2-cyanoethyl)piperazinyl, *4*-(3-hydroxypropyl)piperazinyl, *4*-(2-*N,N*-dimethylaminoethyl)piperazinyl, *4*-(2-*N,N*-diethylaminoethyl)piperazinyl, *4*-(3-*N,N*-dimethylaminopropyl)piperazinyl, *4*-(3-*N,N*-diethylaminopropyl)piperazinyl,

2-oxo-piperazin-4-yl, 4-(*N*-methyl-4-piperidinyl)piperazinyl, 4-(*N*-ethyl-4-piperidinyl)piperazinyl, 4-(*N*-acetyl-4-piperidinyl)piperazinyl; morpholinyl, 3,5-dimethylmorpholinyl, thiomorpholinyl, tetrahydropyrrolyl, 3-*N,N*-dimethylaminotetrahydropyrrolyl, 3-*N,N*-diethylaminotetrahydropyrrolyl;
(4) heteroaryl, for example, but not limited to pyridyl, furyl, thienyl, benzofuryl;
(5) $Z_2$ and $Z_3$ may form oxygen-containing substituted or unsubstituted 5-membered ring or 6-membered ring; the substituent may be selected from the substituents identical with those of $Z_1$;
(6) $Z_4$ and $Z_5$ may form nitrogen-containing substituted or unsubstituted 5-membered ring or 6-membered ring; the substituent may be selected from the substituents identical with those of $Z_1$;

2) H, C1-C6 alkyl, cyclopropyl, cyclobutyl, cyclopentyl, cyclohexyl, N,N-dimethylamino, N,N-diethylamino, N,N-diisopropylamino, 2-N,N-dimethylaminoethyl, 2-hydroxyethyl, 2-morpholinylethyl, 2-thiomorpholinylethyl, 2-(4-methylpiperazinyl)ethyl, 3-N,N-dimethylaminopropyl, 3-N,N-diethylaminopropyl, 3-N,N-diisopropylaminopropyl, 3-hydroxypropyl, 3-morpholinylpropyl, 3-thiomorpholinylpropyl, 3-(4-methylpiperazinyl)propyl, N-methyl-4-piperidinyl, N-ethyl-4-piperidinyl, N-isopropyl-4-piperidinyl, N-acetyl-4-piperidinyl;
$R^3$ preferably is selected from the group consisting of:

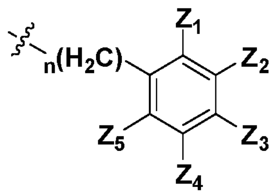
,

wherein n=0, 1 or 2,
when n=0, one of $Z_1$, $Z_2$, $Z_3$, $Z_4$, $Z_5$ is selected from the following groups, the rest being -H: hydroxy, -O-C1-C6 alkyl, -O-C1-C6 fluorine-containing alkyl, -C1-C6 fluorine-containing alkyl, -C1-C6 alkoxycarbonyl, amino; amino optionally substituted by -C1-C6 alkyl, -C1-C6 alkylsulfonyl or -C1-C6 alkylcarbonyl; aminosulfonyl, nitro, substituted phenyl-C1-C6 alkyl-aminocarbonyl-C1-C6 alkyl (more preferably phenyl-C1-C6 alkylaminocarbonyl-C1-C6 alkyl, the phenyl is substituted by halogen); phenyl-O-C1-C6 alkyl, the phenyl is substituted by C1-C6 alkyl-O-, halogen, C1-C6 alkyl-S- or C1-C6 alkylsulfonyl;
or, two of $Z_1$, $Z_2$, $Z_3$, $Z_4$, $Z_5$ each are independently selected from the following groups, the rest being -H (more preferably $Z_2$, $Z_3$ each or $Z_1$, $Z_4$ each or $Z_2$, $Z_4$ each are independently selected from the following groups, the rest being -H): -C1-C6 fluorine-containing alkyl; 6-membered heterocyclyl-C1-C6 alkyl, the 6-membered heterocyclyl is substituted by -C1-C6 alkyl (more preferably piperazinyl-C1-C6 alkyl, the piperazinyl is substituted by -C1-C6 alkyl); -C1-C6 alkyl, substituted phenylcarbonyl-amino, -C1-C6 alkyl-O-carbonyl, 5-membered heteroaryl substituted by -C1-C6 alkyl (more preferably imidazolyl substituted by -C1-C6 alkyl);
when n=1, one of $Z_1$, $Z_2$, $Z_3$, $Z_4$, $Z_5$ is selected from the following groups, the rest being -H: pyridyl, furyl, thienyl, benzofuryl;
when n=2, one of $Z_1$, $Z_2$, $Z_3$, $Z_4$, $Z_5$ is selected from the following groups, the rest being -H: aminosulfonyl;
$R^3$ more preferably is selected from the group consisting of:

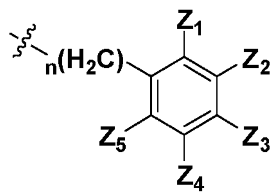
,

wherein n=0 or 1,
when n=0, $Z_1$, $Z_2$, $Z_4$, $Z_5$ each are -H, $Z_3$ is selected from the following groups: hydroxy, -O-C1-C6 alkyl, -O-C1-C6 fluorine-containing alkyl, -C1-C6 fluorine-containing alkyl, -C1-C6 alkoxycarbonyl, amino; amino optionally substituted by -C1-C6 alkyl, -C1-C6 alkylsulfonyl or -C1-C6 alkylcarbonyl; aminosulfonyl, nitro;
or, $Z_2$ or $Z_4$ is selected from the following groups, the rest being -H: -C1-C6 alkoxycarbonyl, substituted phenyl-C1-C6 alkyl-aminocarbonyl-C1-C6 alkyl (more preferably phenyl-C1-C6 alkyl-aminocarbonyl-C1-C6 alkyl, the phenyl is substituted by halogen); phenyl-O-C1-C6 alkyl, the phenyl is substituted by C1-C6

alkyl-O-, halogen, C1-C6 alkyl-S- or C1-C6 alkylsulfonyl,

or, $Z_2$, $Z_3$ each are independently selected from the following groups, the rest being - H: -C1-C6 fluorine-containing alkyl; 6-membered heterocyclyl-C1-C6 alkyl, the 6-membered heterocyclyl is substituted by -C1-C6 alkyl (more preferably piperazinyl-Cl-C6 alkyl, the piperazinyl is substituted by -C1-C6 alkyl);

or, $Z_1$, $Z_4$ each are independently selected from the following groups, the rest being - H: -C1-C6 alkyl, substituted phenylcarbonyl-amino, -C1-C6 alkyl-O-carbonyl;

or, $Z_2$, $Z_4$ each are independently selected from the following groups, the rest being - H: -C1-C6 fluorine-containing alkyl, 5-membered heteroaryl substituted by -C1-C6 alkyl (more preferably imidazolyl substituted by -C1-C6 alkyl);

when n=1, $Z_1$ or $Z_5$ is selected from the following groups, the rest being -H: pyrid-4-yl, pyrid-3-yl, fur-2-yl, fur-3-yl, thien-2-yl, thien-3-yl, benzofuryl;

when n=2, $Z_1$, $Z_2$, $Z_4$, $Z_5$ each are -H, $Z_3$ is aminosulfonyl;

$R^3$ most preferably is selected from the group consisting of:

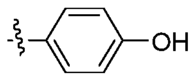 , 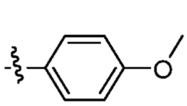 , 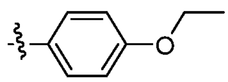 , 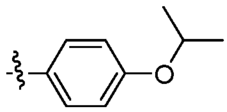 , 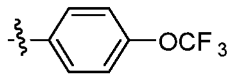 ,

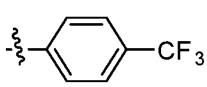 , 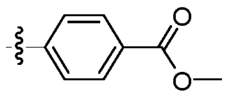 ,

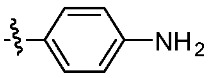 , 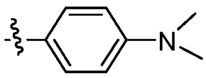 , 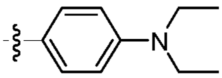 , 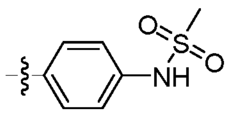 , 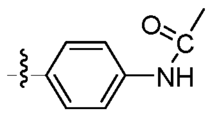 ,

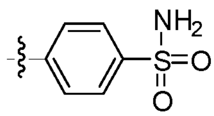 , 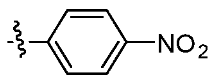 ,

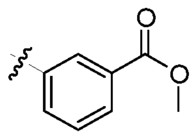 , 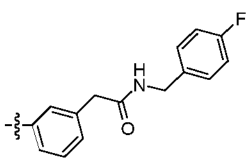 , 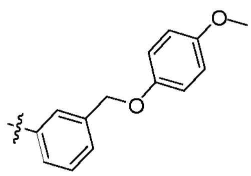 , 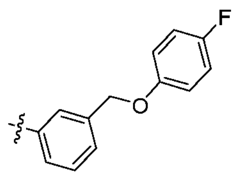 ,

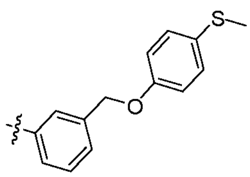 , 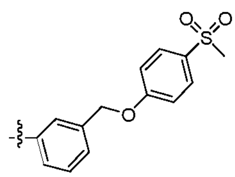 ,

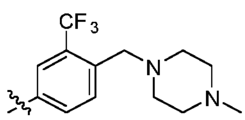 , 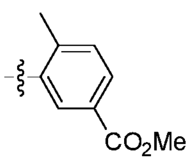 , 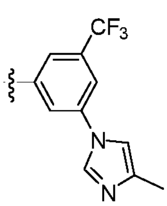 , 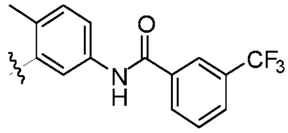 ,

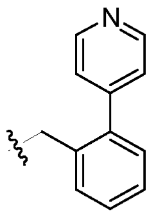,
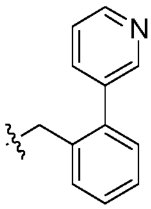,
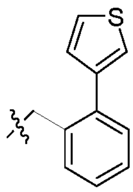,
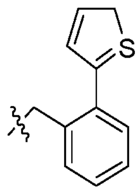,
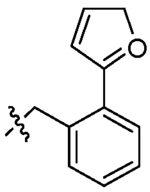,
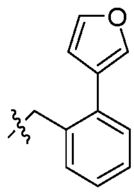,
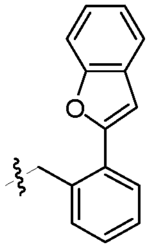,

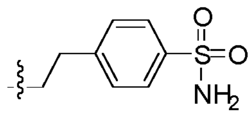,

or a stereoisomer thereof, a prodrug thereof, a pharmaceutically acceptable salt thereof or a pharmaceutically acceptable solvate thereof.

**3.** The compound according to claim 1, which is:

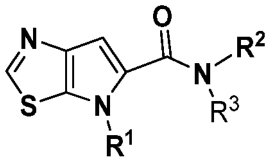

**II**

wherein:

$R^1$ is selected from the group consisting of H, C1-C6 alkyl, C3-C6 cycloalkyl; preferably $R^1$ is selected from the group consisting of H, C1-C3 alkyl; more preferably $R^1$ is selected from the group consisting of H, methyl, ethyl;
$R^2$ is selected from the group consisting of H, C1-C6 alkyl, C3-C6 cycloalkyl; preferably $R^2$ is selected from the group consisting of H, C1-C3 alkyl; more preferably $R^2$ is selected from the group consisting of H, methyl, ethyl;
$R^3$ is selected from the group consisting of:

1)

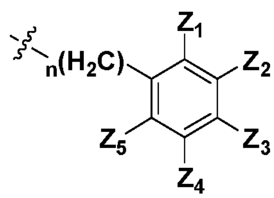,

wherein n=0, 1 or 2, $Z_1$, $Z_2$, $Z_3$, $Z_4$, $Z_5$ each are independently selected from the group consisting of:

(1) H, F, Cl, Br, I, nitro, cyano, amino, hydroxy, hydroxyformyl, methoxyformyl, ethoxyformyl, n-propoxyformyl, isopropoxyformyl, aminoformyl, *N*-methylaminoformyl, *N*-ethylaminoformyl, *N*-n-propylaminoformyl, N-isopropylaminoformyl, *N*-cyclopropylaminoformyl, *N*-n-butylaminoformyl, *N*-isobutylaminoformyl, *N*-t-butylaminoformyl, *N*-cyclobutylaminoformyl, *N*-n-pentylaminoformyl, *N*-isopentylaminoformyl, *N*-cyclopentylaminoformyl, *N*-n-hexylaminoformyl, *N*-isohexylaminoformyl, *N*-cyclohexylaminoformyl, *N,N*-dimethylaminoformyl, *N,N*-diethylaminoformyl, *N,N*-di-n-propylaminoformyl, *N,N*-diisopropylaminoformyl, cyclopropylaminoformyl, cyclobutylaminoformyl, cyclopentylaminoformyl, cyclohexylaminoformyl, 4-hydroxypiperidinylformyl, piperazinylformyl, 4-methylpiperazinylformyl, 4-ethylpiperazinylformyl, 4-n-propylpiperazinylformyl, 4-isopropylpiperazinylformyl, methanesulfonyl, ethylsulfonyl, n-propylsulfonyl, isopropylsulfonyl, n-butylsulfonyl, isobutylsulfonyl, hydroxysulfonyl, aminosulfonyl, *N*-

methylaminosulfonyl, *N*-ethylaminosulfonyl, *N*-n-propylaminosulfonyl, *N*-isopropylaminosulfonyl, *N*-cyclopropylaminosulfonyl, *N*-n-butylaminosulfonyl, *N*-isobutylaminosulfonyl, *N*-t-butylaminosulfonyl, *N*-cyclobutylaminosulfonyl, *N*-n-pentylaminosulfonyl, *N*-isopentylaminosulfonyl, *N*-cyclopentylaminosulfonyl, *N*-n-hexylaminosulfonyl, *N*-isohexylaminosulfonyl, *N*-cyclohexylaminosulfonyl, *N,N*-dimethylaminosulfonyl, *N,N*-diethylaminosulfonyl, *N,N*-di-n-propylaminosulfonyl, *N,N*-diisopropylaminosulfonyl, cyclopropylaminosulfonyl, cyclobutylaminosulfonyl, cyclopentylaminosulfonyl, cyclohexylaminosulfonyl, 4-hydroxypiperidinylsulfonyl, piperazinylsulfonyl, 4-methylpiperazinylsulfonyl, 4-ethylpiperazinylsulfonyl, 4-n-propylpiperazinylsulfonyl, 4-isopropylpiperazinylsulfonyl, formamido, acetamido, propionamido, n-butyramido, isobutyramido, cyclopropylformamido, cyclobutylformamido, cyclopentylformamido, cyclohexylformamido, methanesulfonamido, ethanesulfonamido, n-propanesulfonamido, isopropanesulfonamido, n-butanesulfonamido, isobutanesulfonamido;

(2) -O-C1-C6 alkyl, -O-C1-C6 fluorine-containing alkyl, -C1-C6 fluorine-containing alkyl, C1-C3 alkyl, C1-C3 alkoxy, C1-C3 oxygen-containing alkyl, C1-C3 fluorine-containing alkyl, C1-C3 fluorine-containing alkoxy; 6-membered heterocyclyl-C1-C6 alkyl, the 6-membered heterocyclyl is substituted by -C1-C6 alkyl;

(3) 5- or 6-membered heterocyclyl containing one or more heteroatoms selected from N, O and S, the 5- or 6-membered heterocyclyl is optionally substituted by C1-C6 alkyl, C1-C6 alkoxy, hydroxy, amino, C1-C6 alkoxycarbonyl, C1-C6 acyl, cyano or optionally substituted heterocyclyl,

[6] including but not limited to: piperidinyl, 4-*N,N*-dimethylaminopiperidinyl, *4-N,N*-diethylaminopiperidinyl, 4-*N,N*-diisopropylaminopiperidinyl, 4-hydroxypiperidinyl, 4-(*4*-methylpiperazinyl)piperidinyl, 4-(*4*-ethylpiperazinyl)piperidinyl, 4-(*4*-isopropylpiperazinyl)piperidinyl, 4-(*4*-acetylpiperazinyl)piperidinyl, 4-(*4*-t-butoxyformylpiperazinyl)piperidinyl, 4-(*4*-methanesulfonylpiperazinyl)piperidinyl, 4-(*4*-(2-hydroxyethyl)piperazinyl)piperidinyl, 4-(*4*-(2-cyanoethyl)piperazinyl)piperidinyl, *4*-(*4*-(3-hydroxypropyl)piperazinyl)piperidinyl, 4-(*4*-(2-*N,N*-dimethylaminoethyl)piperazinyl)piperidinyl, 4-(*4*-(2-*N,N*-diethylaminoethyl)piperazinyl)piperidinyl, 4-(*4*-(3-*N,N*-dimethylaminopropyl)piperazinyl)piperidinyl, 4-(4-(3-*N,N*-diethylaminopropyl)piperazinyl)piperidinyl, 4-(tetrahydropyrrolyl)piperidinyl, 4-(3-*N,N*-dimethylaminotetrahydropyrrolyl)piperidinyl;

*4*-methylpiperazinyl, *4*-ethylpiperazinyl, *4*-isopropylpiperazinyl, *4*-acetylpiperazinyl, *4*-t-butoxyformylpiperazinyl, *4*-methanesulfonylpiperazinyl, *4*-(2-hydroxyethyl)piperazinyl, *4*-(2-cyanoethyl)piperazinyl, *4*-(3-hydroxypropyl)piperazinyl, *4*-(2-*N,N*-dimethylaminoethyl)piperazinyl, *4*-(2-*N,N*-diethylaminoethyl)piperazinyl, *4*-(*3*-*N,N*-dimethylaminopropyl)piperazinyl, *4*-(3-*N,N*-diethylaminopropyl)piperazinyl, 2-oxo-piperazin-4-yl, *4*-(*N*-methyl-4-piperidinyl)piperazinyl, *4*-(*N*-ethyl-4-piperidinyl)piperazinyl, *4*-(*N*-acetyl-4-piperidinyl)piperazinyl;

morpholinyl, 3,5-dimethylmorpholinyl, thiomorpholinyl, tetrahydropyrrolyl, 3-*N,N*-dimethylaminotetrahydropyrrolyl, 3-*N,N*-diethylaminotetrahydropyrrolyl;

(4) heteroaryl, for example, but not limited to pyridyl, furyl, thienyl, benzofuryl;

(5) $Z_2$ and $Z_3$ may form oxygen-containing substituted or unsubstituted 5-membered ring or 6-membered ring; the substituent may be selected from the substituents identical with those of $Z_1$;

(6) $Z_4$ and $Z_5$ may form nitrogen-containing substituted or unsubstituted 5-membered ring or 6-membered ring; the substituent may be selected from the substituents identical with those of $Z_1$;

2) H, C1-C6 alkyl, cyclopropyl, cyclobutyl, cyclopentyl, cyclohexyl, N,N-dimethylamino, N,N-diethylamino, N,N-diisopropylamino, 2-N,N-dimethylaminoethyl, 2-hydroxyethyl, 2-morpholinylethyl, 2-thiomorpholinylethyl, 2-(4-methylpiperazinyl)ethyl, 3-N,N-dimethylaminopropyl, 3-N,N-diethylaminopropyl, 3-N,N-diisopropylaminopropyl, 3-hydroxypropyl, 3-morpholinylpropyl, 3-thiomorpholinylpropyl, 3-(4-methylpiperazinyl)propyl, N-methyl-4-piperidinyl, N-ethyl-4-piperidinyl, N-isopropyl-4-piperidinyl, N-acetyl-4-piperidinyl;

$R^3$ preferably is selected from the group consisting of:

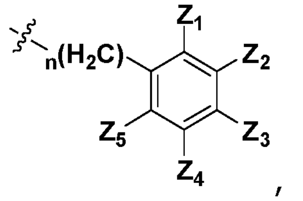
,

wherein n=0 or 1,

when n=0, one of $Z_1$, $Z_2$, $Z_3$, $Z_4$, $Z_5$ is selected from the following groups, the rest being -H: hydroxy, -O-C1-C6 alkyl, -O-C1-C6 fluorine-containing alkyl, -C1-C6 fluorine-containing alkyl;
or, two of $Z_1$, $Z_2$, $Z_3$, $Z_4$, $Z_5$ each are independently selected from the following groups, the rest being -H (more preferably $Z_2$, $Z_4$ each or $Z_2$, $Z_3$ each are independently selected from the following groups, the rest being -H): -C1-C6 fluorine-containing alkyl, 5-membered heteroaryl substituted by -C1-C6 alkyl (more preferably imidazolyl substituted by -C1-C6 alkyl); 6-membered heterocyclyl-C1-C6 alkyl, the 6-membered heterocyclyl is substituted by -C1-C6 alkyl (more preferably piperazinyl-C1-C6 alkyl, the piperazinyl is substituted by -C1-C6 alkyl);
when n=1, one of $Z_1$, $Z_2$, $Z_3$, $Z_4$, $Z_5$ is benzofuryl, the rest being -H;
$R^3$ more preferably is selected from the group consisting of:

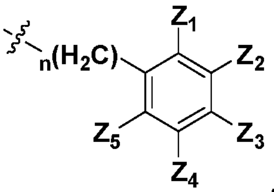,

wherein n=0 or 1, when n=0, $Z_1$, $Z_2$, $Z_4$, $Z_5$ each are -H, $Z_3$ is selected from the following groups: hydroxy, -O-C1-C6 alkyl, -O-C1-C6 fluorine-containing alkyl, -C1-C6 fluorine-containing alkyl;
or, $Z_2$, $Z_4$ each are independently selected from the following groups, the rest being - H: -C1-C6 fluorine-containing alkyl, 5-membered heteroaryl substituted by -C1-C6 alkyl (more preferably imidazolyl substituted by -C1-C6 alkyl);
or, $Z_2$, $Z_3$ each, or $Z_3$, $Z_4$ each are independently selected from the following groups, the rest being -H: -C1-C6 fluorine-containing alkyl; 6-membered heterocyclyl-C1-C6 alkyl, the 6-membered heterocyclyl is substituted by -C1-C6 alkyl (more preferably piperazinyl-C1-C6 alkyl, the piperazinyl is substituted by -C1-C6 alkyl);
when n=1, $Z_1$, $Z_3$, $Z_4$, $Z_5$ each are -H, $Z_2$ is benzofuryl;
$R^3$ most preferably is selected from the group consisting of:

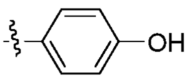, 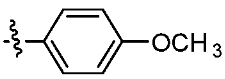, 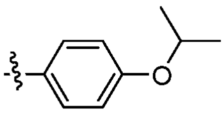, 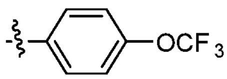, 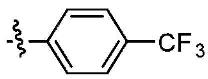,

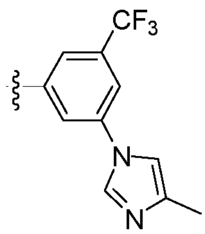, 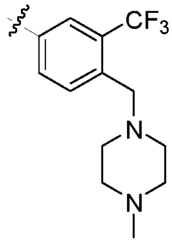, 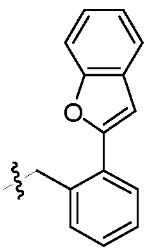,

or a stereoisomer thereof, a prodrug thereof, a pharmaceutically acceptable salt thereof or a pharmaceutically acceptable solvate thereof.

**4.** The compound according to any one of claims 1-3, which is selected from the group consisting of:

| Cmp d ID | Structure | Cmp d ID | Structure |
|---|---|---|---|
| I-1 | 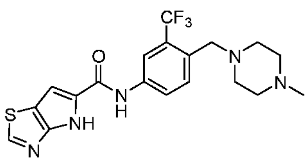 | I-19 | 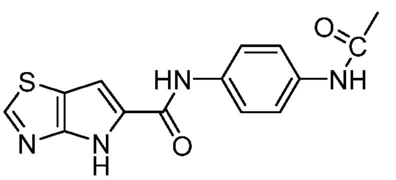 |

(continued)

| Cmp d ID | Structure | Cmp d ID | Structure |
|---|---|---|---|
| I-2 | 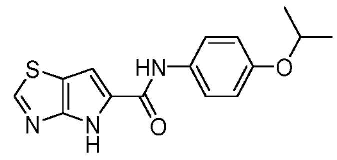 | I-20 | 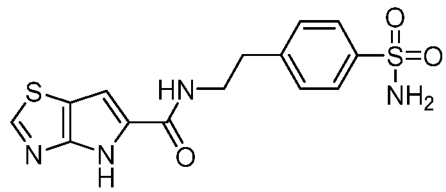 |
| I-3 | 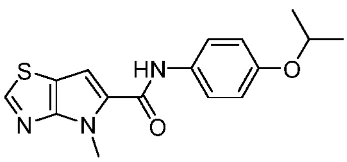 | I-21 | 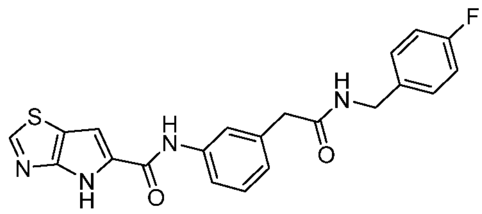 |
| I-4 | 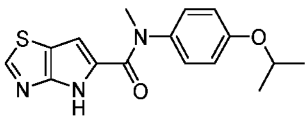 | I-22 | 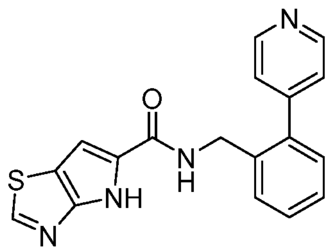 |
| I-5 | 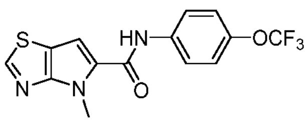 | I-23 | 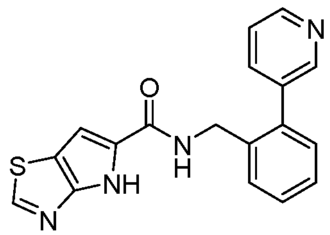 |
| I-6 | 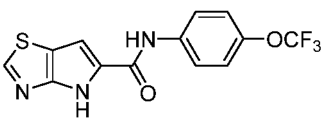 | I-24 | 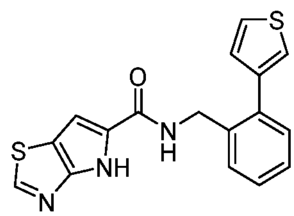 |
| I-7 | 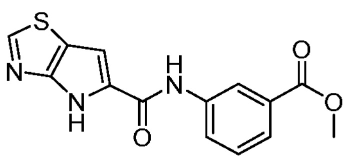 | I-25 | 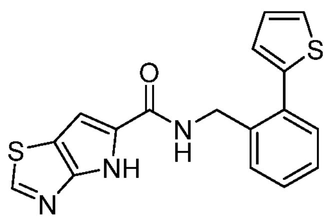 |
| I-8 | 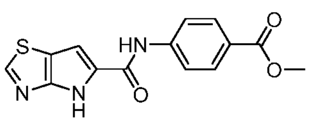 | I-26 | 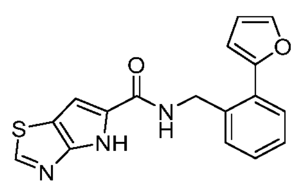 |

(continued)

| Cmp d ID | Structure | Cmp d ID | Structure |
|---|---|---|---|
| I-9 | 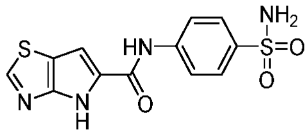 | I-27 | 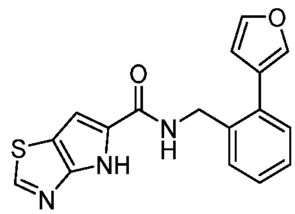 |
| I-10 | 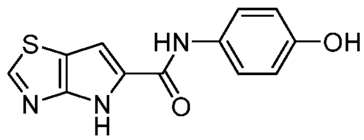 | I-28 | 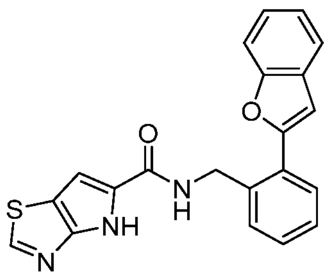 |
| I-11 | 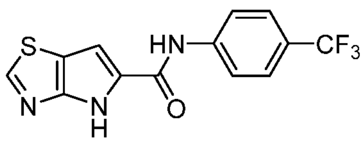 | I-29 | 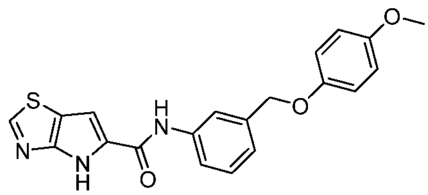 |
| I-12 | 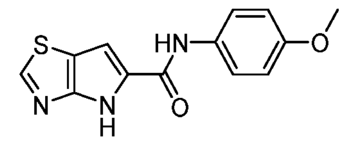 | I-30 | 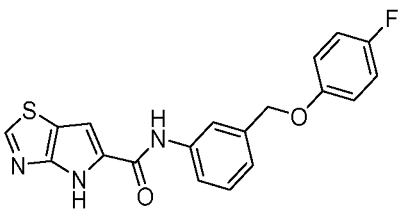 |
| I-13 | 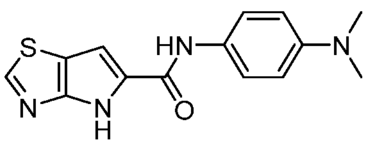 | I-31 | 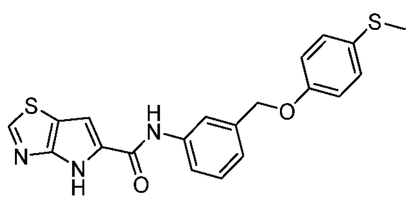 |
| I-14 | 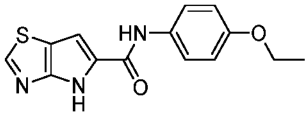 | I-32 | 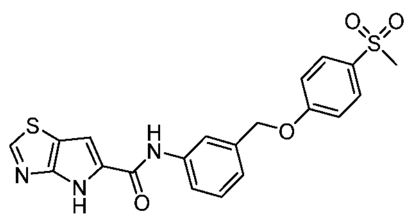 |
| I-15 | 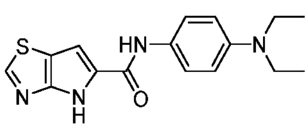 | I-33 | 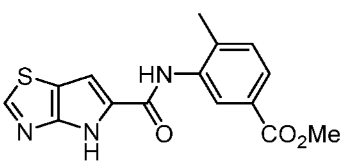 |

(continued)

| Cmp d ID | Structure | Cmp d ID | Structure |
|---|---|---|---|
| I-16 | 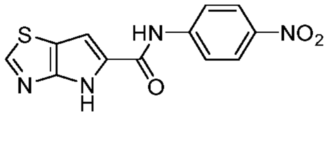 | I-34 | 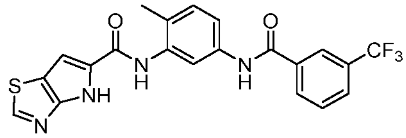 |
| I-17 | 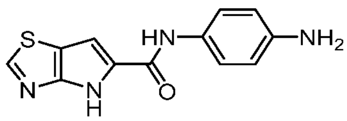 | | |
| I-18 | 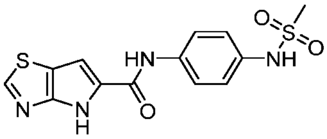 | I-35 | 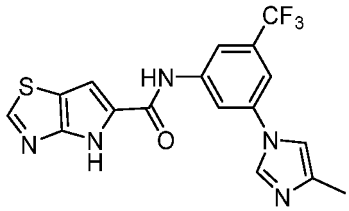 |
| II-1 | 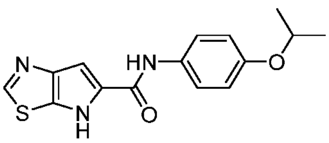 | II-5 | 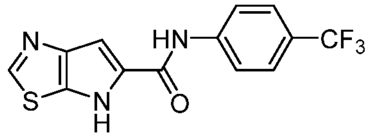 |
| II-2 | 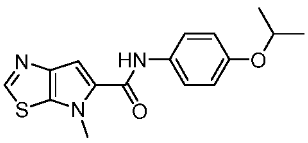 | II-6 | 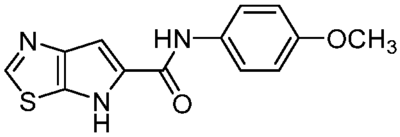 |
| II-3 | 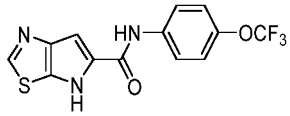 | II-8 | 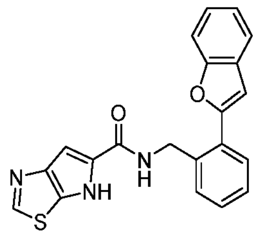 |
| II-4 | 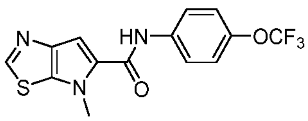 | II-9 | 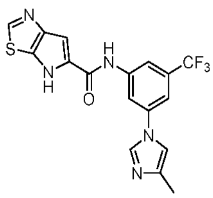 |
| II-7 | 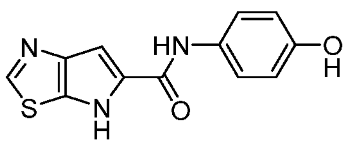 | II-10 | 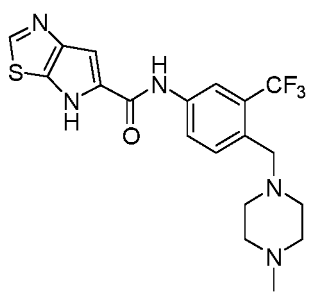 |

or a stereoisomer thereof, a prodrug thereof, a pharmaceutically acceptable salt thereof or a pharmaceutically acceptable solvate thereof.

**5.** A preparation method for the compound according to any one of claims 1-4, comprising the following steps:

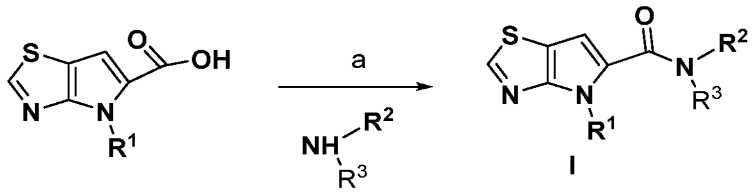


reaction conditions: (a) amide condensation reaction under alkaline condition (triethylamine, diisopropylethylamine);

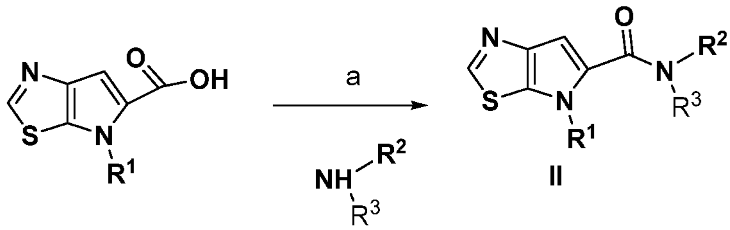


reaction conditions: (a) amide condensation reaction under alkaline condition (triethylamine, diisopropylethylamine).

**6.** A pharmaceutical composition comprising the compound according to any one of claims 1-4 or a stereoisomer thereof, a prodrug thereof, a pharmaceutically acceptable salt thereof or a pharmaceutically acceptable solvate thereof and optionally a pharmaceutically acceptable excipient.

**7.** Use of the compound according to any one of claims 1-4 or a stereoisomer thereof, a prodrug thereof, a pharmaceutically acceptable salt thereof or a pharmaceutically acceptable solvate thereof or the pharmaceutical composition according to claim 6 in the manufacture of a medicament for treating tumor growth and metastasis, wherein the tumor includes but not limited to: gastric cancer, liver cancer, blood tumor, osteosarcoma, prostate cancer, breast cancer, lung cancer.

**INTERNATIONAL SEARCH REPORT**

International application No.
**PCT/CN2018/113549**

**A. CLASSIFICATION OF SUBJECT MATTER**

C07D 513/04(2006.01)i； A61K 31/425(2006.01)i； A61P 35/00(2006.01)i

According to International Patent Classification (IPC) or to both national classification and IPC

**B. FIELDS SEARCHED**

Minimum documentation searched (classification system followed by classification symbols)

C07D 513/04

Documentation searched other than minimum documentation to the extent that such documents are included in the fields searched

Electronic data base consulted during the international search (name of data base and, where practicable, search terms used)

CNKI, CNABS, CNTXT, EPODOC, WPI, REGISTRY, CAPLUS, MARPAT, 吡咯, 噻唑, pyrrol, thiazole, cancer, tumor, 肿瘤, search according to structural formula I.

**C. DOCUMENTS CONSIDERED TO BE RELEVANT**

| Category* | Citation of document, with indication, where appropriate, of the relevant passages | Relevant to claim No. |
|---|---|---|
| X | SARTORI, L. et al. "Thieno[3, 2-b]pyrrole-5-carboxamides as New Reversible Inhibitors of Histone Lysine Demethylase KDM1A/LSD1.Part 1.High-Throughput Screening and Reliminary Exploration" *Journal of Medicinal Chemistry,* Vol. 60, No. (5), 10 February 2017 (2017-02-10), 1673-1692 | 1-7 |
| A | CN 101790313 A (GLAXOSMITHKLINE LLC) 28 July 2010 (2010-07-28) entire document | 1-7 |

☐ Further documents are listed in the continuation of Box C.　☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 January 2019** | **11 February 2019** |

| Name and mailing address of the ISA/CN | Authorized officer |
|---|---|
| **State Intellectual Property Office of the P. R. China No. 6, Xitucheng Road, Jimenqiao Haidian District, Beijing 100088 China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2018/113549**

| Patent document cited in search report | | | Publication date (day/month/year) | Patent family member(s) | | | Publication date (day/month/year) |
|---|---|---|---|---|---|---|---|
| CN | 101790313 | A | 28 July 2010 | TW | 200901969 | A | 16 January 2009 |
| | | | | PE | 03302009 | A1 | 30 April 2009 |
| | | | | UA | 98784 | C2 | 25 June 2012 |
| | | | | US | 2010216746 | A1 | 26 August 2010 |
| | | | | MX | 2009013349 | A | 20 January 2010 |
| | | | | EP | 2166856 | A1 | 31 March 2010 |
| | | | | AR | 066909 | A1 | 23 September 2009 |
| | | | | MA | 31500 | B1 | 01 July 2010 |
| | | | | JP | 2010529965 | A | 02 September 2010 |
| | | | | CR | 11196 | A | 08 March 2010 |
| | | | | NZ | 581409 | A | 30 March 2012 |
| | | | | DO | P2009000266 | A | 15 February 2010 |
| | | | | CO | 6251246 | A2 | 21 February 2011 |
| | | | | ZA | 200908604 | B | 23 February 2011 |
| | | | | WO | 2008154271 | A1 | 18 December 2008 |
| | | | | SG | 182162 | A1 | 30 July 2012 |
| | | | | EA | 200901490 | A1 | 30 June 2010 |
| | | | | EA | 017141 | B1 | 30 October 2012 |
| | | | | BR | PI0812738 | A2 | 30 September 2014 |
| | | | | IL | 202217 | D0 | 16 June 2010 |
| | | | | CA | 2694871 | A1 | 18 December 2008 |
| | | | | EP | 2166856 | A4 | 30 May 2012 |
| | | | | UY | 31132 | A1 | 31 March 2009 |
| | | | | AU | 2008261977 | A1 | 18 December 2008 |
| | | | | KR | 20100039301 | A | 15 April 2010 |
| | | | | US | 8304419 | B2 | 06 November 2012 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Burger's Medicinal Chemistry and Drug Discovery. 1995, vol. 172-178, 949-982
- REMINGTON'S PHARMACEUTICAL SCIENCES. Mack Publishing Company, 1995
- **PERRIN, D. D. ; ARMAREGO, W. L. F. ; PERRINS EDS, D. R.** Purification of Laboratory Chemicals. Pergamon Press, 1980
- *CHEMICAL ABSTRACTS,* 1007386-72-2
- *CHEMICAL ABSTRACTS,* 694499-26-8
- *CHEMICAL ABSTRACTS,* 7664-66-6
- *CHEMICAL ABSTRACTS,* 238749-53-6
- *CHEMICAL ABSTRACTS,* 461-82-5
- *CHEMICAL ABSTRACTS,* 4518-10-9
- *CHEMICAL ABSTRACTS,* 619-45-4
- *CHEMICAL ABSTRACTS,* 63-74-1
- *CHEMICAL ABSTRACTS,* 123-30-8
- *CHEMICAL ABSTRACTS,* 455-14-1
- *CHEMICAL ABSTRACTS,* 104-94-9
- *CHEMICAL ABSTRACTS,* 99-98-9
- *CHEMICAL ABSTRACTS,* 156-43-4
- *CHEMICAL ABSTRACTS,* 93-05-0
- *CHEMICAL ABSTRACTS,* 100-01-6
- *CHEMICAL ABSTRACTS,* 53250-82-1
- *CHEMICAL ABSTRACTS,* 122-80-5
- *CHEMICAL ABSTRACTS,* 35303-76-5
- *European Journal of Medicinal Chemistry,* 2014, vol. 87, 529-539
- *CHEMICAL ABSTRACTS,* 18595-18-1
- *CHEMICAL ABSTRACTS,* 30069-31-9
- *CHEMICAL ABSTRACTS,* 641571-11-1
- *CHEMICAL ABSTRACTS,* 1007386-66-4
- *CHEMICAL ABSTRACTS,* 75103-40-1